(19) Europäisches Patentamt
European Patent Office
Office européen des brevets

(11) EP 4 682 633 A1

(12) EUROPEAN PATENT APPLICATION
published in accordance with Art. 153(4) EPC

(43) Date of publication:
21.01.2026 Bulletin 2026/04

(21) Application number: 24770690.6

(22) Date of filing: 07.03.2024

(51) International Patent Classification (IPC):
G03F 7/004 (2006.01) C07D 233/56 (2006.01)
C07D 235/18 (2006.01) C07D 307/33 (2006.01)
C07D 309/12 (2006.01) C07D 317/38 (2006.01)
C07D 319/20 (2006.01) C07D 327/06 (2006.01)
C07D 333/46 (2006.01) C07D 333/76 (2006.01)
C08F 212/14 (2006.01) C08L 101/02 (2006.01)
G03F 7/20 (2006.01) G03F 7/32 (2006.01)
G03F 7/038 (2006.01) G03F 7/039 (2006.01)

(52) Cooperative Patent Classification (CPC):
C07D 233/56; C07D 235/18; C07D 307/33;
C07D 309/12; C07D 317/38; C07D 319/20;
C07D 327/06; C07D 333/46; C07D 333/76;
C08F 212/14; C08L 101/02; G03F 7/004;
G03F 7/038; G03F 7/039; G03F 7/20; (Cont.)

(86) International application number:
PCT/JP2024/008673

(87) International publication number:
WO 2024/190584 (19.09.2024 Gazette 2024/38)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 16.03.2023 JP 2023041609
08.09.2023 JP 2023146186
17.11.2023 JP 2023196077

(71) Applicant: FUJIFILM Corporation
Tokyo 106-8620 (JP)

(72) Inventors:
- HIURA, Nobuhiro
Haibara-gun, Shizuoka 421-0396 (JP)
- MARUMO, Kazuhiro
Haibara-gun, Shizuoka 421-0396 (JP)
- TANGO, Naohiro
Haibara-gun, Shizuoka 421-0396 (JP)
- GOTO, Akiyoshi
Haibara-gun, Shizuoka 421-0396 (JP)

(74) Representative: HGF
HGF Limited
4th Floor, 1 City Square
Leeds LS1 2AL (GB)

# (54) ACTINIC-RAY-SENSITIVE OR RADIATION-SENSITIVE RESIN COMPOSITION, RESIST FILM, PATTERN FORMATION METHOD, AND ELECTRONIC DEVICE MANUFACTURING METHOD

(57) A first object of the present invention is to provide an actinic ray-sensitive or radiation-sensitive resin composition that provides a narrow space width at which bridge defects start to occur when an L/S pattern is formed.

A second object of the present invention is to provide a resist film, a pattern forming method, and a method for producing an electronic device that are related to the actinic ray-sensitive or radiation-sensitive resin composition.

The actinic ray-sensitive or radiation-sensitive resin composition according to the present invention includes a resin having a group that is decomposed by an action of an acid to generate a polar group, a photoacid generator, and a boron-containing compound and satisfies a requirement 1.

Requirement 1: A value A determined by an equation (1) is 0.120 or more.

EP 4 682 633 A1

(52) Cooperative Patent Classification (CPC): (Cont.)
**G03F 7/32**

## Description

## BACKGROUND OF THE INVENTION

1. Field of the Invention

The present invention relates to an actinic ray-sensitive or radiation-sensitive resin composition, a resist film, a pattern forming method, and a method for producing an electronic device.

2. Description of the Related Art

Since the advent of a resist for KrF excimer laser (248 nm), a pattern forming method utilizing chemical amplification has been used in order to compensate for a decrease in sensitivity due to light absorption. For example, in a positive chemical amplification method, first, a photoacid generator included in portions to be exposed is decomposed by light irradiation to generate an acid. Subsequently, in a process of baking after exposure (PEB: Post Exposure Bake) or the like, for example, an alkali-insoluble group of a resin included in an actinic ray-sensitive or radiation-sensitive resin composition is changed, by the catalytic action of the generated acid, to an alkali-soluble group to change the solubility in a developer. Development is then performed using, for example, a basic aqueous solution. Thus, exposed portions are removed to obtain a desired pattern.

In order to miniaturize semiconductor elements, the wavelengths of exposure light sources have been shortened and the numerical apertures (NAs) of projection lenses have been increased, and currently, exposure apparatuses using ArF excimer laser having a wavelength of 193 nm as light sources have been developed. Furthermore, in recent years, pattern forming methods using extreme ultraviolet rays (EUV light: Extreme Ultraviolet) and an electron beam (EB: Electron Beam) as light sources are also being studied.

Under such circumstances, various configurations have been proposed as actinic ray-sensitive or radiation-sensitive resin compositions.

For example, JP2016-85382A discloses a radiation-sensitive resin composition including a polymer having specific properties and a radiation-sensitive acid generator (photoacid generator) having specific properties.

## SUMMARY OF THE INVENTION

The inventors of the present invention have studied the composition described in JP2016-85382A and found that when a line-and-space pattern (L/S pattern) is formed, bridge defects may occur even in a case where a space width is relatively wide. In recent years, further miniaturization of a pattern is required, and it is required that bridge defects do not occur even in a case where the space width is narrow (in other words, a space distance required to prevent the occurrence of bridge defects be narrow). That is, it has been revealed that there is room for further improvement in narrowing the space width at which bridge defects start to occur when an L/S pattern is formed.

Therefore, an object of the present invention is to provide an actinic ray-sensitive or radiation-sensitive resin composition that provides a narrow space width at which bridge defects start to occur when an L/S pattern is formed.

Another object of the present invention is to provide a resist film, a pattern forming method, and a method for producing an electronic device that are related to the actinic ray-sensitive or radiation-sensitive resin composition.

The inventors of the present invention have found that the above objects can be achieved by the following configurations.

[1] An actinic ray-sensitive or radiation-sensitive resin composition including a resin having a group that is decomposed by an action of an acid to generate a polar group, a photoacid generator, and a boron-containing compound,

wherein a requirement 1 is satisfied.
Requirement 1: A value A determined by an equation (1) described later is 0.120 or more.

[2] The actinic ray-sensitive or radiation-sensitive resin composition according to [1], further including an acid diffusion control agent.

[3] The actinic ray-sensitive or radiation-sensitive resin composition according to [1] or [2],

wherein the photoacid generator is an onium salt compound,
the boron-containing compound is a salt compound,
when the composition includes an acid diffusion control agent which is a salt compound, a requirement 4 is satisfied, and

when the composition does not include an acid diffusion control agent which is a salt compound, a requirement 5 is satisfied.
Requirement 4: A value C1 determined by an equation (4) described later is 0.115 or more.
Requirement 5: A value C2 determined by an equation (5) described later is 0.115 or more.

[4] The actinic ray-sensitive or radiation-sensitive resin composition according to [3],

wherein the composition includes an acid diffusion control agent, and
the acid diffusion control agent is not a salt compound.

[5] The actinic ray-sensitive or radiation-sensitive resin composition according to [3],

wherein the composition includes an acid diffusion control agent, and
the acid diffusion control agent is a salt compound.

[6] The actinic ray-sensitive or radiation-sensitive resin composition according to [5], wherein the acid diffusion control agent is an onium salt compound.
[7] The actinic ray-sensitive or radiation-sensitive resin composition according to any one of [1] to [6], wherein the photoacid generator includes a cation including a fluorine atom.
[8] A resist film formed using the actinic ray-sensitive or radiation-sensitive resin composition according to any one of [1] to [7].
[9] A pattern forming method including:

a step of forming a resist film on a substrate using the actinic ray-sensitive or radiation-sensitive resin composition according to any one of [1] to [7];
a step of exposing the resist film; and
a step of developing the exposed resist film with a developer to form a pattern.

[10] The pattern forming method according to [9], wherein the developer is a developer including an organic solvent.
[11] The pattern forming method according to [10], wherein the developer includes an ester-based solvent having 6 or less carbon atoms and a hydrocarbon-based solvent.
[12] A method for producing an electronic device, the method including the pattern forming method according to [9].
[13] The actinic ray-sensitive or radiation-sensitive resin composition according to claim 1,

wherein the photoacid generator is an onium salt compound,
the boron-containing compound is a salt compound,
when the composition includes an acid diffusion control agent which is an onium salt compound, a requirement 2 is satisfied, and
when the composition does not include an acid diffusion control agent which is an onium salt compound, a requirement 3 is satisfied.
Requirement 2: A value B1 determined by an equation (2) described later is 0.115 or more.
Requirement 3: A value B2 determined by an equation (3) described later is 0.115 or more.

[14] The actinic ray-sensitive or radiation-sensitive resin composition according to [1] or [13], wherein the photoacid generator includes a cation including a fluorine atom.
[15] The actinic ray-sensitive or radiation-sensitive resin composition according to [1], [13], or [14], further including an acid diffusion control agent.
[16] The actinic ray-sensitive or radiation-sensitive resin composition according to [15], wherein the acid diffusion control agent is an onium salt compound.
[17] A resist film formed using the actinic ray-sensitive or radiation-sensitive resin composition according to any one of [1] and [13] to [16].
[18] A pattern forming method including:

a step of forming a resist film on a substrate using the actinic ray-sensitive or radiation-sensitive resin composition according to any one of [1] and [13] to [16];
a step of exposing the resist film; and
a step of developing the exposed resist film with a developer to form a pattern.

[19] The pattern forming method according to [18], wherein the developer is a developer including an organic solvent.
[20] The pattern forming method according to [18] or [19], wherein the developer includes an ester-based solvent having 6 or less carbon atoms and a hydrocarbon-based solvent.
[21] A method for producing an electronic device, the method including the pattern forming method according to any one of [18] to [20].

The present invention can provide an actinic ray-sensitive or radiation-sensitive resin composition that provides a narrow space width at which bridge defects start to occur when an L/S pattern is formed.
The present invention can provide a resist film, a pattern forming method, and a method for producing an electronic device that are related to the actinic ray-sensitive or radiation-sensitive resin composition.

## DESCRIPTION OF THE PREFERRED EMBODIMENTS

The present invention will be described in detail below.
Constituent features may be described on the basis of representative embodiments of the present invention; however the present invention is not limited to such embodiments.
With respect to expressions of groups (atomic groups) in the present specification, an expression without the term of substituted or unsubstituted encompasses groups having no substituent and also groups having a substituent without departing from the spirit and scope of the present invention. For example, "alkyl group" encompasses not only an alkyl group having no substituent (unsubstituted alkyl group) but also an alkyl group having a substituent (substituted alkyl group). In the present specification, "organic group" refers to a group including at least one carbon atom.
The substituent is preferably a monovalent substituent unless otherwise specified.
In the present specification, "actinic ray" or "radiation" means, for example, an emission-line spectrum of a mercury lamp, far ultraviolet rays represented by excimer laser, extreme ultraviolet rays (EUV: Extreme Ultraviolet), X-rays, or an electron beam (EB: Electron Beam). In the present specification, "light" means an actinic ray or a radiation.
In the present specification, "exposure" includes, unless otherwise specified, not only exposure with, for example, an emission-line spectrum of a mercury lamp, far ultraviolet rays represented by excimer laser, extreme ultraviolet rays, X-rays, or EUV light but also patterning with an electron beam or a corpuscular beam such as an ion beam.
In the present specification, a range of numerical values expressed with "to" means a range that includes a numerical value before "to" as a lower limit value and a numerical value after "to" as an upper limit value.
The bonding direction of a divalent group expressed in the present specification is not limited unless otherwise specified. For example, in a compound represented by a formula "X-Y-Z" where Y is -COO-, Y may be -CO-O- or -O-CO-. Furthermore, the compound may be "X-CO-O-Z" or "X-O-CO-Z".
In the present specification, a weight-average molecular weight (Mw), a number-average molecular weight (Mn), and a dispersity (also referred to as a "molecular weight distribution") (Mw/Mn) of a resin are defined as polystyrene equivalent values determined, using a GPC (Gel Permeation Chromatography) apparatus (HLC-8120GPC, manufactured by Tosoh Corporation), by GPC measurement (solvent: tetrahydrofuran, amount of flow (amount of sample injected): 10 μL, column: TSK gel Multipore HXL-M, manufactured by Tosoh Corporation, column temperature: 40°C, flow rate: 1.0 mL/min, detector: differential refractive index detector (Refractive Index Detector)).
In the present specification, the acid dissociation constant (pKa) represents pKa in an aqueous solution, and specifically, is a value determined by calculation using the following software package 1 on the basis of the Hammett's substituent constants and the database of values in publicly known documents. All the values of pKa described in the present specification are values determined by calculation using this software package.
Software package 1: Advanced Chemistry Development (ACD/Labs) Software V8.14 for Solaris (1994-2007 ACD/Labs)
Alternatively, pKa can also be determined by a molecular orbital calculation method. A specific method thereof may be a method of calculating pKa by computing $H^+$ dissociation free energy in an aqueous solution on the basis of a thermodynamic cycle. With regard to the method of calculating the $H^+$ dissociation free energy, the calculation can be performed by, for example, DFT (density functional theory); however, various other methods have been reported in documents etc., and the method is not limited to this. Note that there are a plurality of pieces of software capable of performing DFT; for example, Gaussian 16 may be used.
As described above, pKa in the present specification refers to a value determined by calculation using the software package 1 on the basis of the Hammett's substituent constants and the database of values in publicly known documents; however, when pKa cannot be calculated by this method, a value determined using Gaussian 16 on the basis of DFT (density functional theory) is employed.
In addition, as described above, pKa in the present specification refers to "pKa in an aqueous solution"; however, when pKa in an aqueous solution cannot be calculated, "pKa in a dimethyl sulfoxide (DMSO) solution" is employed.
In the present specification, examples of halogen atoms include a fluorine atom, a chlorine atom, a bromine atom,

and an iodine atom.

In the present specification, the term "solid content" means a component forming a resist film and does not include solvents. Even when a component is in the form of liquid, the component is regarded as a solid content as long as it is a component forming a resist film.

[Actinic ray-sensitive or radiation-sensitive resin composition]

An actinic ray-sensitive or radiation-sensitive resin composition (hereinafter also referred to as a "resist composition") according to the present invention is a resist composition including a resin (hereinafter also referred to as an "acid-decomposable resin") having a group (hereinafter also referred to as an "acid-decomposable group") that is decomposed by an action of an acid to generate a polar group, a photoacid generator, and a boron-containing compound, and satisfies the following requirement 1.

Requirement 1: A value A determined by an equation (1) is 0.12 or more.

$$A = ([H_A] \times 0.04 + [C_A] \times 1.0 + [N_A] \times 2.1 + [O_A] \times 3.6 + [F_A] \times 5.6 + [S_A] \times 1.5 + [B_A] \times 0.5 + [I_A] \times 39.5)/([H_A] \times 1 + [C_A] \times 12 + [N_A] \times 14 + [O_A] \times 16 + [F_A] \times 19 + [S_A] \times 32 + [B_A] \times 11 + [I_A] \times 127) \quad \text{Equation (1):}$$

in the equation (1), $[H_A]$ represents a molar ratio of hydrogen atoms derived from total solid contents in the composition to all atoms of the total solid contents, $[C_A]$ represents a molar ratio of carbon atoms derived from the total solid contents in the composition to all the atoms of the total solid contents, $[N_A]$ represents a molar ratio of nitrogen atoms derived from the total solid contents in the composition to all the atoms of the total solid contents, $[O_A]$ represents a molar ratio of oxygen atoms derived from the total solid contents in the composition to all the atoms of the total solid contents, $[F_A]$ represents a molar ratio of fluorine atoms derived from the total solid contents in the composition to all the atoms of the total solid contents, $[S_A]$ represents a molar ratio of sulfur atoms derived from the total solid contents in the composition to all the atoms of the total solid contents, $[B_A]$ represents a molar ratio of boron atoms derived from the total solid contents in the composition to all the atoms of the total solid contents, and $[I_A]$ represents a molar ratio of iodine atoms derived from the total solid contents in the composition to all the atoms of the total solid contents.

The resist composition according to the present invention having the above-described configuration provides a narrow space width at which bridge defects start to occur when an L/S pattern is formed. That is, when the resist composition according to the present invention is used to form an L/S pattern, the space distance required to prevent the occurrence of bridge defects is narrow. Hereinafter, the features of the resist composition according to the present invention will be described together with a presumed action mechanism.

First, one of the features of the resist composition according to the present invention is that the value A determined by the equation (1) is 0.120 or more (Requirement 1).

For example, light having a relatively short wavelength (for example, EUV light (wavelength 13.5 nm) or the like) among actinic rays and radiations provides a small number of incident photons when exposure is performed at the same sensitivity. Therefore, "photon shot noise", which is stochastic variations in the number of photons, exerts a great influence, and bridge defects are likely to occur. In order to reduce the photon shot noise, a method of increasing the exposure dose to increase the number of incident photons may be employed; however, this method is a trade-off with the demand for higher sensitivity.

On the other hand, in the present invention, the light absorbance is increased by a method which does not depend on an increase in the exposure dose. That is, by increasing the value A shown in the requirement 1, the light absorption per film thickness of a film (resist film) formed from the resist composition is increased to suppress bridge defects and the like caused by the photon shot noise.

For example, in a case where the resist composition includes an acid-decomposable resin, a photoacid generator, a boron-containing compound, an acid diffusion control agent, and a solvent, the acid-decomposable resin, the photoacid generator, the boron-containing compound, and the acid diffusion control agent correspond to solid contents. That is, all atoms of total solid contents correspond to a total of all atoms derived from the acid-decomposable resin, all atoms derived from the photoacid generator, all atoms derived from the boron-containing compound, and all atoms derived from the acid diffusion control agent. For example, $[H_A]$ represents the molar ratio of hydrogen atoms derived from the total solid contents to all the atoms of the total solid contents. On the basis of the above example, $[H_A]$ represents the molar ratio of the total of hydrogen atoms derived from the acid-decomposable resin, hydrogen atoms derived from the photoacid generator, hydrogen atoms derived from the boron-containing compound, and hydrogen atoms derived from the acid diffusion control agent to the total of all the atoms derived from the acid-decomposable resin, all the atoms derived from the photoacid generator, all the atoms derived from the boron-containing compound, and all the atoms derived from the acid diffusion control agent.

When the structures and contents of constituent components of the total solid contents in the resist composition are known, the value A can be determined by calculating the ratio of the numbers of atoms contained. Even when the

constituent components are unknown, the ratio of the numbers of constituent atoms can be calculated by subjecting a resist film obtained by evaporating the solvent component in the resist composition to an analytical method such as elemental analysis.

Another feature of the resist composition according to the present invention is that the resist composition includes a boron-containing compound.

Upon irradiation of a film (resist film) formed from the resist composition according to the present invention with an actinic ray and radiation (preferably EUV light), many radical cations and electrons of the acid-decomposable resin having a relatively high content are generated in the film, and electron transfer from the orbital of the boron-containing compound having a high electron-donating property to the radical cations of the acid-decomposable resin can occur. As a result, the radical cations of the acid-decomposable resin become neutral radicals, and electrons generated upon irradiation with the actinic ray or radiation and present in the system are transferred to the neutral radicals of the acid-decomposable resin to generate radical anions of the acid-decomposable resin. That is, in the resist film formed from the resist composition, the intervention of the boron-containing compound provides excellent generation efficiency of radical anions of the acid-decomposable resin.

It is presumed that, as the generation efficiency of radical anions of the acid-decomposable resin is improved by the boron-containing compound and the amount of radical anions of the acid-decomposable resin generated increases, electron transfer from the radical anions of the acid-decomposable resin to the photoacid generator is more likely to occur in the film, and the decomposition of the photoacid generator and the generation of the generated acid are promoted. It is considered that, as a result, the deprotection reaction of the acid-decomposable resin by the generated acid is likely to appropriately proceed, and the space width at which bridge defects start to occur becomes narrow in the formation of an L/S pattern (that is, the space distance required to prevent the occurrence of bridge defects is narrow in the formation of an L/S pattern).

If the boron-containing compound is not present in the resist film, recombination of electrons and radical cations of the acid-decomposable resin generated in the film is likely to occur, so that the above-described action mechanism does not occur and the desired effect is less likely to be obtained.

As described above, the value A is 0.120 or more, but is preferably 0.130 or more, more preferably 0.135 or more from the viewpoints that the space width at which bridge defects start to occur in the formation of an L/S pattern becomes narrower, a pattern having smaller LER (Line Edge Roughness) can be formed, a pattern having even higher resolution can be formed, and/or sensitivity is even higher. The upper limit is not particularly limited, but if the value A is excessively large, the light transmittance of the resist film decreases, the optical image profile in the resist film deteriorates, and as a result, it becomes difficult to obtain a good pattern shape; therefore, the value A is preferably 0.240 or less, more preferably 0.220 or less.

Hereinafter, when the space width at which bridge defects start to occur in the formation of an L/S pattern becomes narrower, this may be referred to as "a better effect of the present invention is provided".

Hereinafter, various components included in the resist composition will be described.

[Acid-decomposable resin (Resin (A))]

The resist composition includes an acid-decomposable resin (hereinafter, also referred to as a "resin (A)).

The acid-decomposable resin is a resin having a group (acid-decomposable group) that is decomposed by the action of an acid to generate a polar group, and is preferably a resin including a repeating unit having an acid-decomposable group.

The acid-decomposable resin is typically a resin that is decomposed by the action of an acid to undergo an increase in polarity. The acid-decomposable resin undergoes an increase in polarity due to the action of an acid to have an increased degree of solubility in an alkali developer and a decreased degree of solubility in an organic solvent.

That is, in the case of carrying out pattern formation using an acid-decomposable resin, typically, when an alkali developer is adopted as the developer, a positive-type pattern is suitably formed, and when an organic solvent-based developer is adopted as the developer, a negative-type pattern is suitably formed.

<Repeating unit having acid-decomposable group>

The acid-decomposable group preferably has a structure in which a polar group is protected with a leaving group that leaves due to the action of an acid. Hereinafter, the acid-decomposable group will be described, and then the repeating unit having an acid-decomposable group will be described.

(Acid-decomposable group)

The acid-decomposable group refers to a group that is decomposed by the action of an acid to generate a polar

group. The acid-decomposable group preferably has a structure in which a polar group is protected with a leaving group that leaves due to the action of an acid. The acid-decomposable group can be decomposed by the action of an acid to generate a polar group.

The polar group is preferably an alkali-soluble group, and examples thereof include acidic groups such as a carboxyl group, phenolic hydroxyl groups, fluorinated alcohol groups, a sulfonic group, a phosphate group, a sulfonamide group, a sulfonylimide group, (alkylsulfonyl)(alkylcarbonyl)methylene groups, (alkylsulfonyl)(alkylcarbonyl)imide groups, bis(alkylcarbonyl)methylene groups, bis(alkylcarbonyl)imide groups, bis(alkylsulfonyl)methylene groups, bis(alkylsulfonyl)imide groups, tris(alkylcarbonyl)methylene groups, and tris(alkylsulfonyl)methylene groups, and alcoholic hydroxyl groups.

Of these, the polar group is preferably a carboxyl group, a phenolic hydroxyl group, a fluorinated alcohol group (preferably a hexafluoroisopropanol group), or a sulfonic group.

Examples of the leaving group that leaves due to the action of an acid include groups represented by formulae (Y1) to (Y4).

Formula (Y1): $-C(Rx_1)(Rx_2)(Rx_3)$

Formula (Y2): $-C(=O)OC(Rx_1)(Rx_2)(Rx_3)$

Formula (Y3): $-C(R_{36})(R_{37})(OR_{38})$

Formula (Y4): $-C(Rn)(H)(Ar)$

In the formula (Y1) and the formula (Y2), $Rx_1$ to $Rx_3$ each independently represent a linear or branched alkyl group, a cycloalkyl group, an alkenyl group, or an aryl group. Two of $Rx_1$ to $Rx_3$ may be bonded together to form a ring.

Note that, when all of $Rx_1$ to $Rx_3$ are linear or branched alkyl groups, at least two of $Rx_1$ to $Rx_3$ are preferably methyl groups.

In particular, $Rx_1$ to $Rx_3$ are each preferably a linear or branched alkyl group, more preferably a linear alkyl group.

The linear or branched alkyl group represented by $Rx_1$ to $Rx_3$ is preferably linear.

The number of carbon atoms of the linear or branched alkyl group represented by $Rx_1$ to $Rx_3$ is preferably 1 to 10, more preferably 1 to 5, still more preferably 1 to 3.

Specific examples of the linear or branched alkyl group represented by $Rx_1$ to $Rx_3$ include a methyl group, an ethyl group, a n-propyl group, an isopropyl group, a n-butyl group, an isobutyl group, and a t-butyl group.

The cycloalkyl group represented by $Rx_1$ to $Rx_3$ may be monocyclic or polycyclic.

The number of carbon atoms of the cycloalkyl group represented by $Rx_1$ to $Rx_3$ is preferably 6 to 15, more preferably 6 to 10.

Specific examples of the cycloalkyl group represented by $Rx_1$ to $Rx_3$ include monocyclic cycloalkyl groups such as a cyclopentyl group and a cyclohexyl group; and polycyclic cycloalkyl groups such as a norbornyl group, a tetracyclodecanyl group, a tetracyclododecanyl group, and an adamantyl group.

The alkenyl group in $Rx_1$ to $Rx_3$ is preferably a vinyl group.

The aryl group in $Rx_1$ to $Rx_3$ is preferably an aryl group having 6 to 10 carbon atoms, and examples thereof include a phenyl group, a naphthyl group, and an anthryl group.

Two of $Rx_1$ to $Rx_3$ may be bonded together to form a ring.

The ring may be monocyclic or polycyclic.

The ring is preferably a cycloalkyl group, more preferably a five- or six-membered monocyclic cycloalkyl group.

Specific examples of the ring include monocyclic cycloalkyl groups such as a cyclopentyl group and a cyclohexyl group; and polycyclic cycloalkyl groups such as a norbornyl group, a tetracyclodecanyl group, a tetracyclododecanyl group, and an adamantyl group.

In the cycloalkyl group formed by bonding two of $Rx_1$ to $Rx_3$ together, for example, one of methylene groups constituting the ring may be replaced by a heteroatom such as an oxygen atom, a group having a heteroatom, such as a carbonyl group, or a vinylidene group. In the cycloalkyl group, one or more ethylene groups constituting the cycloalkane ring may be replaced by vinylene groups.

In a preferred embodiment of the group represented by the formula (Y1) or the formula (Y2), for example, $Rx_1$ is a methyl group or an ethyl group, and $Rx_2$ and $Rx_3$ are bonded together to form the above-described cycloalkyl group.

When the resist composition is, for example, a resist composition for EUV exposure, the linear or branched alkyl group, the cycloalkyl group, the alkenyl group, and the aryl group represented by $Rx_1$ to $Rx_3$ and the ring formed by bonding two of $Rx_1$ to $Rx_3$ together also preferably further have, as a substituent, a fluorine atom or an iodine atom.

In the formula (Y3), $R_{36}$ to $R_{38}$ each independently represent a hydrogen atom or a monovalent organic group. $R_{37}$ and $R_{38}$ may be bonded together to form a ring.

It is also preferable that $R_{36}$ be a hydrogen atom.

The monovalent organic group may be, for example, a linear or branched alkyl group, a cycloalkyl group, an aryl group, an aralkyl group, or an alkenyl group.

One or more methylene groups in the linear or branched alkyl group, the cycloalkyl group, the aryl group, and the aralkyl group may be replaced by a group selected from the group consisting of a heteroatom such as an oxygen atom and an atomic group having a heteroatom, such as a carbonyl group. That is, the linear or branched alkyl group, the cycloalkyl group, the aryl group, and the aralkyl group may include a heteroatom such as an oxygen atom and an atomic group having a heteroatom, such as a carbonyl group.

Further, in a repeating unit having an acid-decomposable group described later, $R_{38}$ may be bonded to another substituent included in the main chain of the repeating unit to form a ring. The group formed by bonding $R_{38}$ and another substituent included in the main chain of the repeating unit is preferably an alkylene group such as a methylene group.

When the resist composition is, for example, a resist composition for EUV exposure, the monovalent organic group represented by $R_{36}$ to $R_{38}$ and the ring formed by bonding $R_{37}$ and $R_{38}$ together also preferably further have, as a substituent, a fluorine atom or an iodine atom.

The formula (Y3) preferably represents a group represented by a formula (Y3-1) below.

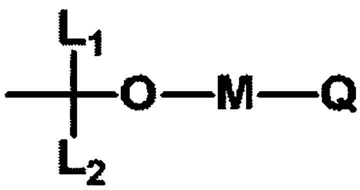

(Y3-1)

Here, $L_1$ and $L_2$ each independently represent a hydrogen atom, a linear or branched alkyl group, a cycloalkyl group, an aryl group, or a group provided by combining any of these groups (for example, a group provided by combining a linear or branched alkyl group and an aryl group).

M represents a single bond or a divalent linking group.

Q represents a linear or branched alkyl group that may include a heteroatom, a cycloalkyl group that may include a heteroatom, an aryl group that may include a heteroatom, an amino group, an ammonium group, a mercapto group, a cyano group, an aldehyde group, or a group provided by combining any of these groups (for example, a group provided by combining a linear or branched alkyl group and a cycloalkyl group).

In the linear or branched alkyl group and the cycloalkyl group, for example, one of methylene groups may be replaced by a group selected from the group consisting of a heteroatom such as an oxygen atom and an atomic group having a heteroatom, such as a carbonyl group.

Note that one of $L_1$ and $L_2$ is preferably a hydrogen atom and the other is preferably a linear or branched alkyl group, a cycloalkyl group, an aryl group, or a group provided by combining an alkylene group and an aryl group.

At least two of Q, M, and $L_1$ may be bonded together to form a ring (preferably a five-membered or six-membered ring).

From the viewpoint of forming a finer pattern, $L_2$ is preferably a secondary or tertiary alkyl group, more preferably a tertiary alkyl group. Examples of the secondary alkyl group include an isopropyl group, a cyclohexyl group, and a norbornyl group. Examples of the tertiary alkyl group include a tert-butyl group and an adamantane group. In the case of such embodiments, the repeating unit having an acid-decomposable group described later increases the Tg (glass transition temperature) and activation energy of the resin (A); therefore, film hardness can be ensured, and in addition, fogging can be suppressed.

When the resist composition is, for example, a resist composition for EUV exposure, the linear or branched alkyl group, the cycloalkyl group, the aryl group, and the group provided by combining any of these groups represented by $L_1$ and $L_2$ also preferably further have, as a substituent, a fluorine atom or an iodine atom. It is also preferable that one of methylene groups in the linear or branched alkyl group, the cycloalkyl group, the aryl group, and the aralkyl group be replaced by a group selected from the group consisting of a heteroatom such as an oxygen atom and an atomic group having a heteroatom, such as a carbonyl group.

It is also preferable that when the resist composition is, for example, a resist composition for EUV exposure, in the linear or branched alkyl group that may include a heteroatom, the cycloalkyl group that may include a heteroatom, the aryl group that may include a heteroatom, the amino group, the ammonium group, the mercapto group, the cyano group, the aldehyde group, and the group provided by combining any of these groups represented by Q, the heteroatom be a heteroatom selected from the group consisting of a fluorine atom, an iodine atom, and an oxygen atom.

In the formula (Y4), Ar represents an aromatic ring group. Rn represents a linear or branched alkyl group, a cycloalkyl group, or an aryl group. Rn and Ar may be bonded together to form a non-aromatic ring. Ar is more preferably an aryl group.

When the resist composition is, for example, a resist composition for EUV exposure, the aromatic ring group represented by Ar, and the linear or branched alkyl group, the cycloalkyl group, and the aryl group represented by Rn also preferably have, as a substituent, a fluorine atom and an iodine atom.
From the viewpoint of further improving acid decomposability, in the leaving group protecting the polar group, when a non-aromatic ring is directly bonded to the polar group (or a residue thereof), a ring member atom adjacent to a ring member atom directly bonded to the polar group (or a residue thereof) in the non-aromatic ring also preferably does not have, as a substituent, a halogen atom such as a fluorine atom.
Alternatively, the leaving group that leaves due to the action of an acid may be a 2-cyclopentenyl group having a substituent (e.g., an alkyl group), such as a 3-methyl-2-cyclopentenyl group, or a cyclohexyl group having a substituent (e.g., an alkyl group), such as a 1,1,4,4-tetramethylcyclohexyl group.

(Repeating unit including acid-decomposable group)

Next, the repeating unit that has an acid-decomposable group and can be included in the resin (A) will be described.
The repeating unit having an acid-decomposable group is preferably a repeating unit having the acid-decomposable group described above and is also preferably a repeating unit represented by a formula (A) below.

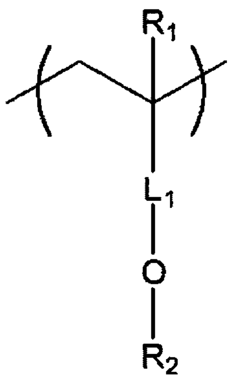

(A)

$L_1$ represents a divalent linking group that may have a fluorine atom or an iodine atom. $R_1$ represents a hydrogen atom, a fluorine atom, an iodine atom, an alkyl group that may have a fluorine atom or an iodine atom, or an aryl group that may have a fluorine atom or an iodine atom. $R_2$ represents a leaving group that leaves due to the action of an acid and that may have a fluorine atom or an iodine atom. In particular, at least one of $L_1$, $R_1$, or $R_2$ preferably has a fluoride atom or an iodide atom.
$L_1$ represents a divalent linking group that may have a fluorine atom or an iodine atom. Examples of the divalent linking group that may have a fluorine atom or an iodine atom include -CO-, -O-, -S-, -SO-, $-SO_2-$, hydrocarbon groups that may have a fluorine atom or an iodine atom (for example, a linear or branched alkylene group, a cycloalkylene group, an alkenylene group, and an arylene group), and a linking group provided by linking a plurality of these together. In particular, $L_1$ is preferably -CO-, an arylene group, or an -arylene group-linear or branched alkylene group having a fluorine atom or an iodine atom-, more preferably -CO- or an -arylene group-linear or branched alkylene group having a fluorine atom or an iodine atom-.
The arylene group is preferably a phenylene group.
The number of carbon atoms of the linear or branched alkylene group is not particularly limited, but is preferably 1 to 10, more preferably 1 to 3.
The total number of fluorine atoms and iodine atoms included in the linear or branched alkylene group having a fluorine atom or an iodine atom is not particularly limited, but is preferably 2 or more, more preferably 2 to 10, still more preferably 3 to 6.
$R_1$ represents a hydrogen atom, a fluorine atom, an iodine atom, a linear or branched alkyl group that may have a fluorine atom or an iodine atom, or an aryl group that may have a fluorine atom or an iodine atom.
The number of carbon atoms of the linear or branched alkyl group is not particularly limited, but is preferably 1 to 10, more preferably 1 to 3.
The total number of fluorine atoms and iodine atoms included in the linear or branched alkyl group having a fluorine atom or an iodine atom is not particularly limited, but is preferably 1 or more, more preferably 1 to 5, still more preferably 1 to 3.
The linear or branched alkyl group having a fluorine atom or an iodine atom may include a heteroatom other than halogen atoms, such as an oxygen atom.

$R_2$ represents a leaving group that leaves due to the action of an acid and that may have a fluorine atom or an iodine atom. Examples of the leaving group that may have a fluorine atom or an iodine atom include leaving groups represented by the formulae (Y1) to (Y4) and leaving groups represented by the formulae (Y1) to (Y4) and having a fluorine atom or an iodine atom, and preferred embodiments thereof are also the same.

The repeating unit having an acid-decomposable group is also preferably a repeating unit represented by a formula (AI).

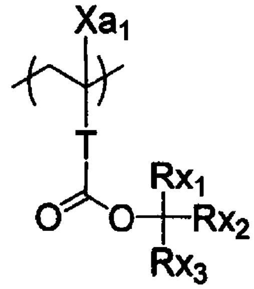

(A I)

In the formula (AI),

$Xa_1$ represents a hydrogen atom or an alkyl group that may have a substituent.
T represents a single bond or a divalent linking group.
$Rx_1$ to $Rx_3$ each independently represent a linear or branched alkyl group, a cycloalkyl group, an alkenyl group, or an aryl group. Note that two of $Rx_1$ to $Rx_3$ may be bonded together to form a ring.

Note that, when all of $Rx_1$ to $Rx_3$ are linear or branched alkyl groups, at least two of $Rx_1$ to $Rx_3$ are preferably methyl groups.

In particular, $Rx_1$ to $Rx_3$ are each preferably a linear or branched alkyl group, more preferably a linear alkyl group.

The alkyl group that may have a substituent and that is represented by $Xa_1$ may be, for example, a methyl group or a group represented by $-CH_2-R_{11}$. $R_{11}$ represents a halogen atom (such as a fluorine atom), a hydroxyl group, or a monovalent organic group, and examples thereof include alkyl groups having 5 or less carbon atoms and optionally substituted with a halogen atom, acyl groups having 5 or less carbon atoms and optionally substituted with a halogen atom, and alkoxy groups having 5 or less carbon atoms and optionally substituted with a halogen atom. $R_{11}$ is preferably an alkyl group having 3 or less carbon atoms, more preferably a methyl group. $Xa_1$ is preferably a hydrogen atom, a methyl group, a trifluoromethyl group, or a hydroxymethyl group.

Examples of the divalent linking group represented by T include alkylene groups, aromatic ring groups, a -COO-Rt- group, and an -O-Rt- group. In the formulae, Rt represents a linear or branched alkylene group or a cycloalkylene group.

T is preferably a single bond or a -COO-Rt- group. When T represents a -COO-Rt- group, Rt is preferably an alkylene group having 1 to 5 carbon atoms, more preferably $-CH_2-$, $-(CH_2)_2-$, or $-(CH_2)_3-$.

The linear or branched alkyl group represented by $Rx_1$ to $Rx_3$ is preferably linear.

The number of carbon atoms of the linear or branched alkyl group represented by $Rx_1$ to $Rx_3$ is preferably 1 to 10, more preferably 1 to 5, still more preferably 1 to 3.

Specific examples of the linear or branched alkyl group represented by $Rx_1$ to $Rx_3$ include a methyl group, an ethyl group, a n-propyl group, an isopropyl group, a n-butyl group, an isobutyl group, and a t-butyl group.

The cycloalkyl group represented by $Rx_1$ to $Rx_3$ may be monocyclic or polycyclic.

The number of carbon atoms of the cycloalkyl group represented by $Rx_1$ to $Rx_3$ is preferably 6 to 15, more preferably 6 to 10.

Specific examples of the cycloalkyl group represented by $Rx_1$ to $Rx_3$ include monocyclic cycloalkyl groups such as a cyclopentyl group and a cyclohexyl group; and polycyclic cycloalkyl groups such as a norbornyl group, a tetracyclodecanyl group, a tetracyclododecanyl group, and an adamantyl group.

The alkenyl group in $Rx_1$ to $Rx_3$ is preferably a vinyl group.

The aryl group in $Rx_1$ to $Rx_3$ is preferably an aryl group having 6 to 10 carbon atoms, and examples thereof include a phenyl group, a naphthyl group, and an anthryl group.

Two of $Rx_1$ to $Rx_3$ may be bonded together to form a ring.

The ring may be monocyclic or polycyclic.

The ring is preferably a cycloalkyl group, more preferably a five- or six-membered monocyclic cycloalkyl group.

Specific examples of the ring include monocyclic cycloalkyl groups such as a cyclopentyl group and a cyclohexyl group; and polycyclic cycloalkyl groups such as a norbornyl group, a tetracyclodecanyl group, a tetracyclododecanyl group, and an adamantyl group.

In the cycloalkyl group formed by bonding two of $Rx_1$ to $Rx_3$ together, for example, one of methylene groups constituting the ring may be replaced by a heteroatom such as an oxygen atom, a group having a heteroatom, such as a carbonyl group, or a vinylidene group. In the cycloalkyl group, one or more ethylene groups constituting the cycloalkane ring may be replaced by vinylene groups.

In a preferred embodiment of the repeating unit represented by the formula (AI), for example, $Rx_1$ is a methyl group or an ethyl group, and $Rx_2$ and $Rx_3$ are bonded together to form the above-described cycloalkyl group.

When any of the above-described groups has a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms).

The repeating unit represented by the formula (AI) is preferably an acid-decomposable (meth)acrylic acid tertiary alkyl ester-based repeating unit (a repeating unit in which $Xa_1$ represents a hydrogen atom or a methyl group, and T represents a single bond).

The following are specific examples of the repeating unit having an acid-decomposable group, but the present invention is not limited thereto. Note that, in the formulae, $Xa_1$ represents any of H, $CH_3$, $CF_3$, and $CH_2OH$, and Rxa and Rxb each represent a linear or branched alkyl group having 1 to 5 carbon atoms.

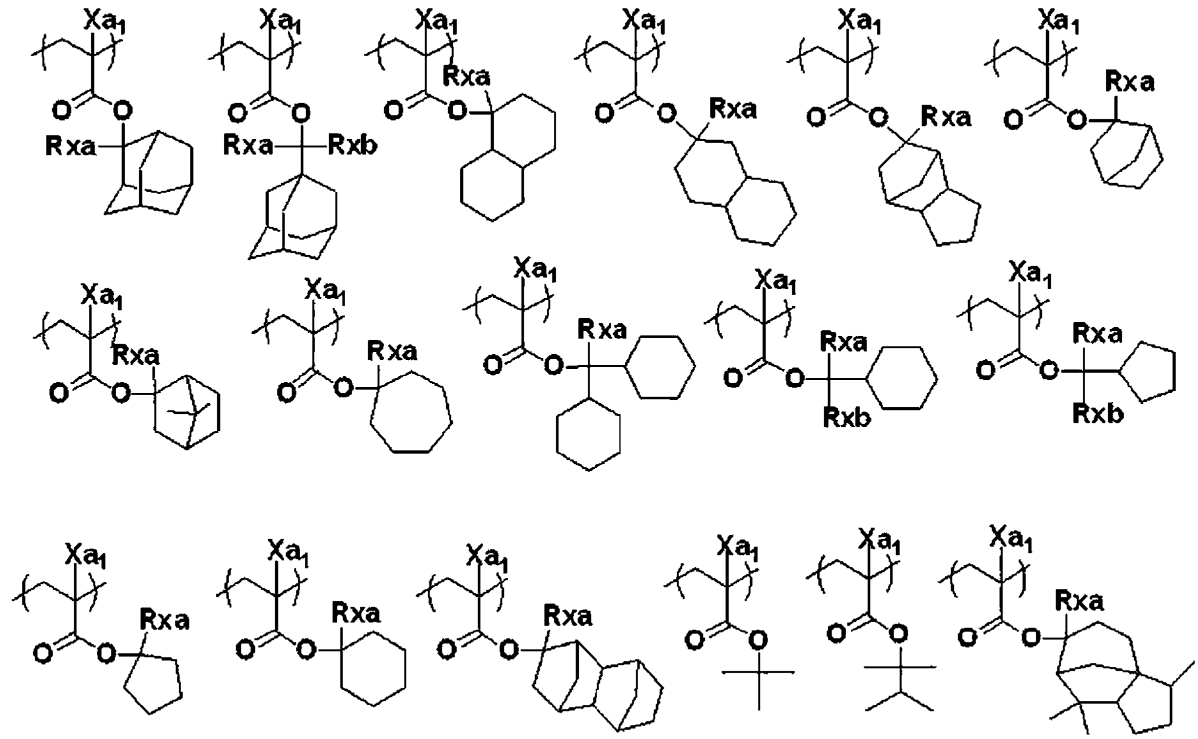

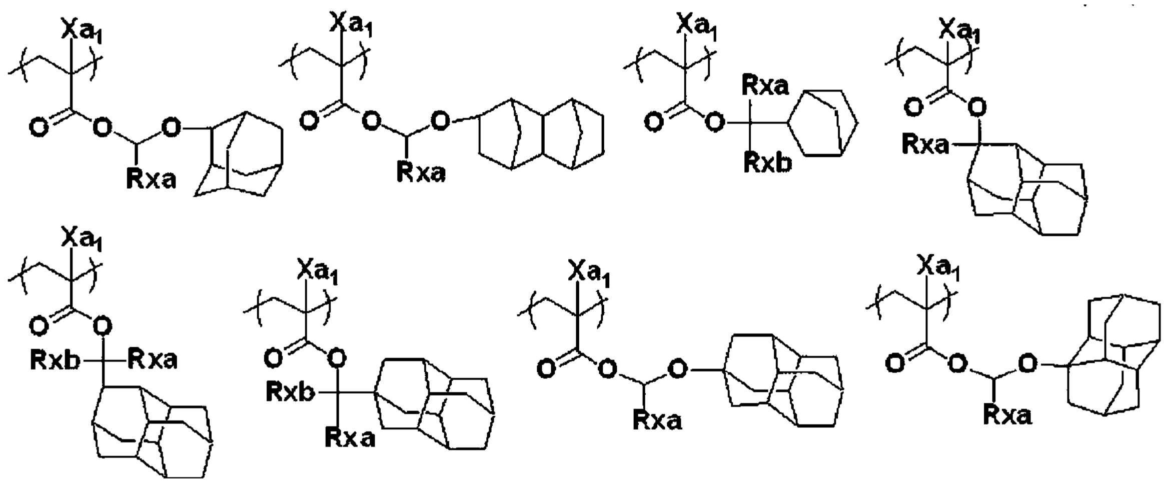

(Repeating unit having acid-decomposable group including unsaturated bond)

It is also preferable that the resin (A) have, as the repeating unit having an acid-decomposable group, a repeating unit having an acid-decomposable group including an unsaturated bond.

The repeating unit having an acid-decomposable group including an unsaturated bond is preferably a repeating unit represented by a formula (B).

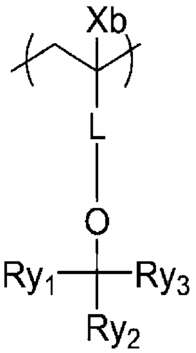

(B)

In the formula (B),

Xb represents a hydrogen atom, a halogen atom, or an alkyl group that may have a substituent.

L represents a single bond or a divalent linking group that may have a substituent.

$Ry_1$ to $Ry_3$ each independently represent a hydrogen atom, a linear or branched alkyl group, a monocyclic or polycyclic cycloalkyl group, an alkenyl group, an alkynyl group, or a monocyclic or polycyclic aryl group. Any two of $Ry_1$ to $Ry_3$ may be bonded together to form a monocyclic or polycyclic ring (for example, a monocyclic or polycyclic cycloalkyl group, cycloalkenyl group, or the like).

However, at least one of $Ry_1$ to $Ry_3$ represents an alkenyl group, an alkynyl group, a monocyclic or polycyclic cycloalkenyl group, or a monocyclic or polycyclic aryl group, or any two of $Ry_1$ to $Ry_3$ are bonded together to form a monocyclic or polycyclic alicycle (for example, a monocyclic or polycyclic cycloalkyl group, cycloalkenyl group, or the like). In addition, two or more of $Ry_1$ to $Ry_3$ are not hydrogen atoms, and when any one of $Ry_1$ to $Ry_3$ represents a hydrogen atom, the other two of $Ry_1$ to $Ry_3$ are bonded together to form a ring having one or more vinylene groups in the ring structure, and at least one of the vinylene groups is present adjacent to a carbon atom to which a hydrogen atom represented by any one of $Ry_1$ to $Ry_3$ is bonded.

The alkyl group in $Ry_1$ to $Ry_3$ is preferably an alkyl group having 1 to 4 carbon atoms such as a methyl group, an ethyl group, a n-propyl group, an isopropyl group, a n-butyl group, an isobutyl group, or a t-butyl group.

The cycloalkyl group in $Ry_1$ to $Ry_3$ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

The aryl group in $Ry_1$ to $Ry_3$ is preferably an aryl group having 6 to 15 carbon atoms, more preferably an aryl group

having 6 to 10 carbon atoms, and may be, for example, a phenyl group, a naphthyl group, an anthryl group, or the like.

The alkenyl group in $Ry_1$ to $Ry_3$ is preferably a vinyl group.

The alkynyl group in $Ry_1$ to $Ry_3$ is preferably an ethynyl group.

The cycloalkenyl group in $Ry_1$ to $Ry_3$ preferably has a structure in which a monocyclic cycloalkyl group, such as a cyclopentyl group or a cyclohexyl group, partially includes a double bond.

The cycloalkyl group formed by bonding two of $Ry_1$ to $Ry_3$ together is preferably a monocyclic cycloalkyl group such as a cyclopentyl group or a cyclohexyl group, or preferably a polycyclic cycloalkyl group such as a norbornyl group, a tetracyclodecanyl group, a tetracyclododecanyl group, or an adamantyl group. Of these, a monocyclic cycloalkyl group having 5 to 6 carbon atoms is preferred.

In the cycloalkyl group and the cycloalkenyl group formed by bonding two of $Ry_1$ to $Ry_3$ together, for example, one of methylene groups constituting the ring may be replaced by a heteroatom such as an oxygen atom, a group having a heteroatom, such as a carbonyl group, an $-SO_2-$ group, or an $-SO_3-$ group, a vinylidene group, or a combination thereof. In the cycloalkyl group and the cycloalkenyl group, one or more ethylene groups constituting the cycloalkane ring and the cycloalkene ring may be replaced by vinylene groups.

Examples of a preferred embodiment of the combination of $Ry_1$ to $Ry_3$ include an embodiment in which $Ry_1$ is a methyl group, an ethyl group, a vinyl group, an allyl group, or an aryl group, and $Ry_2$ and $Rx_3$ are bonded together to form the above-described cycloalkyl group or cycloalkenyl group, and an embodiment in which $Ry_1$ is a hydrogen atom, $Ry_2$ and $Ry_3$ are bonded to each other to form a ring having one or more vinylene groups in the ring structure, and at least one of the vinylene groups is present adjacent to a carbon atom to which a hydrogen atom represented by $Ry_1$ is bonded.

When $Ry_1$ to $Ry_3$ further have a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms).

The alkyl group that may have a substituent and that is represented by Xb may be, for example, a methyl group or a group represented by $-CH_2-R_{11}$. $R_{11}$ represents a halogen atom (such as a fluorine atom), a hydroxyl group, or a monovalent organic group, and examples thereof include alkyl groups having 5 or less carbon atoms and optionally substituted with a halogen atom, acyl groups having 5 or less carbon atoms and optionally substituted with a halogen atom, and alkoxy groups having 5 or less carbon atoms and optionally substituted with a halogen atom. $R_{11}$ is preferably an alkyl group having 3 or less carbon atoms, more preferably a methyl group. Xb is preferably a hydrogen atom, a fluorine atom, a methyl group, a trifluoromethyl group, or a hydroxymethyl group.

The divalent linking group represented by L may be an -Rt- group, a -CO- group, a - COO-Rt- group, a -COO-Rt-CO- group, an -Rt-CO- group, or an -O-Rt- group and is preferably an -Rt- group, a -CO- group, a -COO-Rt-CO- group, or an -Rt-CO- group. Rt represents a linear or branched alkylene group, a cycloalkylene group, or an aromatic ring group and is preferably an aromatic ring group. The aromatic ring group may have a substituent such as a halogen atom, a hydroxyl group, or an alkoxy group.

When any of the above-described groups in the formula (B) has a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms).

The repeating unit represented by the formula (B) is preferably an acid-decomposable (meth)acrylic acid tertiary ester-based repeating unit (a repeating unit in which Xb represents a hydrogen atom or a methyl group, and L represents a -CO- group), an acid-decomposable hydroxystyrene tertiary alkyl ether-based repeating unit (a repeating unit in which Xb represents a hydrogen atom or a methyl group, and L represents a phenyl group), or an acid-decomposable styrenecarboxylic acid tertiary ester-based repeating unit (a repeating unit in which Xb represents a hydrogen atom or a methyl group, and L represents an -Rt-CO- group (where Rt is an aromatic group)).

As specific examples of the repeating unit having an acid-decomposable group including an unsaturated bond, for example, the repeating units described in paragraphs [0067] to [0071] of WO2022/024928A can be referred to.

In the resin (A), the content of the repeating unit having an acid-decomposable group is preferably 15 mol% or more, more preferably 20 mol% or more, still more preferably 30 mol% or more relative to all the repeating units in the resin (A). The upper limit value of the content is preferably 90 mol% or less, more preferably 80 mol% or less, particularly preferably 70 mol% or less, most preferably 60 mol% or less.

The resin (A) may include another repeating unit other than the above-described repeating units.

Examples of the other repeating unit include at least one repeating unit selected from the group consisting of the following Group A.

Group A: Group consisting of the following repeating units (20) to (26)

(20) A repeating unit having an acid group, which will be described later

(21) A repeating unit having no acid-decomposable group and no acid group but having a fluorine atom or an iodine atom, which will be described later

(22) A repeating unit having a lactone group, a sultone group, or a carbonate group, which will be described later (23) A

repeating unit having a photoacid generating group, which will be described later
(24) A repeating unit represented by a formula (V-1) or a formula (V-2) below, which will be described later
(25) A repeating unit for reducing the mobility of the main chain

The repeating units represented by formulae (A) to (E), which will be described later, each correspond to (25) the repeating unit for reducing the mobility of the main chain. (26) A repeating unit that has an alicyclic hydrocarbon structure and that does not exhibit acid decomposability, which will be described later

The resin (A) preferably has an acid group and preferably includes a repeating unit having an acid group, as described later. The definition of the acid group will be described in a later part together with preferred embodiments of the repeating unit having an acid group.

When the resist composition is used as a resist composition for EUV exposure, the resin (A) preferably has at least one repeating unit selected from the group consisting of Group A above.

When the resist composition is used as a resist composition for EUV exposure, the resin (A) preferably includes at least one of a fluorine atom or an iodine atom. When the resin (A) includes both a fluorine atom and an iodine atom, the resin (A) may have one type of repeating unit including both a fluorine atom and an iodine atom, or the resin (A) may include two types of repeating units including a repeating unit having a fluorine atom and a repeating unit including an iodine atom.

When the resist composition is used as a resist composition for EUV exposure, the resin (A) also preferably has a repeating unit having an aromatic group.

<Repeating unit having acid group>

The resin (A) preferably has a repeating unit having an acid group.

The acid group is preferably an acid group having a pKa of 13 or less. The acid group preferably has an acid dissociation constant of 13 or less as described above, more preferably 3 to 13, still more preferably 5 to 10.

When the resin (A) has an acid group having a pKa of 13 or less, the content of the acid group in the resin (A) is not particularly limited but is often 0.2 to 6.0 mmol/g. In particular, the content of the acid group is preferably 0.8 to 6.0 mmol/g, more preferably 1.2 to 5.0 mmol/g, still more preferably 1.6 to 4.0 mmol/g. When the content of the acid group is within the above range, development suitably proceeds, a pattern to be formed has a good pattern shape, and thus high resolution is also achieved.

The acid group is preferably, for example, a carboxyl group, a hydroxyl group, a phenolic hydroxyl group, a fluorinated alcohol group (preferably a hexafluoroisopropanol group), a sulfonic acid group, a sulfonamide group, an isopropanol group, or the like and is more preferably a phenolic hydroxyl group.

The phenolic hydroxyl group means a hydroxyl group substituted on a ring member atom of an aromatic ring.

In the hexafluoroisopropanol group, one or more (preferably one or two) of the fluorine atoms may be substituted with groups other than fluorine atoms (such as alkoxycarbonyl groups). The acid group is also preferably $-C(CF_3)(OH)-CF_2-$ formed in this manner. Alternatively, one or more of the fluorine atoms may be substituted with groups other than fluorine atoms to form a ring including $-C(CF_3)(OH)-CF_2-$.

The repeating unit having an acid group is preferably a repeating unit different from the above-described repeating unit having a structure in which a polar group is protected with a leaving group that leaves due to the action of an acid and a repeating unit having a lactone group, a sultone group, or a carbonate group described later.

The repeating unit having an acid group may have a fluorine atom or an iodine atom.

In particular, the repeating unit having an acid group is preferably a repeating unit having a phenolic hydroxyl group from the viewpoint of providing a better effect of the present invention.

Examples of the repeating unit having a phenolic hydroxyl group include repeating units represented by a formula (1) below.

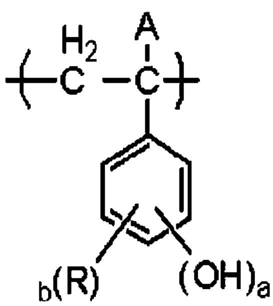

(1)

In the formula (1),

A represents a hydrogen atom, an alkyl group, a cycloalkyl group, a halogen atom, or a cyano group.
R represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group. When a plurality of R's are present, the plurality of R's may be the same or different. When a plurality of R's are present, the plurality of R's may together form a ring. R is preferably a hydrogen atom.
a represents an integer of 1 to 3.
b represents an integer of 0 to (5 - a).

Specific examples of the repeating unit having a phenolic hydroxyl group include the following repeating units. In the following formulae, a represents 1 or 2.

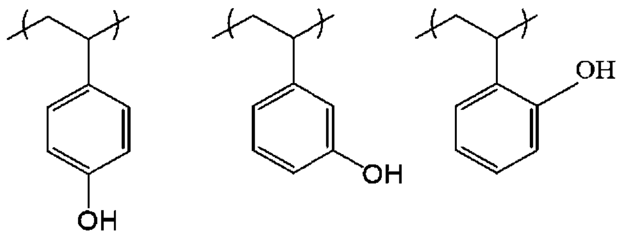

As other specific examples of the repeating unit having an acid group, for example, the repeating units described in paragraphs [0088] to [0089] and paragraphs [0103] to [0110] of WO2022/024928A can be referred to.
The content of the repeating unit having an acid group is preferably 10 mol% or more, more preferably 15 mol% or more relative to all the repeating units in the resin (A). The upper limit value of the content is preferably 70 mol% or less, more preferably 65 mol% or less, still more preferably 60 mol% or less relative to all the repeating units in the resin (A).

<Repeating unit having no acid-decomposable group and no acid group but having fluorine atom or iodine atom>

The resin (A) may have a repeating unit having a fluorine atom or an iodine atom (hereinafter also referred to as a unit X) in addition to the above-described <Repeating unit having acid-decomposable group> and <Repeating unit having acid group>. Herein, the <Repeating unit having fluorine atom or iodine atom> is preferably different from other repeating units belonging to Group A such as <Repeating unit having lactone group, sultone group, or carbonate group> and <Repeating unit having photoacid generating group>, which will be described later.
The unit X is preferably a repeating unit represented by a formula (C).

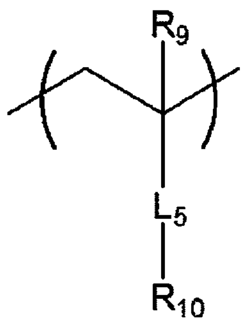

(C)

$L_5$ represents a single bond or an ester group.
$R_9$ represents a hydrogen atom or an alkyl group that may have a fluorine atom or an iodine atom.
$R_{10}$ represents a hydrogen atom, an alkyl group that may have a fluorine atom or an iodine atom, a cycloalkyl group that may have a fluorine atom or an iodine atom, an aryl group that may have a fluorine atom or an iodine atom, or a group provided by combining any of these groups.

Specific examples of the unit X are shown below.

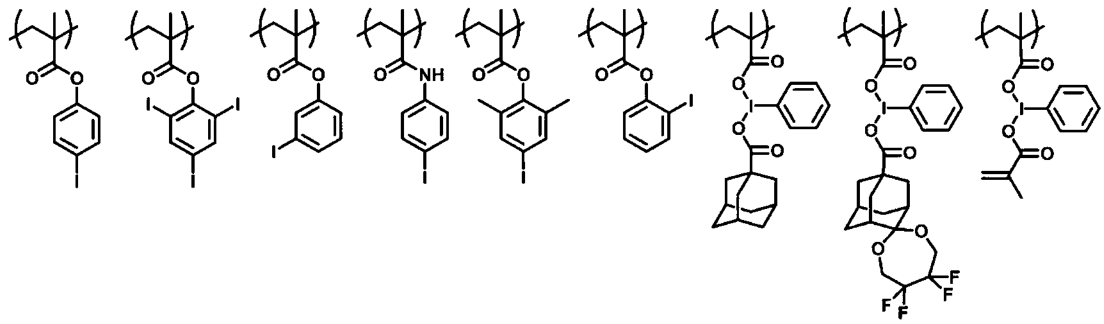

The content of the unit X is preferably 0 mol% or more, more preferably 5 mol% or more, still more preferably 10 mol% or more relative to all the repeating units in the resin (A). The upper limit value of the content is preferably 50 mol% or less, more preferably 45 mol% or less, still more preferably 40 mol% or less.

Among the repeating units in the resin (A), the total content of repeating units including at least one of a fluorine atom or an iodine atom is preferably 10 mol% or more, more preferably 20 mol% or more, still more preferably 30 mol% or more, particularly preferably 40 mol% or more relative to all the repeating units in the resin (A). The upper limit value is not particularly limited, but is, for example, 100 mol% or less.

Note that examples of the repeating units including at least one of a fluorine atom or an iodine atom include a repeating unit having a fluorine atom or an iodine atom and having an acid-decomposable group, a repeating unit having a fluorine atom or an iodine atom and having an acid group, and a repeating unit having a fluorine atom or an iodine atom.

<Repeating unit having lactone group, sultone group, or carbonate group>

The resin (A) may have a repeating unit having at least one selected from the group consisting of a lactone group, a sultone group, and a carbonate group (hereinafter also referred to as a "unit Y").

It is also preferable that the unit Y do not have an acid group such as a hydroxyl group or a hexafluoropropanol group.

The lactone group or the sultone group at least has a lactone structure or a sultone structure. The lactone structure or the sultone structure is preferably a five- to seven-membered lactone structure or a five- to seven-membered sultone structure. In particular, a five- to seven-membered lactone structure to which another ring structure is fused so as to form a bicyclo structure or a spiro structure, or a five- to seven-membered sultone structure to which another ring structure is fused so as to form a bicyclo structure or a spiro structure is more preferred.

The resin (A) preferably has a repeating unit having a lactone group or a sultone group provided by abstracting one or more hydrogen atoms from ring member atoms of a lactone structure represented by any one of formulae (LC1-1) to (LC1-21) below or a sultone structure represented by any one of formulae (SL1-1) to (SL1-3) below.

A lactone group or a sultone group may be directly bonded to the main chain. For example, ring member atoms of a lactone group or a sultone group may constitute the main chain of the resin (A).

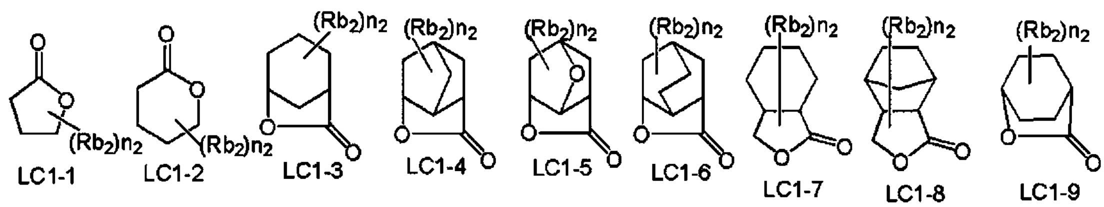

LC1-1 LC1-2 LC1-3 LC1-4 LC1-5 LC1-6 LC1-7 LC1-8 LC1-9

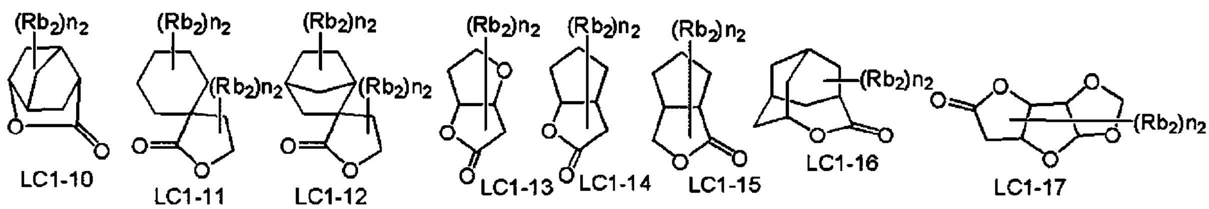

LC1-10 LC1-11 LC1-12 LC1-13 LC1-14 LC1-15 LC1-16 LC1-17

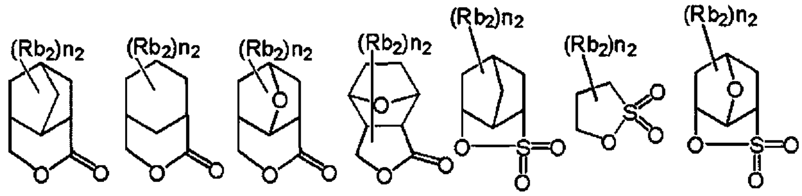

LC1-18 LC1-19 LC1-20 LC1-21 SL1-1 SL1-2 SL1-3

The lactone or sultone structural moiety may have a substituent ($Rb_2$). Preferred examples of the substituent ($Rb_2$) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxyl group, a halogen atom, a cyano group, and an acid-decomposable group. n2 represents an integer of 0 to 4. When n2 is 2 or more, a plurality of $Rb_2$'s present may be different and a plurality of $Rb_2$'s present may be bonded together to form a ring.

The repeating unit having a group having the lactone structure represented by any one of the formulae (LC1-1) to (LC1-21) or the sultone structure represented by any one of the formulae (SL1-1) to (SL1-3) is, for example, a repeating unit represented by a formula (AI) below.

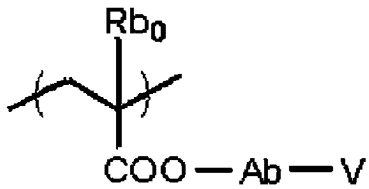

(AI)

In the formula (AI), $Rb_0$ represents a hydrogen atom, a halogen atom, or an alkyl group having 1 to 4 carbon atoms.

Preferred examples of the substituent that the alkyl group in $Rb_0$ may have include a hydroxyl group and a halogen atom.

Examples of the halogen atom in $Rb_0$ include a fluorine atom, a chlorine atom, a bromine atom, and an iodine atom. $Rb_0$ is preferably a hydrogen atom or a methyl group.

Ab represents a single bond, an alkylene group, a divalent linking group having a monocyclic or polycyclic alicyclic hydrocarbon structure, an ether group, an ester group, a carbonyl group, a carboxyl group, or a divalent group provided by combining any of these groups. In particular, a single bond or a linking group represented by $-Ab_1-CO_2-$ is preferred. $Ab_1$ is a linear or branched alkylene group or a monocyclic or polycyclic cycloalkylene group and is preferably a methylene group, an ethylene group, a cyclohexylene group, an adamantylene group, or a norbornylene group.

V represents a group provided by abstracting one hydrogen atom from a ring member atom of the lactone structure represented by any one of the formulae (LC1-1) to (LC1-21) or a group provided by abstracting one hydrogen atom from a ring member atom of the sultone structure represented by any one of the formulae (SL1-1) to (SL1-3).

When the repeating unit having a lactone group or a sultone group has an optical isomer, any optical isomer may be used. A single optical isomer may be used alone, or a mixture of a plurality of optical isomers may be used. When a single optical isomer is mainly used, its optical purity (ee) is preferably 90 or more, more preferably 95 or more.

The carbonate group is preferably a cyclic carbonate group.

As the repeating unit having a cyclic carbonate group, for example, the repeating units described in paragraphs [0127] to [0133] of WO2022/024928A can be referred to.

The content of the unit Y is preferably 1 mol% or more, more preferably 5 mol% or more, still more preferably 10 mol% or more relative to all the repeating units in the resin (A). The upper limit value of the content is preferably 85 mol% or less, more preferably 80 mol% or less, still more preferably 70 mol% or less, particularly preferably 60 mol% or less relative to all the repeating units in the resin (A).

<Repeating unit having photoacid generating group>

The resin (A) may be integrated with a photoacid generator as described later. That is, the resin (A) and the photoacid generator may be integrated into one structure. In the case where the photoacid generator is integrated with the resin (A), the resin (A) includes a repeating unit having a photoacid generating group. Examples of such a repeating unit include repeating units represented by a formula (4) below.

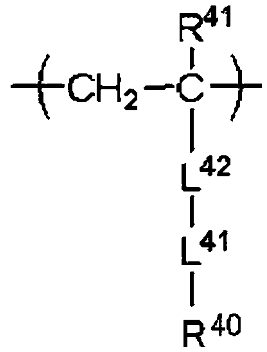

(4)

$R^{41}$ represents a hydrogen atom or a methyl group. $L^{41}$ represents a single bond or a divalent linking group. $L^{42}$ represents a divalent linking group. $R^{40}$ represents a photoacid generating group.
The photoacid generating group represented by $R^{40}$ is preferably a group obtained by removing one hydrogen atom from a photoacid generator described later, more preferably a group obtained by removing one hydrogen atom of an anionic moiety in a photoacid generator having an onium salt structure described later.
Examples of the repeating unit having a photoacid generating group include the following repeating units.

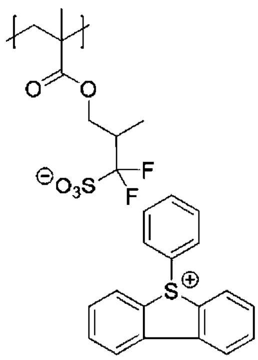

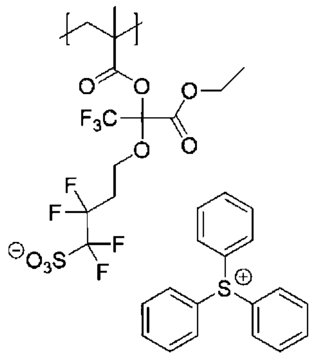

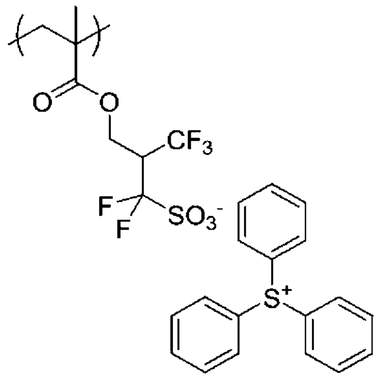

Examples of the repeating units represented by the formula (4) further include the repeating units described in paragraphs [0094] to [0105] of JP2014-041327A and the repeating units described in paragraph [0094] of WO2018/193954A.
The content of the repeating unit having a photoacid generating group is preferably 1 mol% or more, more preferably 5 mol% or more relative to all the repeating units in the resin (A). The upper limit value of the content is preferably 40 mol% or less, more preferably 35 mol% or less, still more preferably 30 mol% or less.

<Repeating unit represented by formula (V-1) or formula (V-2) below>

The resin (A) may have a repeating unit represented by a formula (V-1) below or a formula (V-2) below.
The repeating units represented by the formula (V-1) below and the formula (V-2) below are preferably repeating units different from the repeating units described above.

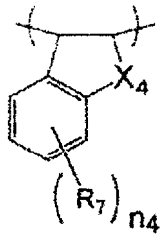

(V-1)

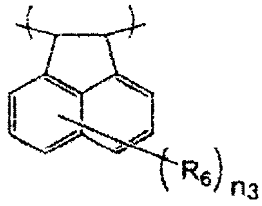

(V-2)

In the formulae,

$R_6$ and $R_7$ each independently represent a hydrogen atom, a hydroxyl group, an alkyl group, an alkoxy group, an acyloxy group, a cyano group, a nitro group, an amino group, a halogen atom, an ester group (-OCOR or -COOR: R represents an alkyl group or fluorinated alkyl group having 1 to 6 carbon atoms), or a carboxyl group. The alkyl group is preferably a linear, branched, or cyclic alkyl group having 1 to 10 carbon atoms.
$n_3$ represents an integer of 0 to 6.
$n_4$ represents an integer of 0 to 4.
$X^4$ is a methylene group, an oxygen atom, or a sulfur atom.

Examples of the repeating unit represented by the formula (V-1) or (V-2) are as follows.
Examples of the repeating unit represented by the formula (V-1) or (V-2) include the repeating units described in paragraph [0100] of WO2018/193954A.

<Repeating unit for reducing mobility of main chain>

The resin (A) preferably has a high glass transition temperature (Tg) from the viewpoint that excessive diffusion of the generated acid or pattern collapse during development can be suppressed. Tg is preferably higher than 90°C, more preferably higher than 100°C, still more preferably higher than 110°C, particularly preferably higher than 125°C. Tg is preferably 400°C or lower, more preferably 350°C or lower from the viewpoint of a high dissolution rate in a developer.
In the present specification, the glass transition temperature (Tg) of a polymer such as the resin (A) (hereinafter also referred to as "Tg of repeating units") is calculated by the following method. First, Tg's of homopolymers composed only of the repeating units included in the polymer are calculated by the Bicerano method. Next, the mass ratios (%) of the repeating units relative to all the repeating units in the polymer are calculated. Next, Tg's at the mass ratios are calculated using the Fox equation (described in, for example, Materials Letters 62 (2008) 3152), and the sum total of the Tg's is defined as the Tg (°C) of the polymer.
The Bicerano method is described in, for example, Prediction of polymer properties, Marcel Dekker Inc., New York (1993). The calculation of Tg by the Bicerano method can be performed using software for estimating physical properties of polymers, MDL Polymer (MDL Information Systems, Inc.).
In order to increase Tg of the resin (A) (preferably, to make Tg higher than 90°C), the mobility of the main chain of the resin (A) is preferably reduced. Examples of the method for reducing the mobility of the main chain of the resin (A) include the following methods (a) to (e):

(a) Introduction of a bulky substituent into the main chain
(b) Introduction of a plurality of substituents into the main chain
(c) Introduction of a substituent that induces an interaction between molecules of the resin (A) into the vicinity of the main chain
(d) Formation of the main chain with a ring structure
(e) Linking of a ring structure to the main chain

The resin (A) preferably has a repeating unit whose homopolymer has a Tg of 130°C or higher.
The type of the repeating unit whose homopolymer has a Tg of 130°C or higher is not particularly limited as long as it is a repeating unit whose homopolymer has a Tg of 130°C or higher as calculated by the Bicerano method. Note that repeating units represented by a formula (A) to a formula (E) described below may each correspond to the repeating unit whose homopolymer has a Tg of 130°C or higher, although it depends on the types of functional groups in the repeating units.

(Repeating unit represented by formula (A))

An example of specific means for achieving (a) above is a method of introducing a repeating unit represented by a formula (A) into the resin (A).

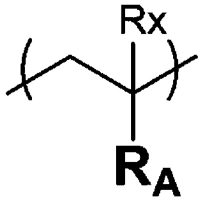

(A)

In the formula (A), $R_A$ represents a group having a polycyclic structure. $R_x$ represents a hydrogen atom, a methyl group, or an ethyl group. The group having a polycyclic structure is a group having a plurality of ring structures, and the plurality of ring structures may be fused together or may not be fused together.
Specific examples of the repeating unit represented by the formula (A) include those described in paragraphs [0107] to [0119] of WO2018/193954A.

(Repeating unit represented by formula (B))

An example of specific means for achieving (b) above is a method of introducing a repeating unit represented by a formula (B) into the resin (A).

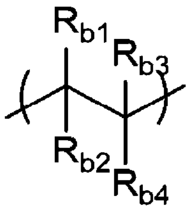

(B)

In the formula (B), $R_{b1}$ to $R_{b4}$ each independently represent a hydrogen atom or an organic group, and at least two or more of $R_{b1}$ to $R_{b4}$ represent organic groups.
When at least one of the organic groups is a group whose ring structure is directly linked to the main chain in the repeating unit, the types of other organic groups are not particularly limited.
When none of the organic groups is a group whose ring structure is directly linked to the main chain in the repeating unit, at least two or more of the organic groups are substituents in which the number of constituent atoms excluding hydrogen atoms is 3 or more.
Specific examples of the repeating unit represented by the formula (B) include those described in paragraphs [0113] to [0115] of WO2018/193954A.

(Repeating unit represented by formula (C))

An example of specific means for achieving (c) above is a method of introducing a repeating unit represented by a formula (C) into the resin (A).

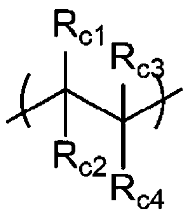

(C)

In the formula (C), $R_{c1}$ to $R_{c4}$ each independently represent a hydrogen atom or an organic group, and at least one of $R_{c1}$ to $R_{c4}$ is a group having a hydrogen-bond-forming hydrogen atom positioned within three atoms from a carbon atom in the main chain. In particular, from the viewpoint of inducing the interaction between the main chains of molecules of the resin (A), it is preferable to have a hydrogen-bond-forming hydrogen atom positioned within two atoms (closer to the main

chain).

Specific examples of the repeating unit represented by the formula (C) include those described in paragraphs [0119] to [0121] of WO2018/193954A.

(Repeating unit represented by formula (D))

An example of specific means for achieving (d) above is a method of introducing a repeating unit represented by a formula (D) into the resin (A).

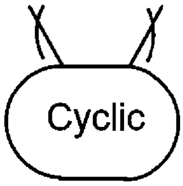

(D)

In the formula (D), "Cyclic" represents a group that forms the main chain with a ring structure. The number of atoms constituting the ring is not particularly limited.

Specific examples of the repeating unit represented by the formula (D) include those described in paragraphs [0126] and [0127] of WO2018/193954A.

(Repeating unit represented by formula (E))

An example of specific means for achieving (e) above is a method of introducing a repeating unit represented by a formula (E) into the resin (A).

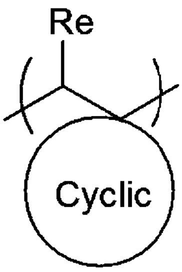

(E)

In the formula (E), each Re independently represents a hydrogen atom or an organic group. The organic group may be, for example, an alkyl group, cycloalkyl group, aryl group, aralkyl group, or alkenyl group that may have a substituent.

"Cyclic" is a cyclic group including a carbon atom of the main chain. The number of atoms included in the cyclic group is not particularly limited.

Specific examples of the repeating unit represented by the formula (E) include those described in paragraphs [0131] to [0133] of WO2018/193954A.

<Repeating unit that has alicyclic hydrocarbon structure and that does not exhibit acid decomposability>

The resin (A) may have a repeating unit that has an alicyclic hydrocarbon structure and that does not exhibit acid decomposability. Examples of such a repeating unit include repeating units derived from 1-adamantyl (meth)acrylate, diamantyl (meth)acrylate, tricyclodecanyl (meth)acrylate, or cyclohexyl (meth)acrylate.

It is also preferable that the alicyclic hydrocarbon structure be substituted with a hydroxyl group or a cyano group from the viewpoint of improving adhesiveness to a substrate and affinity for a developer.

Specific examples of the repeating unit having the alicyclic hydrocarbon structure having a hydroxyl group or a cyano group include those described in paragraphs [0081] to [0084] of JP2014-098921A.

<Other repeating units>

Furthermore, the resin (A) may have a repeating unit other than the above-described repeating units.

For example, the resin (A) may have a repeating unit selected from the group consisting of a repeating unit having

an oxathiane ring group, a repeating unit having an oxazolone ring group, a repeating unit having a dioxane ring group, and a repeating unit having a hydantoin ring group.

Examples of such repeating units are as follows.

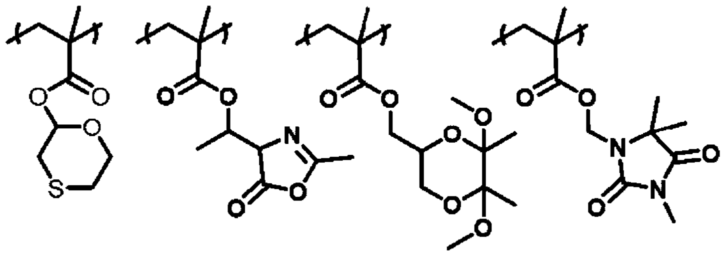

The resin (A) may have, in addition to the foregoing repeating structural units, various repeating structural units for the purpose of adjusting, for example, dry etching resistance, suitability for a standard developer, adhesiveness to a substrate, resist profile, resolving power, heat resistance, and sensitivity.

The resin (A) can be synthesized by an ordinary method (for example, radical polymerization).

The resin (A) preferably has a weight-average molecular weight of 30,000 or less, more preferably 1,000 to 30,000, still more preferably 3,000 to 30,000, particularly preferably 5,000 to 15,000 in terms of polystyrene as determined by the GPC method.

The dispersity (molecular weight distribution) of the resin (A) is preferably 1 to 5, more preferably 1 to 3, still more preferably 1.2 to 3.0, particularly preferably 1.2 to 2.0. The smaller the dispersity, the better the resolution and the resist profile, the smoother the sidewalls of the resist pattern, and the better the roughness performance.

In the resist composition, the lower limit value of the content of the resin (A) is preferably 35.0 mass% or more, more preferably 40.0 mass% or more, still more preferably 45.0 mass% or more relative to the total solid contents of the resist composition. The upper limit value of the content of the resin (A) is preferably 99.9 mass%% or less, more preferably 90.0 mass% or less, still more preferably 80.0 mass%% or less relative to the total solid contents of the resist composition.

Such resins (A) may be used alone or in combination of two or more thereof.

One of such resins (A) may be used alone, or two or more of such resins (A) may be used. When two or more resins (A) are used, the total content thereof is preferably within the above preferred content range.

[Boron-containing compound]

The resist composition includes a boron-containing compound.

The boron-containing compound is not particularly limited as long as it is a compound including a boron atom, but is more preferably a compound (salt compound) having a borate anion from the viewpoint of providing a better effect of the present invention.

In particular, the compound having a borate anion is preferably an onium salt compound, more preferably a sulfonium salt compound, an iodonium salt compound, a quaternary ammonium salt compound, or a quaternary phosphonium salt compound.

The compound having a borate anion may be a low-molecular-weight compound or a high-molecular-weight compound, but is preferably a low-molecular-weight compound.

The molecular weight of the compound having a borate anion is preferably 1,200 or less, more preferably 1,000 or less, still more preferably 800 or less. The lower limit is not particularly limited, but is preferably 400 or more.

In particular, the compound having a borate anion is more preferably a compound represented by a formula (3) below.

<Compound represented by formula (3) below>

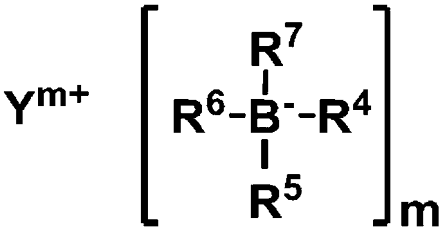

(3)

In the formula (3), $R^4$ to $R^7$ each independently represent a substituent.

The substituents represented by $R^4$ to $R^7$ are not particularly limited, and examples thereof include groups described as examples of a substituent T.

(Substituent T)

Examples of the substituent T include halogen atoms such as a fluorine atom, a chlorine atom, a bromine atom, and an iodine atom; alkoxy groups such as a methoxy group, an ethoxy group, and a tert-butoxy group; aryloxy groups such as a phenoxy group and a p-tolyloxy group; alkoxycarbonyl groups such as a methoxycarbonyl group, a butoxycarbonyl group, and a phenoxycarbonyl group; acyloxy groups such as an acetoxy group, a propionyloxy group, and a benzoyloxy group; acyl groups such as an acetyl group, a benzoyl group, an isobutyryl group, an acryloyl group, a methacryloyl group, and a methoxalyl group; alkylsulfanyl groups such as a methylsulfanyl group and a tert-butylsulfanyl group; arylsulfanyl groups such as a phenylsulfanyl group and a p-tolylsulfanyl group; alkyl groups; aryl groups; heteroaryl groups; a hydroxyl group; a carboxy group; a formyl group; a sulfo group; a cyano group; alkylaminocarbonyl groups; arylaminocarbonyl groups; a sulfonamide group; a silyl group; an amino group; an imino group; monoalkylamino groups; dialkylamino groups; arylamino groups; a nitro group; a formyl group; a thiol group; a thioether group; and combinations of the foregoing.

The alkyl groups may be linear, branched, or cyclic. When the alkyl groups have a fluorine atom, the alkyl groups may be perfluoroalkyl groups.

The aryl groups and the heteroaryl groups may be monocyclic or polycyclic.

Examples of the substituents represented by $R^4$ to $R^7$ include, in particular, an aryl group that may have a substituent, a heteroaryl group that may have a substituent, a halogen atom (preferably a fluorine atom), and a cyano group.

The aryl group and the heteroarysl group may be monocyclic or polycyclic.

The number of carbon atoms of the aryl group is, for example, preferably 6 to 20, more preferably 6 to 10. A specific example of the aryl group is preferably a phenyl group or a naphthyl group, more preferably a phenyl group.

Examples of the heteroatom included in the heteroaryl group include, but are not particularly limited to, an oxygen atom, a nitrogen atom, and a sulfur atom.

The number of carbon atoms of the heteroaryl group is, for example, preferably 3 to 15, more preferably 3 to 10. Specific examples of the ring constituting the heteroaryl group include a pyrrole ring, a furan ring, a thiophene ring, an indole ring, a benzofuran ring, and a benzothiophene ring.

Examples of the substituent that the aryl group and the heteroaryl group may have include, but are not particularly limited to, the groups described as examples of the above substituent T.

Examples of the substituent include alkyl groups (such as linear alkyl groups having 1 to 15 carbon atoms, branched alkyl groups having 3 to 15 carbon atoms, and cyclic alkyl groups having 3 to 15 carbon atoms); aryl groups (that may be monocyclic or polycyclic and that preferably have 6 to 20 carbon atoms, more preferably 6 to 10 carbon atoms) heteroaryl groups (that may be monocyclic or polycyclic and that preferably have 3 to 15 carbon atoms, more preferably 3 to 10 carbon atoms); alkoxy groups (such as linear alkoxy groups having 1 to 15 carbon atoms, branched alkoxy groups having 1 to 15 carbon atoms, and cyclic alkoxy groups having 3 to 15 carbon atoms); halogen atoms (such as a fluorine atom, a chlorine atom, and an iodine atom); a hydroxyl group; a cyano group; and a nitro group.

The substituent may further have another substituent, and, for example, the alkyl group may have a halogen atom as a substituent to serve as an alkyl halide group such as a trifluoromethyl group.

Among $R^4$ to $R^7$, adjacent groups may be bonded together to form a ring.

The ring formed by linking adjacent groups together is not particularly limited, and may be monocyclic or polycyclic. The ring may include, as a ring member atom, a heteroatom such as an oxygen atom, a nitrogen atom, or a sulfur atom, and/or carbonyl carbon.

The ring may be an aromatic ring or an alicyclic ring.

$Y^{m+}$ represents an organic cation or an inorganic cation having an m-valent positive charge.

m represents an integer of 1 or more.
m is not particularly limited as long as it is an integer of 1 or more, and, for example, m is preferably 1 to 3, more preferably 1 to 2, still more preferably 1.

Examples of the organic cation represented by $Y^{m+}$ include, but are not particularly limited to, an organic cation represented by a formula (ZaI) below (cation (ZaI)), an organic cation represented by a formula (ZaII) below (cation (ZaII)), and quaternary ammonium cations and quaternary phosphonium cations such as an organic cation represented by a formula (ZaIII) below (cation (ZaIII)) and an organic cation represented by a formula (ZaIV) below (cation (ZaIV)).

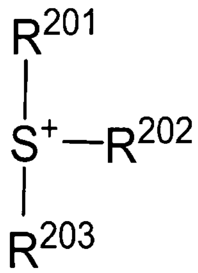

(ZaI)

$R^{204}$-$I^+$-$R^{205}$ (ZaII)

In the formula (ZaI), $R^{201}$ to $R^{203}$ each independently represent an organic group.
The number of carbon atoms of the organic groups represented by $R^{201}$ to $R^{203}$ is preferably 1 to 30, more preferably 1 to 20. Furthermore, two of the organic groups represented by $R^{201}$ to $R^{203}$ may be bonded together to form a ring structure, and the formed ring may include an oxygen atom, a sulfur atom, an ester group, an amide group, or a carbonyl group. Examples of the group formed by bonding two of the organic groups represented by $R^{201}$ to $R^{203}$ together in the ring structure include alkylene groups (such as a butylene group and a pentylene group) and $-CH_2-CH_2-O-CH_2-CH_2-$.
In particular, the cation (ZaI) described above is preferably a cation (ZaI-1), a cation (ZaI-2), an organic cation represented by a formula (ZaI-3b) (cation (ZaI -3b)), or an organic cation represented by a formula (ZaI-4b) (cation (ZaI -4b)).
First, the cation (ZaI-1) will be described.
In the cation (ZaI-1), at least one of $R^{201}$ to $R^{203}$ represents a monovalent aromatic ring group that may have a substituent. All of $R^{201}$ to $R^{203}$ may be monovalent aromatic ring groups, or some of $R^{201}$ to $R^{203}$ may be monovalent aromatic ring groups and the remainder may be an alkyl group that may have a substituent.
Alternatively, one of $R^{201}$ to $R^{203}$ may be a monovalent aromatic ring group, the other two of $R^{201}$ to $R^{201}$ may be bonded together to form a ring structure, and the formed ring may include an oxygen atom, a sulfur atom, an ester group, an amide group, or a carbonyl group. Examples of the group formed by bonding two of $R^{201}$ to $R^{203}$ together include alkylene groups in which one or more methylene groups may be substituted with an oxygen atom, a sulfur atom, an ester group, an amide group, and/or a carbonyl group (such as a butylene group, a pentylene group, and $-CH_2-CH_2-O-CH_2-CH_2-$).
In the cation (ZaI-1), examples of the monovalent aromatic ring group include an aryl group and a heteroaryl group.
The aryl group and the heteroarysl group may be monocyclic or polycyclic.
The number of carbon atoms of the aryl group is, for example, preferably 6 to 20, more preferably 6 to 10. A specific example of the aryl group is preferably a phenyl group or a naphthyl group, more preferably a phenyl group.
Examples of the heteroatom included in the heteroaryl group include, but are not particularly limited to, an oxygen atom, a nitrogen atom, and a sulfur atom.
The number of carbon atoms of the heteroaryl group is, for example, preferably 3 to 15, more preferably 3 to 10. Specific examples of the ring constituting the heteroaryl group include a pyrrole ring, a furan ring, a thiophene ring, an indole ring, a benzofuran ring, and a benzothiophene ring.
In the cation (ZaI-1), when two or more of $R^{201}$ to $R^{203}$ are monovalent aromatic ring groups, the two or more monovalent aromatic ring groups may be the same or different.
In the cation (ZaI-1), the alkyl group is preferably a linear alkyl group having 1 to 15 carbon atoms, a branched alkyl group having 3 to 15 carbon atoms, or a cycloalkyl group having 3 to 15 carbon atoms, and examples thereof include a methyl group, an ethyl group, a propyl group, a n-butyl group, a sec-butyl group, a t-butyl group, a cyclopropyl group, a cyclobutyl group, and a cyclohexyl group.
Examples of the substituent that the monovalent aromatic ring group and the alkyl group in $R^{201}$ to $R^{203}$ may have

may each independently include alkyl groups (such as linear alkyl groups having 1 to 15 carbon atoms, branched alkyl groups having 3 to 15 carbon atoms, and cyclic alkyl groups having 3 to 15 carbon atoms); aryl groups (that may be monocyclic or polycyclic and that preferably have 6 to 20 carbon atoms, more preferably 6 to 10 carbon atoms) heteroaryl groups (that may be monocyclic or polycyclic and that preferably have 3 to 15 carbon atoms, more preferably 3 to 10 carbon atoms); alkoxy groups (such as linear alkoxy groups having 1 to 15 carbon atoms, branched alkoxy groups having 1 to 15 carbon atoms, and cyclic alkoxy groups having 3 to 15 carbon atoms); halogen atoms (such as a fluorine atom, a chlorine atom, and an iodine atom); a hydroxyl group; a cyano group; and a nitro group.

The substituent may further have another substituent, and, for example, the alkyl group may have a halogen atom as a substituent to serve as an alkyl halide group such as a trifluoromethyl group.

Examples of the cation (ZaI-1) include a triarylsulfonium cation that may have a substituent, a diarylalkylsulfonium cation that may have a substituent, and an aryldialkylsulfonium cation that may have a substituent, and among these, a triarylsulfonium cation that may have a substituent is preferable from the viewpoint of providing a better effect of the present invention.

Next, the cation (ZaI-2) will be described.

In the cation (ZaI-2), $R^{201}$ to $R^{203}$ each independently represent an organic group having no aromatic ring.

The organic group having no aromatic ring and represented by $R^{201}$ to $R^{203}$ generally has 1 to 30 carbon atoms and preferably has 1 to 20 carbon atoms.

The organic group having no aromatic ring and represented by $R^{201}$ to $R^{203}$ is preferably an alkyl group, an allyl group, a vinyl group, a linear or branched 2-oxoalkyl group, a 2-oxocycloalkyl group, or an alkoxycarbonylmethyl group, more preferably a linear or branched 2-oxoalkyl group.

In the cation (ZaI-2), examples of the alkyl group include linear alkyl groups having 1 to 15 carbon atoms (preferably 1 to 10 carbon atoms), branched alkyl groups having 3 to 15 carbon atoms (preferably 3 to 10 carbon atoms), and cyclic alkyl groups that have 3 to 15 carbon atoms (preferably 3 to 10 carbon atoms) (and that may be monocyclic or polycyclic).

In the cation (ZaI-2), the number of carbon atoms of the linear or branched 2-oxoalkyl group is, for example, preferably 2 to 15, more preferably 2 to 10.

In the cation (ZaI-2), the number of carbon atoms of the 2-oxocycloalkyl group is preferably 4 to 15, more preferably 4 to 10.

In the cation (ZaI-2), the number of carbon atoms of the alkoxycarbonylmethyl group is, for example, preferably 3 to 15, more preferably 3 to 10.

The above groups represented by $R^{201}$ to $R^{203}$ may further have a substituent. Examples of the substituent include halogen atoms, alkoxy groups (for example, having 1 to 5 carbon atoms), a hydroxyl group, a cyano group, and a nitro group.

Next, the cation (ZaI-3b) will be described.

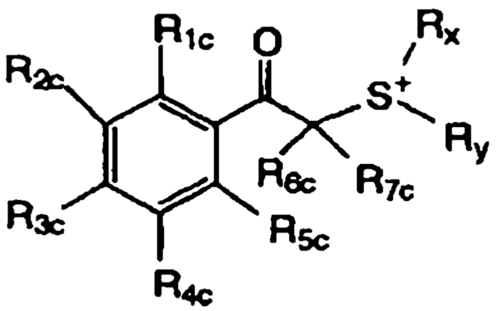

(ZaI-3b)

In the formula (ZaI-3b), $R_{1c}$ to $R_{5c}$ each independently represent a hydrogen atom, an alkyl group, a monovalent aromatic ring group, an alkoxy group, an aryloxy group, an alkoxycarbonyl group, an alkylcarbonyloxy group, a halogen atom, a hydroxyl group, a nitro group, an alkylthio group, or an arylthio group.

The alkyl group represented by $R_{1c}$ to $R_{5c}$ is preferably a linear alkyl group having 1 to 15 carbon atoms, a branched alkyl group having 3 to 15 carbon atoms, or a cyclic alkyl group having 3 to 15 carbon atoms.

The alkyl group may further have a substituent. Examples of the substituent include halogen atoms, alkoxy groups (for example, having 1 to 5 carbon atoms), a hydroxyl group, a cyano group, and a nitro group.

The alkyl moieties in the alkoxy group, the alkoxycarbonyloxy group, and the alkylthio group represented by $R_{1c}$ to $R_{5c}$ are each preferably a linear alkyl group having 1 to 15 carbon atoms, a branched alkyl group having 3 to 15 carbon atoms, or a cyclic alkyl group having 3 to 15 carbon atoms.

Each of the alkyl moieties in the alkoxy group, the alkoxycarbonyloxy group, and the alkylthio group represented by $R_{1c}$ to $R_{5c}$ may further have a substituent. Examples of the substituent include halogen atoms, alkoxy groups (for

example, having 1 to 5 carbon atoms), a hydroxyl group, a cyano group, and a nitro group.

The monovalent aromatic ring group represented by $R_{1c}$ to $R_{5c}$ may be an aryl group or a heteroaryl group.

The aryl group and the heteroarysl group may be monocyclic or polycyclic.

The number of carbon atoms of the aryl group is, for example, preferably 6 to 20, more preferably 6 to 10. A specific example of the aryl group is preferably a phenyl group or a naphthyl group, more preferably a phenyl group.

Examples of the heteroatom included in the heteroaryl group include, but are not particularly limited to, an oxygen atom, a nitrogen atom, and a sulfur atom.

The number of carbon atoms of the heteroaryl group is, for example, preferably 3 to 15, more preferably 3 to 10. Specific examples of the ring constituting the heteroaryl group include a pyrrole ring, a furan ring, a thiophene ring, an indole ring, a benzofuran ring, and a benzothiophene ring.

Examples of the substituent that the aryl group and the heteroaryl group may have include, but are not particularly limited to, the groups described as examples of the above substituent T.

Examples of the substituent include alkyl groups (such as linear alkyl groups having 1 to 15 carbon atoms, branched alkyl groups having 3 to 15 carbon atoms, and cyclic alkyl groups having 3 to 15 carbon atoms); aryl groups (that may be monocyclic or polycyclic and that preferably have 6 to 20 carbon atoms, more preferably 6 to 10 carbon atoms) heteroaryl groups (that may be monocyclic or polycyclic and that preferably have 3 to 15 carbon atoms, more preferably 3 to 10 carbon atoms); alkoxy groups (such as linear alkoxy groups having 1 to 15 carbon atoms, branched alkoxy groups having 1 to 15 carbon atoms, and cyclic alkoxy groups having 3 to 15 carbon atoms); halogen atoms (such as a fluorine atom, a chlorine atom, and an iodine atom); a hydroxyl group; a cyano group; and a nitro group.

The substituent may further have another substituent, and, for example, the alkyl group may have a halogen atom as a substituent to serve as an alkyl halide group such as a trifluoromethyl group.

Each of the aryl moieties in the aryloxy group and the arylthio group represented by $R_{1c}$ to $R_{5c}$ may each be monocyclic or polycyclic.

The number of carbon atoms of the aryl moiety is, for example, preferably 6 to 20, more preferably 6 to 10. A specific example of the aryl moiety is preferably a phenyl group or a naphthyl group, more preferably a phenyl group.

Each of the aryl moieties in the aryloxy group and the arylthio group represented by $R_{1c}$ to $R_{5c}$ may further have a substituent. Examples of the substituent include the same substituents as those that the monovalent aromatic ring group represented by $R_{1c}$ to $R_{5c}$ can have, and preferred embodiments thereof are also the same.

In the formula (ZaI-3b), $R_{6c}$ and $R_{7c}$ each independently represent a hydrogen atom, an alkyl group, a halogen atom, a cyano group, or an aryl group.

The alkyl groups represented by $R_{6c}$ and $R_{7c}$ have the same definition and preferred embodiments as in the alkyl groups represented by $R_{1c}$ to $R_{5c}$ in the formula (ZaI-3b).

The aryl groups represented by $R_{6c}$ and $R_{7c}$ have the same definition and preferred embodiments as in the aryl groups represented by $R_{1c}$ to $R_{5c}$ in the formula (ZaI-3b).

In the formula (ZaI-3b), $R_x$ and $R_y$ each independently represent an alkyl group, a linear or branched 2-oxoalkyl group, a 2-oxocycloalkyl group, an alkoxycarbonylalkyl group, an allyl group, or a vinyl group.

The alkyl groups represented by $R_x$ and $R_y$ have the same definition and preferred embodiments as in the alkyl groups represented by $R_{1c}$ to $R_{5c}$ in the formula (ZaI-3b).

The number of carbon atoms of the linear or branched 2-oxoalkyl group represented by $R_x$ and $R_y$ is, for example, preferably 2 to 15, more preferably 2 to 10.

The number of carbon atoms of the 2-oxocycloalkyl group represented by $R_x$ and $R_y$ is preferably 4 to 15, more preferably 4 to 10.

The number of carbon atoms of the alkoxycarbonylmethyl group represented by $R_x$ and $R_y$ is, for example, preferably 3 to 15, more preferably 3 to 10.

The above groups represented by $R_x$ and $R_y$ may further have a substituent. Examples of the substituent include halogen atoms, alkoxy groups (for example, having 1 to 5 carbon atoms), a hydroxyl group, a cyano group, and a nitro group.

In the formula (ZaI-3b), any two or more of $R_{1c}$ to $R_{5c}$, $R_{5c}$ and $R_{6c}$, $R_{6c}$ and $R_{7c}$, $R_{5c}$ and $R_x$, and $R_x$ and $R_y$ may be individually bonded together to form rings, and the formed rings may each independently include an oxygen atom, a sulfur atom, a ketone group, an ester bond, or an amide bond.

Examples of the rings include an aromatic or non-aromatic monocyclic (preferably three-to ten-membered, more preferably four- to eight-membered, still more preferably five- or six-membered) hydrocarbon ring, an aromatic or non-aromatic monocyclic (preferably three- to ten-membered, more preferably four- to eight-membered, still more preferably five- or six-membered) heterocycle), and a polycyclic fused ring provided by combining two or more of these monocyclic rings.

Examples of the groups formed by bonding together any two or more of $R_{1c}$ to $R_{5c}$, $R_{6c}$ and $R_{7c}$, and $R_x$ and $R_y$ include alkylene groups such as a butylene group and a pentylene group. A methylene group in such an alkylene group may be substituted with a heteroatom such as an oxygen atom.

The groups formed by bonding together $R_{5c}$ and $R_{6c}$, and $R_{5c}$ and $R_x$ are each preferably a single bond or an alkylene group. Examples of the alkylene group include a methylene group and an ethylene group.
Next, the cation (ZaI-4b) will be described.

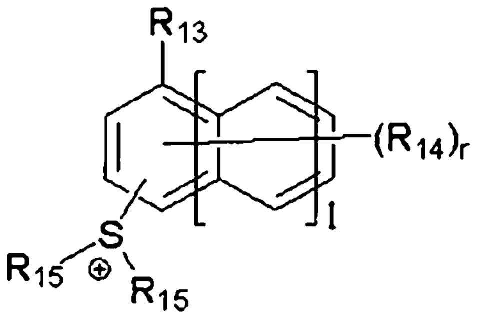

(ZaI-4b)

In the formula (ZaI-4b), l represents an integer of 0 to 2, and r represents an integer of 0 to 8.
$R_{13}$ represents a hydrogen atom, a fluorine atom, a hydroxyl group, an alkyl group, an alkoxy group, or an alkoxycarbonyl group.
The alkyl group represented by $R_{13}$ is preferably a linear alkyl group having 1 to 15 carbon atoms, a branched alkyl group having 3 to 15 carbon atoms, or a cyclic alkyl group having 3 to 15 carbon atoms.
The alkyl group may further have a substituent. Examples of the substituent include halogen atoms, alkoxy groups (for example, having 1 to 5 carbon atoms), a hydroxyl group, a cyano group, and a nitro group.
The alkyl moieties in the alkoxy group and the alkoxycarbonyl group represented by $R_{13}$ are each preferably a linear alkyl group having 1 to 15 carbon atoms, a branched alkyl group having 3 to 15 carbon atoms, or a cyclic alkyl group having 3 to 15 carbon atoms.
Each of the alkyl moieties in the alkoxy group and the alkoxycarbonyl group represented by $R_{13}$ may further have a substituent. Examples of the substituent include halogen atoms, alkoxy groups (for example, having 1 to 5 carbon atoms), a hydroxyl group, a cyano group, and a nitro group.
$R_{14}$ represents a hydroxyl group, an alkyl group, an alkoxy group, an alkoxycarbonyl group, an alkylcarbonyl group, or an alkylsulfonyl group.
The alkyl group represented by $R_{14}$ is preferably a linear alkyl group having 1 to 10 carbon atoms, a branched alkyl group having 3 to 10 carbon atoms, or a cyclic alkyl group having 3 to 10 carbon atoms.
The alkyl group may further have a substituent. Examples of the substituent include halogen atoms, alkoxy groups (for example, having 1 to 5 carbon atoms), a hydroxyl group, a cyano group, and a nitro group.
The alkyl moieties in the alkoxy group, the alkoxycarbonyl group, the alkylcarbonyl group, and the alkylsulfonyl group represented by $R_{14}$ are each preferably a linear alkyl group having 1 to 10 carbon atoms, a branched alkyl group having 3 to 10 carbon atoms, or a cyclic alkyl group having 3 to 10 carbon atoms.
Each of the alkyl moieties in the alkoxy group, the alkoxycarbonyl group, the alkylcarbonyl group, and the alkylsulfonyl group represented by $R_{14}$ may further have a substituent. Examples of the substituent include halogen atoms, alkoxy groups (for example, having 1 to 5 carbon atoms), a hydroxyl group, a cyano group, and a nitro group.
$R_{15}$'s each independently represent an alkyl group or a naphthyl group.
The alkyl group represented by $R_{15}$ may be linear, branched, or cyclic. The number of carbon atoms of the alkyl group is preferably 1 to 10. The alkyl group is preferably a methyl group, an ethyl group, a n-butyl group, or a t-butyl group.
The alkyl group and the naphthyl group represented by $R_{15}$ may further have a substituent. Examples of the substituent include, but are not particularly limited to, the groups described as examples of the above substituent T.
Two $R_{15}$'s may be bonded together to form a ring. When two $R_{15}$'s are bonded together to form a ring, the ring skeleton may include a heteroatom such as an oxygen atom or a nitrogen atom. In one embodiment, two $R_{15}$'s are preferably alkylene groups and bonded together to form an aliphatic hydrocarbon ring structure.
Next, the cation (ZaII) will be described.
In the formula (ZaII), $R^{204}$ and $R^{205}$ each independently represent a monovalent aromatic ring group that may have a substituent or an alkyl group that may have a substituent, and, from the viewpoint of providing a better effect of the present invention, a monovalent aromatic ring group is preferred.
The monovalent aromatic ring group represented by $R^{204}$ and $R^{205}$ may be an aryl group or a heteroaryl group.
The aryl group is preferably a phenyl group or a naphthyl group, more preferably a phenyl group.
The heteroaryl group has a heteroatom such as an oxygen atom, a nitrogen atom, or a sulfur atom. Examples of the ring constituting the heteroaryl group include a pyrrole ring, a furan ring, a thiophene ring, an indole ring, a benzofuran ring, and a benzothiophene ring.
The alkyl group in $R^{204}$ and $R^{205}$ is preferably a linear alkyl group having 1 to 10 carbon atoms or a branched alkyl

group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

The monovalent aromatic ring group and the alkyl group in $R^{204}$ and $R^{205}$ each may further have another substituent, and examples of the other substituent include an alkyl group (for example, having 1 to 15 carbon atoms), a monovalent aromatic ring group (for example, having 6 to 15 carbon atoms), an alkoxy group (for example, having 1 to 15 carbon atoms), a halogen atom, a hydroxyl group, and a phenylthio group.

In the formula (ZaII), $R^{204}$ and $R^{205}$ each independently represent a monovalent aromatic ring group that may have a substituent or an alkyl group that may have a substituent, and, from the viewpoint of providing a better effect of the present invention, a monovalent aromatic ring group is preferred.

Next, the cation (ZaIII) will be described.

The cation (ZaIII) corresponds to a quaternary ammonium cation or a quaternary phosphonium cation.

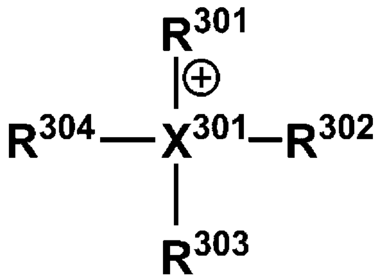

(ZaIII)

In the formula (ZaIII), $X^{301}$ represents a nitrogen atom or a phosphorus atom. $R^{301}$ to $R^{304}$ each independently represent a monovalent aromatic ring group that may have a substituent or an alkyl group that may have a substituent.

In the cation (ZaIII), the nitrogen atom or the phosphorus atom represented by $X^{301}$ is cationized.

The alkyl group represented by $R^{301}$ to $R^{304}$ may be linear, branched, or cyclic, but is preferably linear or branched, more preferably linear.

The number of carbon atoms of the alkyl group is preferably 1 to 20, more preferably 4 to 18, still more preferably 6 to 12.

Examples of the substituent that the alky group may have include, but are not particularly limited to, the groups described as examples of the above substituent T.

The monovalent aromatic ring group represented by $R^{301}$ to $R^{304}$ may be an aryl group or a heteroaryl group.

The aryl group and the heteroarysl group may be monocyclic or polycyclic.

The number of carbon atoms of the aryl group is, for example, preferably 6 to 20, more preferably 6 to 10. A specific example of the aryl group is preferably a phenyl group or a naphthyl group, more preferably a phenyl group.

Examples of the heteroatom included in the heteroaryl group include, but are not particularly limited to, an oxygen atom, a nitrogen atom, and a sulfur atom.

The number of carbon atoms of the heteroaryl group is, for example, preferably 3 to 15, more preferably 3 to 10. Specific examples of the ring constituting the heteroaryl group include a pyrrole ring, a furan ring, a thiophene ring, an indole ring, a benzofuran ring, and a benzothiophene ring.

Examples of the substituent that the aryl group and the heteroaryl group may have include, but are not particularly limited to, the groups described as examples of the above substituent T.

Examples of the substituent include alkyl groups (such as linear alkyl groups having 1 to 15 carbon atoms, branched alkyl groups having 3 to 15 carbon atoms, and cyclic alkyl groups having 3 to 15 carbon atoms); aryl groups (that may be monocyclic or polycyclic and that preferably have 6 to 20 carbon atoms, more preferably 6 to 10 carbon atoms) heteroaryl groups (that may be monocyclic or polycyclic and that preferably have 3 to 15 carbon atoms, more preferably 3 to 10 carbon atoms); alkoxy groups (such as linear alkoxy groups having 1 to 15 carbon atoms, branched alkoxy groups having 1 to 15 carbon atoms, and cyclic alkoxy groups having 3 to 15 carbon atoms); halogen atoms (such as a fluorine atom, a chlorine atom, and an iodine atom); a hydroxyl group; a cyano group; and a nitro group.

The substituent may further have another substituent, and, for example, the alkyl group may have a halogen atom as a substituent to serve as an alkyl halide group such as a trifluoromethyl group.

In the formula (ZaIII), $R^{301}$ to $R^{304}$ may be bonded together to form an alicyclic ring.

The alicyclic ring may include, as a ring member atom, a heteroatom (for example, an oxygen atom, a nitrogen atom, or an oxygen atom) other than the nitrogen atom or the phosphorus atom represented by $X^{301}$ specified in the formula. The ring member atom may also be substituted with a carbonyl carbon (>C=O).

The alicyclic ring may be monocyclic or polycyclic.

The number of ring member atoms of the alicyclic ring is preferably 5 to 15, more preferably 5 to 10, still more

preferably 5 or 6.

The alicyclic ring may further have a substituent. Examples of the substituent include the groups described as examples of the above substituent T.

Next, the cation (ZaIV) will be described.

The cation (ZaIV) corresponds to a quaternary ammonium cation or a quaternary phosphonium cation.

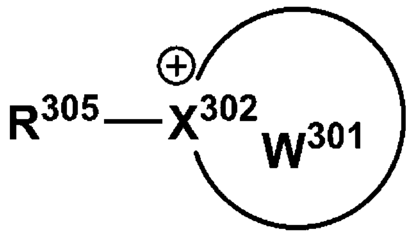

(ZaIV)

In the formula (ZaIV), $X^{302}$ represents a nitrogen atom or a phosphorus atom. $R^{305}$ represents an alkyl group that may have a substituent.

$W^{301}$ represents an aromatic ring including, as a ring member atom, a nitrogen atom or a phosphorus atom represented by $X^{302}$.

In the cation (ZaIV), the nitrogen atom or the phosphorus atom represented by $X^{302}$ is cationized.

The alkyl group that may have a substituent and that is represented by $R^{305}$ has the same definition and preferred embodiments as in the alkyl group that may have a substituent and that is represented by $R^{301}$ to $R^{304}$ in the formula (ZaIII).

The aromatic ring represented by $W^{301}$ may be monocyclic or polycyclic.

The aromatic ring represented by $W^{301}$ may include, as a ring member atom, a heteroatom (for example, an oxygen atom, a nitrogen atom, or an oxygen atom) other than the nitrogen atom or the phosphorus atom specified in the formula.

The number of ring member atoms of the aromatic ring represented by $W^{301}$ is preferably 5 to 15, more preferably 5 to 10, still more preferably 5 or 6.

The aromatic ring represented by $W^{301}$ may further have a substituent. Examples of the substituent include the groups described as examples of the above substituent T.

Examples of the substituent include alkyl groups (such as linear alkyl groups having 1 to 15 carbon atoms, branched alkyl groups having 3 to 15 carbon atoms, and cyclic alkyl groups having 3 to 15 carbon atoms); aryl groups (that may be monocyclic or polycyclic and that preferably have 6 to 20 carbon atoms, more preferably 6 to 10 carbon atoms) heteroaryl groups (that may be monocyclic or polycyclic and that preferably have 3 to 15 carbon atoms, more preferably 3 to 10 carbon atoms); alkoxy groups (such as linear alkoxy groups having 1 to 15 carbon atoms, branched alkoxy groups having 1 to 15 carbon atoms, and cyclic alkoxy groups having 3 to 15 carbon atoms); halogen atoms (such as a fluorine atom, a chlorine atom, and an iodine atom); a hydroxyl group; a cyano group; and a nitro group.

Specific examples of the aromatic ring include a pyridinium cation.

Examples of the inorganic cation represented by $Y^{m+}$ in the formula (3) include, but are not particularly limited to, alkali metal ions such as a lithium ion, a potassium ion, a sodium ion, a rubidium ion, and a cesium ion; and alkaline earth metal ions such as a magnesium ion and a calcium ion.

In the formula (3), $Y^{m+}$ is preferably an organic cation, more preferably a sulfonium cation or an iodonium cation, still more preferably the cation (ZaI) or the cation (ZaII) described above.

The boron-containing compound is also preferably a compound in which an atom in an atomic group bonded to a boron atom, the atom being located at a bonding site to the boron atom, is selected from the group consisting of a carbon atom, an oxygen atom, and a nitrogen atom, from the viewpoint of providing a better effect of the present invention.

The boron-containing compound also preferably has at least one of a fluorine atom or an iodine atom, from the viewpoint of providing a better effect of the present invention.

An example of a preferred embodiment of the boron-containing compound is a compound having a borate anion in which an atom in an atomic group bonded to a boron atom, the atom being located at a bonding site to the boron atom, is any of a carbon atom, an oxygen atom, and a nitrogen atom, the compound including a fluorine atom or an iodine atom. In the above embodiment, the boron atom is anionized. The counter cation of the borate anion is preferably an organic cation, more preferably a sulfonium cation or an iodonium cation, still more preferably the cation (ZaI) or the cation (ZaII) described above.

The content of the boron-containing compound in the resin composition is preferably 1.0 mass% or more, more preferably 2.0 mass% or more, still more preferably 4.0 mass% or more relative to the total solid contents of the resist composition. The upper limit value is preferably 30.0 mass% or less, more preferably 25.0 mass% or less, still more preferably 20.0 mass% or less, particularly preferably 10.0 mass% or less.

Such boron-containing compounds may be used alone or in combination of two or more thereof. When two or more boron-containing compounds are used, the total content thereof is preferably within the above preferred content range.

[Photoacid generator]

The resist composition includes a photoacid generator.
The photoacid generator is a compound that generates an acid upon irradiation with an actinic ray or a radiation.
The photoacid generator may be in the form of a low-molecular-weight compound or in the form of a polymer.
The form in which the photoacid generator is a polymer may be, for example, a form in which the photoacid generator is incorporated into a part of the above-described resin (A) (acid-decomposable resin), and more specifically, a form in which the photoacid generator and the above-described resin (A) (acid-decomposable resin) are linked to each other by a covalent bond.
A photoacid generator in the form of a low-molecular-weight compound may be used in combination with a photoacid generator in the form of a polymer (for example, in the form of being incorporated into a part of the above-described resin (A) (acid-decomposable resin)).
When the photoacid generator is in the form of a low-molecular-weight compound, the molecular weight of the photoacid generator is preferably 3,000 or less, more preferably 2,000 or less, still more preferably 1,000 or less. The lower limit is not particularly limited, but is preferably 100 or more.
When the photoacid generator is in the form of being incorporated into a part of a polymer, the photoacid generator may be incorporated into a part of the resin (A) or incorporated into a resin different from the resin (A).
In the present specification, the photoacid generator is preferably in the form of a low-molecular-weight compound.
The photoacid generator is preferably an onium salt compound, and as described later, preferably a sulfonium salt compound or an iodonium salt compound.

<Photoacid generator PG1>

An example of a preferred embodiment of the photoacid generator is a compound that is represented by "$M^+ X^-$" and that generates an organic acid upon exposure (hereinafter, also referred to as a "photoacid generator PG1").
In the compound represented by "$M^+ X^-$", $M^+$ represents an organic cation, and $X^-$ represents an organic anion.
Examples of the organic acid include sulfonic acids (such as aliphatic sulfonic acids, aromatic sulfonic acids, and camphor sulfonic acid), carboxylic acids (such as aliphatic carboxylic acids, aromatic carboxylic acids, and aralkyl carboxylic acids), carbonylsulfonylimidic acid, bis(alkylsulfonyl)imidic acids, and tris(alkylsulfonyl)methide acids.
The pKa of the organic acid is preferably 2.0 or less, more preferably less than 0. The lower limit value of the acid dissociation constant a1 is preferably -20.0 or more.
The photoacid generator PG1 will be described below.
In the compound represented by "$M^+ X^-$", $M^+$ represents an organic cation.
The organic cation is not particularly limited. The valence of the organic cation may be mono-, di-, or higher valent.
Specific examples of the organic cation include the cation (ZaI) and the cation (ZaII) that the boron-containing compound described above may have, and preferred embodiments thereof are also the same.
The organic cation preferably includes a fluorine atom from the viewpoint of providing a better effect of the present invention.
In the compound represented by "$M^+ X^-$", $X^-$ represents an organic anion.
The organic anion is not particularly limited, and may be a mono-, di-, or higher valent organic anion.
The organic anion is preferably an anion having an extremely low ability to cause a nucleophilic reaction, more preferably a non-nucleophilic anion.
Examples of the non-nucleophilic anion include sulfonate anions (such as aliphatic sulfonate anions, aromatic sulfonate anions, and a camphorsulfonate anion), carboxylate anions (such as aliphatic carboxylate anions, aromatic carboxylate anions, and aralkyl carboxylate anions), sulfonylimide anions, bis(alkylsulfonyl)imide anions, and tris(alkylsulfonyl)methide anions.
The organic anion is also preferably, for example, an organic anion represented by a formula (DA) below.

$$\overset{\ominus}{A_{31}}—L_{a1}—R_{a1} \quad \text{(DA)}$$

In the formula (DA), $A^{31-}$ represents an anionic group. $R_{a1}$ represents a hydrogen atom or a monovalent organic

group. $L_{a1}$ represents a single bond or a divalent linking group.

$A^{31-}$ represents an anionic group. The anionic group represented by $A^{31-}$ is not particularly limited, but is preferably, for example, a group selected from the group consisting of groups represented by formulae (B-1) to (B-14).

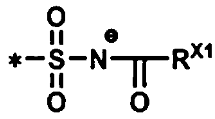
B-1

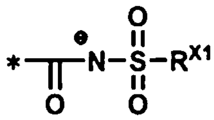
B-2

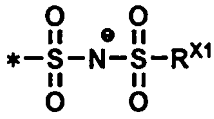
B-3

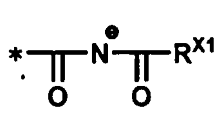
B-4

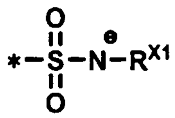
B-5

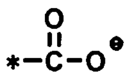
B-6

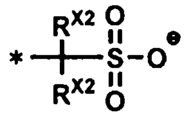
B-7

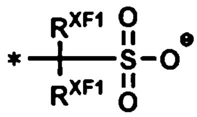
B-8

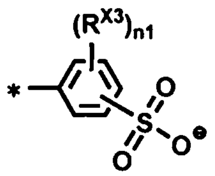
B-9

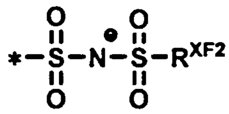
B-10

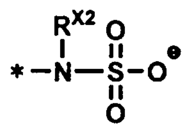
B-11

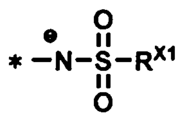
B-12

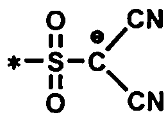
B-13

$*-O^-$ Formula (B-14)

In the formulae (B-1) to (B-14), * represents a bonding site.

In the formulae (B-1) to (B-5) and the formula (B-12), each $R^{X1}$ independently represents a monovalent organic group.

In the formula (B-7) and the formula (B-11), each $R^{X2}$ independently represents a hydrogen atom or a substituent other than a fluorine atom and a perfluoroalkyl group. Two $R^{X2}$'s in the formula (B-7) may be the same or different.

In the formula (B-8), $R^{XF1}$'s represent a hydrogen atom, a fluorine atom, or a perfluoroalkyl group. However, at least one of two $R^{XF1}$'s represents a fluorine atom or a perfluoroalkyl group. Two $R^{XF1}$'s in the formula (B-8) may be the same or different.

In the formula (B-9), $R^{X3}$ represents a hydrogen atom, a halogen atom, or a monovalent organic group. n1 represents an integer of 0 to 4. When n1 represents an integer of 2 to 4, a plurality of $R^{X3}$'s may be the same or different.

In the formula (B-10), $R^{XF2}$ represents a fluorine atom or a perfluoroalkyl group.

The partner bonded to the bonding site represented by * in the formula (B-14) is preferably a phenylene group that may have a substituent. Examples of the substituent that the phenylene group may have include halogen atoms.

In the formulae (B-1) to (B-5) and the formula (B-12), each $R^{X1}$ independently represents a monovalent organic group.

$R^{X1}$ is preferably an alkyl group (that may be linear or branched and preferably has 1 to 15 carbon atoms), a cycloalkyl group (that may be monocyclic or polycyclic and preferably has 3 to 20 carbon atoms), or an aryl group (that may be monocyclic or polycyclic and preferably has 6 to 20 carbon atoms). The above group represented by $R^{X1}$ may have a substituent.

In $R^{X1}$ in the formula (B-5), the atom directly bonded to N- is also preferably neither a carbon atom in -CO- nor a sulfur atom in $-SO_2-$.

The cycloalkyl group in $R^{X1}$ may be monocyclic or polycyclic.

Examples of the cycloalkyl group in $R^{X1}$ include a norbornyl group and an adamantyl group.

The substituent that the cycloalkyl group in $R^{X1}$ may have is not particularly limited, but is preferably an alkyl group (that may be linear or branched and preferably has 1 to 5 carbon atoms). One or more of carbon atoms which are ring member atoms of the cycloalkyl group in $R^{X1}$ may be replaced by carbonyl carbon atoms.

The number of carbon atoms of the alkyl group in $R^{X1}$ is preferably 1 to 10, more preferably 1 to 5.

The substituent that the alkyl group in $R^{X1}$ may have is not particularly limited, but is preferably, for example, a cycloalkyl group, a fluorine atom, or a cyano group.

Examples of the cycloalkyl group serving as the substituent are also the same as the cycloalkyl groups described in the case where $R^{X1}$ is a cycloalkyl group.

When the alkyl group in $R^{X1}$ has a fluorine atom serving as the substituent, the alkyl group may be a perfluoroalkyl group.

In addition, one or more $-CH_2-$ of the alkyl group in $R^{X1}$ may be substituted with carbonyl groups.

The aryl group in $R^{X1}$ is preferably a benzene ring group.

The substituent that the aryl group in $R^{X1}$ may have is not particularly limited, but is preferably an alkyl group, a fluorine atom, or a cyano group. Examples of the alkyl group serving as the substituent are also the same as the alkyl groups described in the case where $R^{X1}$ is an alkyl group.

In the formulae (B-7) and (B-11), each $R^{X2}$ independently represents a hydrogen atom or a substituent other than a fluorine atom and a perfluoroalkyl group (e.g., an alkyl group including no fluorine atoms or a cycloalkyl group including no fluorine atoms). Two $R^{X2}$'s in the formula (B-7) may be the same or different.

In the formula (B-8), $R^{XF1}$'s represent a hydrogen atom, a fluorine atom, or a perfluoroalkyl group. However, at least one of the plurality of $R^{XF1}$'s represents a fluorine atom or a perfluoroalkyl group. Two $R^{XF1}$'s in the formula (B-8) may be the same or different. The number of carbon atoms of the perfluoroalkyl group represented by $R^{XF1}$ is preferably 1 to 15, more preferably 1 to 10, still more preferably 1 to 6.

In the formula (B-9), $R^{X3}$ represents a hydrogen atom, a halogen atom, or a monovalent organic group. Examples of the halogen atom serving as $R^{X3}$ include a fluorine atom, a chlorine atom, a bromine atom, and an iodine atom. In particular, a fluorine atom is preferred.

The monovalent organic group serving as $R^{X3}$ is the same as the monovalent organic group described as $R^{X1}$.

n1 represents an integer of 0 to 4.

n1 is preferably an integer of 0 to 2, preferably 0 or 1. When n1 represents an integer of 2 to 4, a plurality of $R^{X3}$'s may be the same or different.

In the formula (B-10), $R^{XF2}$ represents a fluorine atom or a perfluoroalkyl group.

The number of carbon atoms of the perfluoroalkyl group represented by $R^{XF2}$ is preferably 1 to 15, more preferably 1 to 10, still more preferably 1 to 6.

In the formula (DA), the monovalent organic group in $R_{a1}$ is not particularly limited, but generally has 1 to 30 carbon atoms, preferably 1 to 20 carbon atoms.

$R_{a1}$ is preferably an alkyl group, a cycloalkyl group, or an aryl group.

The alkyl group may be linear or branched, and is preferably an alkyl group having 1 to 20 carbon atoms, more preferably an alkyl group having 1 to 15 carbon atoms, still more preferably an alkyl group having 1 to 10 carbon atoms.

The cycloalkyl group may be monocyclic or polycyclic, and is preferably a cycloalkyl group having 3 to 20 carbon atoms, more preferably a cycloalkyl group having 3 to 15 carbon atoms, still more preferably a cycloalkyl group having 3 to 10 carbon atoms.

The aryl group may be monocyclic or polycyclic, and is preferably an aryl group having 6 to 20 carbon atoms, more preferably an aryl group having 6 to 15 carbon atoms, still more preferably an aryl group having 6 to 10 carbon atoms.

The cycloalkyl group may include a heteroatom as a ring member atom.

The heteroatom is not particularly limited, and may be, for example, a nitrogen atom or an oxygen atom.

The cycloalkyl group may include a carbonyl bond (>C=O) as a ring member atom.

The alkyl group, the cycloalkyl group, and the aryl group may further have a substituent.

The divalent linking group serving as $L_{a1}$ is not particularly limited, but represents an alkylene group, a cycloalkylene group, an aromatic group, -O-, -CO-, -SO-, $-SO_2-$, or a group provided by combining two or more of these groups.

The alkylene group may be linear or branched, and preferably has 1 to 20 carbon atoms, more preferably 1 to 10 carbon atoms.

The cycloalkylene group may be monocyclic or polycyclic, and preferably has 3 to 20 carbon atoms, more preferably 3 to 10 carbon atoms.

The aromatic group is a divalent aromatic group, preferably an aromatic group having 6 to 20 carbon atoms, more preferably an aromatic group having 6 to 15 carbon atoms.

The aromatic ring constituting the aromatic group is not particularly limited, but may be, for example, an aromatic ring having 6 to 20 carbon atoms, and specific examples thereof include a benzene ring, a naphthalene ring, an anthracene ring, and a thiophene ring. The aromatic ring constituting the aromatic group is preferably a benzene ring or a naphthalene

ring, more preferably a benzene ring.

The alkylene group, the cycloalkylene group, and the aromatic group may further have a substituent, and the substituent is preferably a halogen atom.

$A^{31-}$ and $R_{a1}$ may be bonded together to form a ring.

It is also preferable to use, as the photoacid generator PG1, for example, the photoacid generators disclosed in paragraphs [0135] to [0171] of WO2018/193954A, paragraphs [0077] to [0116] of WO2020/066824A, and paragraphs [0018] to [0075] and [0334] and [0335] of WO2017/154345A.

The molecular weight of the photoacid generator PG1 is preferably 3,000 or less, more preferably 2,000 or less, still more preferably 1,000 or less.

<Photoacid generator PG2>

Another preferred example of the photoacid generator is the following compound (I) (hereinafter, also referred to as a "photoacid generator PG2"). The photoacid generator PG2 is a compound that has two or more of salt structural moieties described above and that generates a polyvalent organic acid upon exposure.

The photoacid generator PG2 will be described below.

(Compound (I))

The compound (I) is a compound having one or more structural moieties X described below and one or more structural moieties Y describe below and is a compound that generates an acid including a first acidic moiety described below and derived from the structural moiety X and a second acidic moiety described below and derived from the structural moiety Y upon irradiation with an actinic ray or a radiation.

Structural moiety X: A structural moiety that is constituted by an anionic moiety $A_1^-$ and a cationic moiety $M_1^+$ and that forms the first acidic moiety represented by $HA_1$ upon irradiation with an actinic ray or a radiation

Structural moiety Y: A structural moiety that is constituted by an anionic moiety $A_2^-$ and a cationic moiety $M_2^+$ and that forms the second acidic moiety represented by $HA_2$ upon irradiation with an actinic ray or a radiation

Here, the compound (I) satisfies the following condition I.

Condition I: A compound PI in which, in the compound (I), the cationic moiety $M_1^+$ in the structural moiety X and the cationic moiety $M_2^+$ in the structural moiety Y are replaced by $H^+$ has an acid dissociation constant a1 derived from an acidic moiety represented by $HA_1$ in which the cationic moiety $M_1^+$ in the structural moiety X is replaced by $H^+$ and an acid dissociation constant a2 derived from an acidic moiety represented by $HA_2$ in which the cationic moiety $M_2^+$ in the structural moiety Y is replaced by $H^+$, and the acid dissociation constant a2 is larger than the acid dissociation constant a1.

One embodiment of the compound (I) is an embodiment in which the compound has two structural moieties X and one structural moiety Y.

When the compound (I) is, for example, a compound having two structural moieties X and one structural moiety Y (i.e., a compound that generates an acid having two first acidic moieties derived from the structural moieties X and a single second acidic moiety derived from the structural moiety Y), the compound PI corresponds to a "compound having two $HA_1$ and a single $HA_2$".

In determination of acid dissociation constants of such a compound PI, an acid dissociation constant determined when the compound PI is turned into a "compound having a single $A_1^-$, a single $HA_1$, and a single $HA_2$" and an acid dissociation constant determined when the "compound having a single $A_1^-$, a single $HA_1$, and a single $HA_2$" is turned into a "compound having two $A_1^-$ and a single $HA_2$" each correspond to the above-described acid dissociation constant a1. An acid dissociation constant determined when the "compound having two $A_1^-$ and a single $HA_2$" is turned into a "compound having two $A_1^-$ and $A_2^-$" corresponds to the acid dissociation constant a2. That is, in the case where the compound PI has a plurality of acid dissociation constants derived from acidic moieties represented by $HA_1$ in which the cationic moiety $M_1^+$ in the structural moiety X is replaced by $H^+$, the value of the acid dissociation constant a2 is larger than the maximum value of the plurality of acid dissociation constants a1. Note that, when the acid dissociation constant determined when the compound PI is turned into a "compound having a single $A_1^-$, a single $HA_1$, and a single $HA_2$" is defined as aa and the acid dissociation constant determined when the "compound having a single $A_1^-$, a single $HA_1$, and a single $HA_2$" is turned into a "compound having two $A_1^-$ and a single $HA_2$" is defined as ab, the relation between aa and ab satisfies aa < ab.

The acid dissociation constant a1 and the acid dissociation constant a2 can be determined by the above-described method of measuring an acid dissociation constant.

The compound PI corresponds to an acid generated when the compound (I) is irradiated with an actinic ray or a radiation.

In the compound PI, the difference (absolute value) between the acid dissociation constant a1 (in the case where a plurality of acid dissociation constants a1 are present, the maximum value thereof) and the acid dissociation constant a2 is preferably 0.1 or more, more preferably 0.5 or more, still more preferably 1.0 or more. The upper limit value of the

difference (absolute value) between the acid dissociation constant a1 (in the case where a plurality of acid dissociation constants a1 are present, the maximum value thereof) and the acid dissociation constant a2 is not particularly limited, but is, for example, 16 or less.

In the compound PI, the acid dissociation constant a2 is preferably 20 or less, more preferably 15 or less. The lower limit value of the acid dissociation constant a2 is preferably - 4.0 or more, more preferably -1.0 or more, still more preferably 0 or more, most preferably 0.5 or more.

In the compound PI, the acid dissociation constant a1 is preferably 2.0 or less, more preferably less than 0. The lower limit value of the acid dissociation constant a1 is preferably - 20.0 or more.

When the compound (I) has two or more structural moieties X, the structural moieties X may be the same as or different from each other. The two or more $A_1^-$ and the two or more $M_1^+$ may be individually the same or different.

In the compound (I), the $A_1^-$ and the $A_2^-$, and the $M_1^+$ and the $M_2^+$ may be individually the same or different, but the $A_1^-$ and the $A_2^-$ are preferably different.

The anionic moiety $A_1^-$ and the anionic moiety $A_2^-$ are structural moieties including a negatively charged atom or atomic group and are, for example, structural moieties selected from the group consisting of formulae (AA-1) to (AA-3) and formulae (BB-1) to (BB-6) below.

The anionic moiety $A_1^-$ is preferably one that can form an acidic moiety having a small acid dissociation constant, more preferably any one of the formulae (AA-1) to (AA-3), still more preferably any one of the formulae (AA-1) and (AA-3).

The anionic moiety $A_2^-$ is preferably one that can form an acidic moiety having a larger acid dissociation constant than the anionic moiety $A_1^-$, more preferably any one of the formulae (BB-1) to (BB-6), still more preferably any one of the formulae (BB-1) and (BB-4).

In the formulae (AA-1) to (AA-3) and the formulae (BB-1) to (BB-6) below, * represents a bonding site.

In the formula (AA-2), $R^A$ each represent a monovalent organic group. The monovalent organic group represented by $R^A$ is not particularly limited and may be, for example, a cyano group, a trifluoromethyl group, or a methanesulfonyl group.

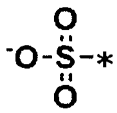

AA-1

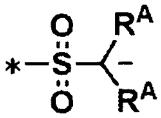

AA-2

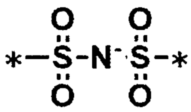

AA-3

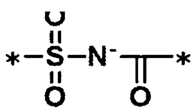

BB-1

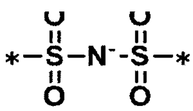

BB-2

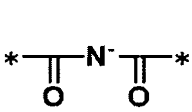

BB-3

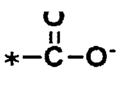

BB-4

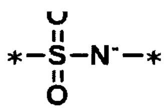

BB-5

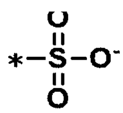

BB-6

The cationic moiety $M_1^+$ and the cationic moiety $M_2^+$ are structural moieties including a positively charged atom or atomic group and are, for example, organic cations having a charge number of 1.

Specific examples of the organic cation include the cation (ZaI) and the cation (ZaII) that the boron-containing compound described above may have, and preferred embodiments thereof are also the same.

The organic cation preferably includes a fluorine atom from the viewpoint of providing a better effect of the present invention.

The molecular weight of the photoacid generator PG2 is preferably 100 to 10,000, more preferably 100 to 2,500, still more preferably 100 to 1,500.

As the photoacid generator PG2, the compounds described as examples in paragraphs [0023] to [0078] of WO2020/158313A can be cited.

The photoacid generator is also preferably a compound including a cation including a fluorine atom, and is more preferably an onium salt compound in which an organic cation including a fluorine atom and a nucleophilic organic anion form a pair, from the viewpoints that a better effect of the present invention is provided, a pattern having smaller LER can be formed, a pattern having even higher resolution can be formed, and/or sensitivity is even higher.

The content of the photoacid generator in the resist composition is not particularly limited, but is preferably 0.5 mass% or more, more preferably 5.0 mass% or more, still more preferably 10.0 mass% or more, particularly preferably 20.0 mass% or more relative to the total solid contents of the resist composition from the viewpoint that the cross-sectional shape of a pattern to be formed becomes more rectangular. The content is preferably 50.0 mass% or less, more preferably 45.0 mass% or less, still more preferably 40.0 mass% or less relative to the total solid contents of the resist composition.

Such photoacid generators may be used alone or in combination of two or more thereof. When two or more

photoacid generators are used, the total content thereof is preferably within the above preferred content range.

[Acid diffusion control agent]

The resist composition also preferably includes an acid diffusion control agent.

The acid diffusion control agent serves as a quencher that traps an acid generated from the photoacid generator or the like upon exposure and that suppresses a reaction of the acid-decomposable resin in unexposed portions, the reaction being caused by an excess of the generated acid.

Examples of the kind of acid diffusion control agent include, but are not particularly limited to, a basic compound (DA), a compound (DB) that undergoes a reduction or loss of the basicity upon irradiation with an actinic ray or radiation, a low-molecular-weight compound (DC) having a group that leaves due to the action of an acid, and an onium salt compound (DD) that generates an acid serving as a weak acid weaker than the photoacid generator.

Specific examples of the basic compound (DA) include those described in paragraphs [0132] to [0136] of WO2020/066824A. Specific examples of the basic compound (DB) that undergoes a reduction or loss of the basicity upon irradiation with an actinic ray or radiation include those described in paragraphs [0137] to [0155] of WO2020/066824A. Specific examples of the low-molecular-weight compound (DC) having a group that leaves due to the action of an acid include those described in paragraphs [0156] to [0163] of WO2020/066824A. Specific examples of the onium salt compound (DD) that generates an acid serving as a weak acid weaker than the photoacid generator include those described in paragraph [0164] of WO2020/066824A.

Specific examples of the onium salt compound (DD) that generates an acid serving as a weak acid weaker than the photoacid generator include those described in paragraphs [0305] to [0314] of WO2020/158337A.

In addition to the above, publicly known compounds disclosed in, for example, paragraphs [0627] to [0664] of US2016/0070167A, paragraphs [0095] to [0187] of US2015/0004544A, paragraphs [0403] to [0423] of US2016/0237190A, and paragraphs [0259] to [0328] of US2016/0274458A can be suitably used as the acid diffusion control agent.

When the acid diffusion control agent is the onium salt compound (DD) that generates an acid serving as a weak acid weaker than the photoacid generator, the acid dissociation constant of the generated acid is preferably 20 or less, more preferably 15 or less. The lower limit value of the acid dissociation constant is preferably -4.0 or more, more preferably -1.0 or more, still more preferably 0 or more, most preferably 0.5 or more.

The onium salt compound is preferably a sulfonium salt compound or an iodonium salt compound.

When the resist composition includes an acid diffusion control agent, the content of the acid diffusion control agent (the total content in the case where a plurality of acid diffusion control agents are present) is preferably 0.1 to 20.0 mass%, more preferably 0.1 to 15.0 mass%, still more preferably 1.0 to 15.0 mass% relative to the total solid contents of the resist composition.

In the resist composition, such acid diffusion control agents may be used alone or in combination of two or more thereof. When two or more acid diffusion control agents are used, the total content thereof is preferably within the above preferred content range.

[Surfactant]

The resist composition may include a surfactant. When a surfactant is included, a pattern having even higher adhesiveness and fewer development defects can be formed.

The surfactant is preferably a fluorine-based and/or silicon-based surfactant.

Examples of the fluorine-based and/or silicon-based surfactant include the surfactants disclosed in paragraphs [0218] and [0219] of WO2018/193954A.

Such surfactants may be used alone or in combination of two or more thereof.

When the resist composition includes a surfactant, the content of the surfactant is preferably 0.0001 to 2.0 mass%, more preferably 0.0005 to 1.0 mass%, still more preferably 0.1 to 1.0 mass% relative to the total solid contents of the resist composition. When two or more surfactants are used, the total content thereof is preferably within the above preferred content range.

[Hydrophobic resin]

The resist composition may further include a hydrophobic resin different from the resin (A).

The hydrophobic resin is preferably designed so as to be localized in the surface of a resist film. However, unlike surfactants, the hydrophobic resin does not necessarily need to have a hydrophilic group in the molecule, and does not necessarily contribute to homogeneous mixing of a polar substance and a nonpolar substance.

Advantages due to the addition of the hydrophobic resin may be control of static and dynamic contact angles at

the surface of the resist film for water, and suppression of outgassing.

From the viewpoint of localization in the surface layer of the film, the hydrophobic resin preferably has one or more, more preferably two or more selected from the group consisting of a fluorine atom, a silicon atom, and a $CH_3$ partial structure included in a side chain moiety of the resin. The hydrophobic resin preferably has a hydrocarbon group having 5 or more carbon atoms. The resin may have such a group in the main chain thereof or, as a substituent, in a side chain thereof.

Examples of the hydrophobic resin include the compounds described in paragraphs [0275] to [0279] of WO2020/004306A.

When the resist composition includes a hydrophobic resin, the content of the hydrophobic resin is preferably 0.01 to 20.0 mass%, more preferably 0.1 to 15.0 mass% relative to the total solid contents of the resist composition.

In the resist composition, such hydrophobic resins may be used alone or in combination of two or more thereof. When two or more hydrophobic resins are used, the total content thereof is preferably within the above preferred content range.

[Solvent]

The resist composition preferably includes a solvent.

The solvent preferably includes at least one of (M1) a propylene glycol monoalkyl ether carboxylate or (M2) at least one selected from the group consisting of a propylene glycol monoalkyl ether, a lactate, an acetate, an alkoxypropionate, a chain ketone, a cyclic ketone, a lactone (e.g., γ-butyrolactone), and an alkylene carbonate. The solvent may further include a component other than the components (M1) and (M2).

It is preferable to combine the above-described solvent and the above-described resin from the viewpoints of improving coatability of the resist composition and reducing the number of development defects in a pattern. Since the above-described solvent is well-balanced in terms of solubility of the resin described above, boiling point, and viscosity, for example, unevenness of the film thickness of the resist film can be reduced, and the generation of precipitates during spin coating can be suppressed.

Details of the component (M1) and the component (M2) are described in paragraphs [0218] to [0226] of WO2020/004306A, the contents of which are incorporated herein by reference.

When the solvent further includes a component other than the components (M1) and (M2), the content of the component other than the components (M1) and (M2) is preferably 5 to 30 mass% relative to the total amount of the solvent.

The content of the solvent in the resist composition is determined such that the concentration of solid contents is preferably 0.5 to 30% by mass, more preferably 1 to 20% by mass. In such a case, the coatability of the resist composition can be further improved.

[Other additives]

The resist composition may further include a dissolution inhibiting compound, a dye, a plasticizer, a photosensitizer, a light absorbent, and/or a compound that improves solubility in developers (for example, a phenolic compound having a molecular weight of 1,000 or less or an alicyclic or aliphatic compound including a carboxyl group).

The "dissolution-inhibiting compound" refers to a compound that has a molecular weight of 3,000 or less and that is decomposed by the action of an acid to undergo a decrease in the degree of solubility in organic solvent-based developers.

[Preferred embodiments of resist composition]

The resist composition is also preferably an embodiment E1 described below or an embodiment E2 described below from the viewpoints that a better effect of the present invention is provided, a pattern having smaller LER can be formed, and/or sensitivity is even higher.

<<Embodiment E1 of resist composition>>

In the resist composition,

the photoacid generator is an onium salt compound,
the boron-containing compound is a salt compound, and
when the resist composition includes an acid diffusion control agent which is a salt compound, a requirement 4 is satisfied, and when the resist composition does not include an acid diffusion control agent which is a salt compound, a

requirement 5 is satisfied.

Requirement 4: A value C1 determined by an equation (4) is 0.115 or more.

$$C1 = ([H_{C1}] \times 0.04 + [C_{C1}] \times 1.0 + [N_{C1}] \times 2.1 + [O_{C1}] \times 3.6 + [F_{C1}] \times 5.6 + [S_{C1}] \times 1.5 + [B_{C1}] \times 0.5 + [I_{C1}] \times 39.5)/([H_{C1}] \times 1 + [C_{C1}] \times 12 + [N_{C1}] \times 14 + [O_{C1}] \times 16 + [F_{C1}] \times 19 + [S_{C1}] \times 32 + [B_{C1}] \times 11 + [I_{C1}] \times 127) \quad \text{Equation (4):}$$

In the equation (4), $[H_{C1}]$ represents a molar ratio of a total of hydrogen atoms derived from a cation of the photoacid generator, hydrogen atoms derived from a cation of the boron-containing compound, and hydrogen atoms derived from a cation of the acid diffusion control agent to a total of all atoms derived from the cation of the photoacid generator, all atoms derived from the cation of the boron-containing compound, and all atoms derived from the cation of the acid diffusion control agent. $[C_{C1}]$ represents a molar ratio of a total of carbon atoms derived from the cation of the photoacid generator, carbon atoms derived from the cation of the boron-containing compound, and carbon atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[N_{C1}]$ represents a molar ratio of a total of nitrogen atoms derived from the cation of the photoacid generator, nitrogen atoms derived from the cation of the boron-containing compound, and nitrogen atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[O_{C1}]$ represents a molar ratio of a total of oxygen atoms derived from the cation of the photoacid generator, oxygen atoms derived from the cation of the boron-containing compound, and oxygen atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[F_{C1}]$ represents a molar ratio of a total of fluorine atoms derived from the cation of the photoacid generator, fluorine atoms derived from the cation of the boron-containing compound, and fluorine atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[S_{C1}]$ represents a molar ratio of a total of sulfur atoms derived from the cation of the photoacid generator, sulfur atoms derived from the cation of the boron-containing compound, and sulfur atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[B_{C1}]$ represents a molar ratio of a total of boron atoms derived from the cation of the photoacid generator, boron atoms derived from the cation of the boron-containing compound, and boron atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[I_{C1}]$ represents a molar ratio of a total of iodine atoms derived from the cation of the photoacid generator, iodine atoms derived from the cation of the boron-containing compound, and iodine atoms derived from the cation of the acid diffusion control agent to the total of all the atoms derived from the cation of the photoacid generator, all the atoms derived from the cation of the boron-containing compound, and all the atoms derived from the cation of the acid diffusion control agent.

Requirement 5: A value C2 determined by an equation (5) is 0.115 or more.

$$C2 = ([H_{C2}] \times 0.04 + [C_{C2}] \times 1.0 + [N_{C2}] \times 2.1 + [O_{C2}] \times 3.6 + [F_{C2}] \times 5.6 + [S_{C2}] \times 1.5 + [B_{C2}] \times 0.5 + [I_{C2}] \times 39.5)/([H_{C2}] \times 1 + [C_{C2}] \times 12 + [N_{C2}] \times 14 + [O_{C2}] \times 16 + [F_{C2}] \times 19 + [S_{C2}] \times 32 + [B_{C2}] \times 11 + [I_{C2}] \times 127) \quad \text{Equation (5):}$$

In the equation (5), $[H_{C2}]$ represents a molar ratio of a total of hydrogen atoms derived from the cation of the photoacid generator and hydrogen atoms derived from the cation of the boron-containing compound to a total of all the atoms derived from the cation of the photoacid generator and all the atoms derived from the cation of the boron-containing compound. $[C_{C2}]$ represents a molar ratio of a total of carbon atoms derived from the cation of the photoacid generator and carbon atoms derived from the cation of the boron-containing compound to the total of all the atoms derived from the cation of the photoacid generator and all the atoms derived from the cation of the boron-containing compound. $[N_{C2}]$ represents a molar ratio of a total of nitrogen atoms derived from the cation of the photoacid generator and nitrogen atoms derived from the cation of the boron-containing compound to the total of all the atoms derived from the cation of the photoacid generator and all the atoms derived from the cation of the boron-containing compound. $[O_{C2}]$ represents a molar ratio of a total of oxygen atoms derived from the cation of the photoacid generator and oxygen atoms derived from the cation of the boron-containing compound to the total of all the atoms derived from the cation of the photoacid generator and all the atoms derived from the cation of the boron-containing compound. [$F_{C2}$] represents a molar ratio of a total of fluorine atoms derived from the cation of the photoacid generator and fluorine atoms derived from the cation of the boron-containing compound to the total of all the atoms derived from the cation of the photoacid generator and all the atoms derived from the cation of the boron-containing compound. [$S_{C2}$] represents a molar ratio of a total of sulfur atoms derived from the cation of the photoacid generator and sulfur atoms derived from the cation of the boron-containing compound to the total of all the atoms derived from the cation of the photoacid generator and all the atoms derived from the cation of the boron-containing compound. [$B_{C2}$] represents a molar ratio of a total of boron atoms derived from the cation of the photoacid generator and boron atoms derived from the cation of the boron-containing compound to the total of all the atoms derived from the cation of the photoacid generator and all the atoms derived from the cation of the boron-containing compound. [$I_{C2}$] represents a molar ratio of a total of iodine atoms derived from the cation of the photoacid generator and iodine atoms derived from the cation of the boron-containing compound to the total of all the atoms derived from the cation of the photoacid generator and all the atoms derived from the cation of the boron-containing compound.

<Resist composition satisfying requirement 4>

As described above, the resist composition preferably satisfies the requirement 4 when the photoacid generator is an onium salt compound, the boron-containing compound is a salt compound, and the resist composition includes an acid diffusion control agent which is a salt compound. The requirement 4 is as described above.

When the resist composition includes a photoacid generator which is an onium salt compound, a boron-containing compound which is a salt compound, and an acid diffusion control agent which is a salt compound, and satisfies the requirement 4, the resist composition may further include an acid diffusion control agent other than salt compounds.

In the equation (4), for example, [$H_{C1}$] represents a molar ratio of a total of hydrogen atoms derived from a cation of the photoacid generator, hydrogen atoms derived from a cation of the boron-containing compound, and hydrogen atoms derived from a cation of the acid diffusion control agent to a total of all the atoms derived from the cation of the photoacid generator, all the atoms derived from the cation of the boron-containing compound, and all the atoms derived from the cation of the acid diffusion control agent. To be more specific, [$H_{C1}$] represents a molar ratio of a total of hydrogen atoms derived from a cationic moiety of the photoacid generator which is an onium salt compound, hydrogen atoms derived from a cationic moiety of the boron-containing compound which is a salt compound, and hydrogen atoms derived from a cationic moiety of the acid diffusion control agent which is a salt compound to a total of all the atoms derived from the cationic moiety of the photoacid generator which is an onium salt compound, all the atoms derived from the cationic moiety of the boron-containing compound which is a salt compound, and all the atoms derived from the cationic moiety of the acid diffusion control agent which is a salt compound.

When the resist composition includes two or more photoacid generators which are onium salt compounds, for example, in the case of taking [$H_{C1}$] as an example, all the atoms derived from cations of the photoacid generators are a total of all the atoms derived from each of the cations of the photoacid generators, and the molar ratio of hydrogen atoms derived from the cations of the photoacid generators means a molar ratio of a total of hydrogen atoms derived from each of the cations of the photoacid generators.

When the resist composition includes two or more boron-containing compounds which are salt compounds, for example, in the case of taking [$H_{C1}$] as an example, all the atoms derived from cations of the boron-containing compounds are a total of all the atoms derived from each of the cations of the boron-containing compounds, and the molar ratio of hydrogen atoms derived from the cations of the boron-containing compounds means a molar ratio of a total of hydrogen atoms derived from each of the cations of the boron-containing compounds.

When the resist composition includes two or more acid diffusion control agents which are salt compounds, for example, in the case of taking [$H_{C1}$] as an example, all the atoms derived from cations of the acid diffusion control agents are a total of all the atoms derived from each of the cations of the acid diffusion control agents, and the molar ratio of hydrogen atoms derived from the cations of the acid diffusion control agents means a molar ratio of a total of hydrogen atoms derived from each of the cations of the acid diffusion control agents.

<Resist composition satisfying requirement 5>

As described above, the resist composition preferably satisfies the requirement 5 when the photoacid generator is an onium salt compound, the boron-containing compound is a salt compound, and the resist composition does not include an acid diffusion control agent which is a salt compound.

Here, the phrase "when an acid diffusion control agent which is a salt compound is not included" refers to a case where the resist composition does not include an acid diffusion control agent or a case where the resist composition includes, as an acid diffusion control agent, only an acid diffusion control agent other than a salt compound.

The requirement 5 is as described above.

In the equation (5), for example, $[H_{C2}]$ represents a molar ratio of a total of hydrogen atoms derived from a cation of the photoacid generator and hydrogen atoms derived from a cation of the boron-containing compound to a total of all the atoms derived from the cation of the photoacid generator and all the atoms derived from the cation of the boron-containing compound. To be more specific, $[H_{C2}]$ represents a molar ratio of a total of hydrogen atoms derived from a cationic moiety of the photoacid generator which is an onium salt compound, and hydrogen atoms derived from a cationic moiety of the boron-containing compound which is a salt compound to a total of all the atoms derived from the cationic moiety of the photoacid generator which is an onium salt compound and all the atoms derived from the cationic moiety of the boron-containing compound which is a salt compound.

When the resist composition includes two or more photoacid generators which are onium salt compounds, for example, in the case of taking $[H_{C2}]$ as an example, all the atoms derived from cations of the photoacid generators are a total of all the atoms derived from each of the cations of the photoacid generators, and the molar ratio of hydrogen atoms derived from the cations of the photoacid generators means a molar ratio of a total of hydrogen atoms derived from each of the cations of the photoacid generators.

When the resist composition includes two or more boron-containing compounds which are salt compounds, for example, in the case of taking $[H_{C2}]$ as an example, all the atoms derived from cations of the boron-containing compounds are a total of all the atoms derived from each of the cations of the boron-containing compounds, and the molar ratio of hydrogen atoms derived from the cations of the boron-containing compounds means a molar ratio of a total of hydrogen atoms derived from each of the cations of the boron-containing compounds.

When the structures and contents of the photoacid generator, the boron-containing compound, and the acid diffusion control agent in the resist composition are known, the value C1 and the value C2 can be calculated by calculating the ratio of the number of atoms contained in the resist composition. Even when the constituent components are unknown, the ratio of the numbers of constituent atoms can be calculated by subjecting a resist film obtained by evaporating the solvent component in the resist composition to an analytical method such as elemental analysis.

As described above, the value C1 and the value C2 are each 0.115 or more, but are preferably 0.130 or more from the viewpoints that a better effect of the present invention is provided, a pattern having smaller LER can be formed, a pattern having even higher resolution can be formed, and/or sensitivity is even higher. The upper limit is not particularly limited, but if the value C1 and the value C2 are excessively large, the light transmittance of the resist film decreases, the optical image profile in the resist film deteriorates, and as a result, it becomes difficult to obtain a good pattern shape; therefore, the value C1 and the value C2 are each preferably 0.240 or less, more preferably 0.220 or less.

<<Embodiment E2 of resist composition>>

In the resist composition,

the photoacid generator is an onium salt compound,
the boron-containing compound is a salt compound, and
when the resist composition includes an acid diffusion control agent which is an onium salt compound, a requirement 2 is satisfied, and when the resist composition does not include an acid diffusion control agent which is an onium salt compound, a requirement 3 is satisfied.

Requirement 2: A value B1 determined by an equation (2) is 0.115 or more.

$$B1 = ([H_{B1}] \times 0.04 + [C_{B1}] \times 1.0 + [N_{B1}] \times 2.1 + [O_{B1}] \times 3.6 + [F_{B1}] \times 5.6 + [S_{B1}] \times 1.5 + [B_{B1}] \times 0.5 + [I_{B1}] \times 39.5)/([H_{B1}] \times 1 + [C_{B1}] \times 12 + [N_{B1}] \times 14 + [O_{B1}] \times 16 + [F_{B1}] \times 19 + [S_{B1}] \times 32 + [B_{B1}] \times 11 + [I_{B1}] \times 127) \quad \text{Equation (2):}$$

In the equation (2), $[H_{B1}]$ represents a molar ratio of a total of hydrogen atoms derived from a cation of the photoacid generator, hydrogen atoms derived from a cation of the boron-containing compound, and hydrogen atoms derived from a cation of the acid diffusion control agent to a total of all atoms derived from the cation of the photoacid generator, all atoms derived from the cation of the boron-containing compound, and all atoms derived from the cation of the acid diffusion control agent. $[C_{B1}]$ represents a molar ratio of a total of carbon atoms derived from the cation of the photoacid generator, carbon atoms derived from the cation of the boron-containing compound, and carbon atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[N_{B1}]$ represents a molar ratio of a total of nitrogen atoms derived from the cation of the photoacid generator, nitrogen atoms derived from the cation of the boron-containing compound, and nitrogen atoms

derived from the cation of the acid diffusion control agent to the total of all the atoms derived from the cation of the photoacid generator, all the atoms derived from the cation of the boron-containing compound, and all the atoms derived from the cation of the acid diffusion control agent. $[O_{B1}]$ represents a molar ratio of a total of oxygen atoms derived from the cation of the photoacid generator, oxygen atoms derived from the cation of the boron-containing compound, and oxygen atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[F_{B1}]$ represents a molar ratio of a total of fluorine atoms derived from the cation of the photoacid generator, fluorine atoms derived from the cation of the boron-containing compound, and fluorine atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[S_{B1}]$ represents a molar ratio of a total of sulfur atoms derived from the cation of the photoacid generator, sulfur atoms derived from the cation of the boron-containing compound, and sulfur atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[B_{B1}]$ represents a molar ratio of a total of boron atoms derived from the cation of the photoacid generator, boron atoms derived from the cation of the boron-containing compound, and boron atoms derived from the cation of the acid diffusion control agent to the total of all the atoms derived from the cation of the photoacid generator, all the atoms derived from the cation of the boron-containing compound, and all the atoms derived from the cation of the acid diffusion control agent. $[I_{B1}]$ represents a molar ratio of a total of iodine atoms derived from the cation of the photoacid generator, iodine atoms derived from the cation of the boron-containing compound, and iodine atoms derived from the cation of the acid diffusion control agent to the total of all the atoms derived from the cation of the photoacid generator, all the atoms derived from the cation of the boron-containing compound, and all the atoms derived from the cation of the acid diffusion control agent.

Requirement 3: A value B2 determined by an equation (3) is 0.115 or more.

$$B2 = ([H_{B2}] \times 0.04 + [C_{B2}] \times 1.0 + [N_{B2}] \times 2.1 + [O_{B2}] \times 3.6 + [F_{B2}] \times 5.6 + [S_{B2}] \times 1.5 + [B_{B2}] \times 0.5 + [I_{B2}] \times 39.5)/([H_{B2}] \times 1 + [C_{B2}] \times 12 + [N_{B2}] \times 14 + [O_{B2}] \times 16 + [F_{B2}] \times 19 + [S_{B2}] \times 32 + [B_{B2}] \times 11 + [I_{B2}] \times 127) \quad \text{Equation (3):}$$

In the equation (3), $[H_{B2}]$ represents a molar ratio of a total of hydrogen atoms derived from the cation of the photoacid generator and hydrogen atoms derived from the cation of the boron-containing compound to a total of all the atoms derived from the cation of the photoacid generator and all the atoms derived from the cation of the boron-containing compound. $[C_{B2}]$ represents a molar ratio of a total of carbon atoms derived from the cation of the photoacid generator and carbon atoms derived from the cation of the boron-containing compound to the total of all the atoms derived from the cation of the photoacid generator and all the atoms derived from the cation of the boron-containing compound. $[N_{B2}]$ represents a molar ratio of a total of nitrogen atoms derived from the cation of the photoacid generator and nitrogen atoms derived from the cation of the boron-containing compound to the total of all the atoms derived from the cation of the photoacid generator and all the atoms derived from the cation of the boron-containing compound. $[O_{B2}]$ represents a molar ratio of a total of oxygen atoms derived from the cation of the photoacid generator and oxygen atoms derived from the cation of the boron-containing compound to the total of all the atoms derived from the cation of the photoacid generator and all the atoms derived from the cation of the boron-containing compound. $[F_{B2}]$ represents a molar ratio of a total of fluorine atoms derived from the cation of the photoacid generator and fluorine atoms derived from the cation of the boron-containing compound to the total of all the atoms derived from the cation of the photoacid generator and all the atoms derived from the cation of the boron-containing compound. $[S_{B2}]$ represents a molar ratio of a total of sulfur atoms derived from the cation of the photoacid generator and sulfur atoms derived from the cation of the boron-containing compound to the total of all the atoms derived from the cation of the photoacid generator and all the atoms derived from the cation of the boron-containing compound. $[B_{B2}]$ represents a molar ratio of a total of boron atoms derived from the cation of the photoacid generator and boron atoms derived from the cation of the boron-containing compound to the total of all the atoms derived from the cation of the photoacid generator and all the atoms derived from the cation of the boron-containing compound. $[I_{B2}]$ represents a molar ratio of a total of iodine atoms derived from the cation of the photoacid generator and iodine atoms derived from the cation of the boron-containing compound to the total of all the atoms derived from the cation of the photoacid generator and all the atoms derived from the cation of the boron-containing compound.

<Resist composition satisfying requirement 2>

As described above, the resist composition preferably satisfies the requirement 2 when the photoacid generator is an onium salt compound, the boron-containing compound is a salt compound, and the resist composition includes an

acid diffusion control agent which is an onium salt compound. The requirement 2 is as described above.

When the resist composition includes a photoacid generator which is an onium salt compound, a boron-containing compound which is a salt compound, and an acid diffusion control agent which is an onium salt compound, and satisfies the requirement 2, the resist composition may further include an acid diffusion control agent other than onium salt compounds.

In the equation (2), for example, [$H_{B1}$] represents a molar ratio of a total of hydrogen atoms derived from a cation of the photoacid generator, hydrogen atoms derived from a cation of the boron-containing compound, and hydrogen atoms derived from a cation of the acid diffusion control agent to a total of all the atoms derived from the cation of the photoacid generator, all the atoms derived from the cation of the boron-containing compound, and all the atoms derived from the cation of the acid diffusion control agent. To be more specific, [$H_{B1}$] represents a molar ratio of a total of hydrogen atoms derived from a cationic moiety of the photoacid generator which is an onium salt compound, hydrogen atoms derived from a cationic moiety of the boron-containing compound which is a salt compound, and hydrogen atoms derived from a cationic moiety of the acid diffusion control agent which is an onium salt compound to a total of all the atoms derived from the cationic moiety of the photoacid generator which is an onium salt compound, all the atoms derived from the cationic moiety of the boron-containing compound which is a salt compound, and all the atoms derived from the cationic moiety of the acid diffusion control agent which is an onium salt compound.

When the resist composition includes two or more photoacid generators which are onium salt compounds, for example, in the case of taking [$H_{B1}$] as an example, all the atoms derived from cations of the photoacid generators are a total of all the atoms derived from each of the cations of the photoacid generators, and the molar ratio of hydrogen atoms derived from the cations of the photoacid generators means a molar ratio of a total of hydrogen atoms derived from each of the cations of the photoacid generators.

When the resist composition includes two or more boron-containing compounds which are salt compounds, for example, in the case of taking [$H_{B1}$] as an example, all the atoms derived from cations of the boron-containing compounds are a total of all the atoms derived from each of the cations of the boron-containing compounds, and the molar ratio of hydrogen atoms derived from the cations of the boron-containing compounds means a molar ratio of a total of hydrogen atoms derived from each of the cations of the boron-containing compounds.

When the resist composition includes two or more acid diffusion control agents which are onium salt compounds, for example, in the case of taking [$H_{B1}$] as an example, all the atoms derived from cations of the acid diffusion control agents are a total of all the atoms derived from each of the cations of the acid diffusion control agents, and the molar ratio of hydrogen atoms derived from the cations of the acid diffusion control agents means a molar ratio of a total of hydrogen atoms derived from each of the cations of the acid diffusion control agents.

<Resist composition satisfying requirement 3>

As described above, the resist composition preferably satisfies the requirement 3 when the photoacid generator is an onium salt compound, the boron-containing compound is a salt compound, and the resist composition does not include an acid diffusion control agent which is an onium salt compound.

Here, the phrase "when an acid diffusion control agent which is an onium salt compound is not included" refers to a case where the resist composition does not include an acid diffusion control agent or a case where the resist composition includes, as an acid diffusion control agent, only an acid diffusion control agent other than an onium salt compound.

The requirement 3 is as described above.

In the equation (3), for example, [$H_{B2]}$ represents a molar ratio of a total of hydrogen atoms derived from a cation of the photoacid generator and hydrogen atoms derived from a cation of the boron-containing compound to a total of all the atoms derived from the cation of the photoacid generator and all the atoms derived from the cation of the boron-containing compound. To be more specific, [$H_{B2]}$ represents a molar ratio of a total of hydrogen atoms derived from a cationic moiety of the photoacid generator which is an onium salt compound, and hydrogen atoms derived from a cationic moiety of the boron-containing compound which is a salt compound to a total of all the atoms derived from the cationic moiety of the photoacid generator which is an onium salt compound and all the atoms derived from the cationic moiety of the boron-containing compound which is a salt compound.

When the resist composition includes two or more photoacid generators which are onium salt compounds, for example, in the case of taking [$H_{B2]}$ as an example, all the atoms derived from cations of the photoacid generators are a total of all the atoms derived from each of the cations of the photoacid generators, and the molar ratio of hydrogen atoms derived from the cations of the photoacid generators means a molar ratio of a total of hydrogen atoms derived from each of the cations of the photoacid generators.

When the resist composition includes two or more boron-containing compounds which are salt compounds, for example, in the case of taking [$H_{B2]}$ as an example, all the atoms derived from cations of the boron-containing compounds are a total of all the atoms derived from each of the cations of the boron-containing compounds, and the molar ratio of hydrogen atoms derived from the cations of the boron-containing compounds means a molar ratio of a total of hydrogen

atoms derived from each of the cations of the boron-containing compounds.

When the structures and contents of the photoacid generator, the boron-containing compound, and the acid diffusion control agent in the resist composition are known, the value B1 and the value B2 can be calculated by calculating the ratio of the number of atoms contained in the resist composition. Even when the constituent components are unknown, the ratio of the numbers of constituent atoms can be calculated by subjecting a resist film obtained by evaporating the solvent component in the resist composition to an analytical method such as elemental analysis.

As described above, the value B1 and the value B2 are each 0.115 or more, but are preferably 0.130 or more from the viewpoints that a better effect of the present invention is provided, a pattern having smaller LER can be formed, a pattern having even higher resolution can be formed, and/or sensitivity is even higher. The upper limit is not particularly limited, but if the value B1 and the value B2 are excessively large, the light transmittance of the resist film decreases, the optical image profile in the resist film deteriorates, and as a result, it becomes difficult to obtain a good pattern shape; therefore, the value B1 and the value B2 are each preferably 0.240 or less, more preferably 0.220 or less.

[Resist film and pattern forming method]

The procedure of a pattern forming method using the above-described resist composition is not particularly limited, but the method preferably has steps below.

Step 1: A step of forming a resist film on a substrate using a resist composition
Step 2: A step of exposing the resist film
Step 3: A step of developing the exposed resist film with a developer

The procedure of each of the steps will be described in detail below.

<Step 1: Resist film-forming step>

The step 1 is a step of forming a resist film on a substrate using a resist composition.

The definition of the resist composition is the same as that described above.

The method for forming a resist film on a substrate using a resist composition is, for example, a method in which the resist composition is applied to the substrate.

Prior to the application, the resist composition is preferably subjected to filtration through a filter, if necessary. The filter preferably has a pore size of 0.1 μm or less, more preferably 0.05 μm or less, still more preferably 0.03 μm or less. The filter is preferably formed of polytetrafluoroethylene, polyethylene, or nylon.

The resist composition can be applied to a substrate (for example, formed of silicon and covered with silicon dioxide) as used in the production of an integrated circuit element by a suitable coating method using a spinner, a coater, or the like. The coating method is preferably spin-coating using a spinner. The rotational speed during spin coating using a spinner is preferably 1,000 to 3,000 rpm.

After the application of the resist composition, the substrate may be dried to form a resist film. Note that, as needed, an underlying film (an inorganic film, an organic film, or an antireflection film) may be formed as an underlayer of the resist film.

The drying method may be, for example, a method of performing drying by heating. The heating can be performed using means included in an ordinary exposure apparatus and/or an ordinary developing apparatus, or may be performed using a hot plate or the like. The heating temperature is preferably 80°C to 150°C, more preferably 80°C to 140°C, still more preferably 80°C to 130°C. The heating time is preferably 30 to 1,000 seconds, more preferably 60 to 800 seconds, still more preferably 60 to 600 seconds.

The film thickness of the resist film is not particularly limited, but is preferably 10 to 120 nm from the viewpoint that a fine pattern with higher accuracy can be formed. In particular, in the case of EUV exposure, the film thickness of the resist film is more preferably 10 to 65 nm, still more preferably 15 to 50 nm. In the case of ArF liquid immersion exposure, the film thickness of the resist film is more preferably 10 to 120 nm, still more preferably 15 to 90 nm.

As an upper layer of the resist film, a topcoat may be formed using a topcoat composition.

The topcoat composition preferably does not mix with the resist film and can be uniformly applied as an upper layer of the resist film. The topcoat is not particularly limited, a publicly known topcoat can be formed by a publicly known method, and, for example, a topcoat can be formed on the basis of the description of paragraphs [0072] to [0082] of JP2014-059543A.

For example, a topcoat including a basic compound and described in JP2013-61648A is preferably formed on the resist film. Specific examples of the basic compound that can be included in the topcoat include basic compounds that may be included in the resist composition.

The topcoat also preferably includes a compound including at least one group or bond selected from the group

consisting of an ether bond, a thioether bond, a hydroxyl group, a thiol group, a carbonyl bond, and an ester bond.

<Step 2: Exposure step>

The step 2 is a step of exposing the resist film.

The exposure method may be a method of irradiating the formed resist film with an actinic ray or a radiation through a predetermined mask.

Examples of the actinic ray or radiation include infrared light, visible light, ultraviolet light, far-ultraviolet light, extreme ultraviolet light, X-rays, and an electron beam; 250 nm or less is preferred, 220 nm or less is more preferred, and far-ultraviolet light having a wavelength of 1 to 200 nm and, specifically, KrF excimer laser (248 nm), ArF excimer laser (193 nm), $F_2$ excimer laser (157 nm), EUV (13.5 nm), X-rays, and an electron beam are particularly preferred.

After the exposure and before development, baking (heating) is preferably performed. The baking accelerates the reaction in exposed portions to provide higher sensitivity and a better pattern shape.

The heating temperature is preferably 80°C to 150°C, more preferably 80°C to 140°C, still more preferably 80°C to 130°C.

The heating time is preferably 10 to 1,000 seconds, more preferably 10 to 180 seconds, still more preferably 30 to 120 seconds.

The heating can be performed using means included in an ordinary exposure apparatus and/or an ordinary developing apparatus, or may be performed using a hot plate or the like.

This step is also referred to as post-exposure baking.

<Step 3: Developing step>

The step 3 is a step of developing the exposed resist film with a developer to form a pattern.

The developer may be an organic solvent-based developer (a developer including an organic solvent) or an alkali developer, but is preferably an organic solvent-based developer.

Examples of the developing method include a method of immersing the substrate for a predetermined time in a tank filled with a developer (dipping method), a method of puddling a developer over a surface of the substrate using surface tension and allowing the developer to stand for a predetermined time to perform development (puddling method), a method of spraying a developer onto a surface of the substrate (spraying method), and a method of continuously ejecting a developer over the substrate rotating at a constant rate, while scanning a developer ejection nozzle at a constant rate, (dynamic dispensing method).

After the step of performing development, a step of stopping the development while performing replacement with another solvent may be performed.

The development time is not particularly limited as long as the resin in unexposed portions is sufficiently dissolved within the time, and is preferably 10 to 300 seconds, more preferably 20 to 120 seconds.

The temperature of the developer is preferably 0°C to 50°C, more preferably 15°C to 35°C.

The organic solvent included in the organic solvent-based developer is preferably at least one selected from the group consisting of ketone-based solvents, ester-based solvents, alcohol-based solvents, amide-based solvents, ether-based solvents, and hydrocarbon-based solvents.

The ClogP value of the organic solvent included in the organic solvent-based developer is not particularly limited, but is preferably 0.00 or more, more preferably 1.00 or more. When two or more organic solvents are included, the ClogP value of a mixed solvent thereof is preferably in the above range.

Examples of the ketone-based solvents include 1-octanone, 2-octanone, 1-nonanone, 2-nonanone, acetone, 2-heptanone (methyl amyl ketone), 4-heptanone, 1-hexanone, 2-hexanone, diisobutyl ketone, cyclohexanone, methylcyclohexanone, phenylacetone, methyl ethyl ketone, methyl isobutyl ketone, acetylacetone, acetonylacetone, ionone, diacetonyl alcohol, acetyl carbinol, acetophenone, methyl naphthyl ketone, isophorone, and propylene carbonate.

Examples of the ester-based solvents include methyl acetate, butyl acetate, ethyl acetate, isopropyl acetate, pentyl acetate, isopentyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxypropionate, 3-methoxybutyl acetate, 3-methyl-3-methoxybutyl acetate, methyl formate, ethyl formate, butyl formate, propyl formate, ethyl lactate, butyl lactate, propyl lactate, butyl butyrate, methyl 2-hydroxyisobutyrate, isoamyl acetate, isobutyl isobutyrate, and butyl propionate.

The alcohol-based solvents, the amide-based solvents, the ether-based solvents, and the hydrocarbon-based solvents that can be used are, for example, the solvents disclosed in paragraphs [0715] to [0718] of US2016/0070167A.

A plurality of such solvents may be mixed together, or such a solvent may be mixed with a solvent other than those described above or water. The moisture content of the developer as a whole is preferably less than 50 mass%, more preferably less than 20 mass%, still more preferably less than 10 mass%, and it is particularly preferable that the developer

include substantially no moisture.

The content of the organic solvent in the organic solvent-based developer is preferably 50 to 100 mass%, more preferably 80 to 100 mass%, still more preferably 90 to 100 mass%, particularly preferably 95 to 100 mass%, relative to the total amount of the developer.

From the viewpoint of providing a better effect of the present invention, the organic solvent-based developer preferably includes a first organic solvent and a second organic solvent, and more preferably, the boiling point of the first organic solvent is higher than the boiling point of the second organic solvent and the ClogP value of the first organic solvent is larger than the ClogP value of the second organic solvent.

From the viewpoint of providing a better effect of the present invention, the organic solvent-based developer preferably includes a first organic solvent and a second organic solvent, and the evaporation rate of the second organic solvent is more preferably higher than the evaporation rate of the first organic solvent. In particular, the difference in evaporation rate between the second organic solvent and the first organic solvent is preferably as large as possible. One of the indicators of the evaporation rates of the organic solvents is the vapor pressures at 20°C. The vapor pressure of the second organic solvents is preferably higher than the vapor pressure of the first organic solvent, and the difference in vapor pressure between the second organic solvent and the first organic solvent is preferably 0. 2 kPa or more. In particular, the difference in vapor pressures between the second organic solvent and the first organic solvent is more preferably 0.5 kPa or more, still more preferably 0.8 kPa or more, particularly preferably 1.0 kPa or more. Although the mechanism of action of the organic solvent-based developer having the above-described composition is not clear, it is presumed that the use of the organic solvent-based developer having the above-described composition suppresses the swelling of the resist pattern by the developer.

The content ratio of the first organic solvent to the second organic solvent in the organic solvent-based developer is not particularly limited, but, from the viewpoint of providing a better effect of the present invention, the mass ratio of the content of the first organic solvent to the content of the second organic solvent is preferably 10/90 to 50/50, more preferably 10/90 to 30/70.

In the organic solvent-based developer, the second organic solvent is preferably the ketone-based solvent or the ester-based solvent, more preferably an ester-based solvent, still more preferably an ester-based solvent having 6 or less carbon atoms, particularly preferably butyl acetate, from the viewpoint of providing a better effect of the present invention. The first organic solvent is not particularly limited, but is preferably an organic solvent having a ClogP value of 3.00 or more, more preferably a hydrocarbon-based solvent (preferably a hydrocarbon-based solvent having 10 or more carbon atoms, such as undecane).

The organic solvent-based developer includes a hydrocarbon-based solvent and an ester-based solvent having 6 or less carbon atoms, and the mass ratio of the content of the hydrocarbon-based solvent to the content of the ester-based solvent having 6 or less carbon atoms is preferably 10/90 to 50/50, more preferably 10/90 to 30/70, still more preferably 10/90 to 25/75, from the viewpoint of providing a better effect of the present invention.

As the alkali developer, an alkali aqueous solution including an alkali is preferably used. The type of the alkaline aqueous solution is not particularly limited, but may be, for example, an alkaline aqueous solution including a quaternary ammonium salt represented by tetramethylammonium hydroxide, an inorganic alkali, a primary amine, a secondary amine, a tertiary amine, an alcoholamine, a cyclic amine, or the like. In particular, the alkali developer is preferably an aqueous solution of a quaternary ammonium salt represented by tetramethylammonium hydroxide (TMAH). An appropriate amount of an alcohol, a surfactant, or the like may be added to the alkali developer. Typically, the alkali developer preferably has an alkali concentration of 0.1 to 20 mass%. Typically, the alkali developer preferably has a pH of 10.0 to 15.0.

<Other steps>

The pattern forming method preferably includes, after the step 3, a step of performing washing with a rinsing liquid.

The rinsing liquid used in the rinsing step after the developing step using an organic solvent-based developer is preferably an organic solvent-based rinsing liquid.

An organic solvent included in the rinsing liquid is preferably at least one organic solvent selected from the group consisting of hydrocarbon-based solvents, ketone-based solvents, ester-based solvents, alcohol-based solvents, amide-based solvents, and ether-based solvents.

Examples of the hydrocarbon-based solvents, the ketone-based solvents, the ester-based solvents, the alcohol-based solvents, the amide-based solvents, and the ether-based solvents include the same solvents as those described in the developer including an organic solvent.

From the viewpoint of providing a better effect of the present invention, the organic solvent-based rinsing liquid preferably includes a first organic solvent and a second organic solvent, and more preferably, the boiling point of the first organic solvent is higher than the boiling point of the second organic solvent and the ClogP value of the first organic solvent is larger than the ClogP value of the second organic solvent. Note that the boiling point means a boiling point at 1 atm (760

mmHg).

The content ratio of the first organic solvent to the second organic solvent in the organic solvent-based rinsing liquid is not particularly limited, but, from the viewpoint of providing a better effect of the present invention, the mass ratio of the content of the first organic solvent to the content of the second organic solvent is preferably 10/90 to 50/50, more preferably 10/90 to 30/70.

In the organic solvent-based rinsing liquid, the second organic solvent is preferably the ketone-based solvent or the ester-based solvent, more preferably an ester-based solvent, still more preferably butyl acetate or isoamyl butyrate, from the viewpoint of providing a better effect of the present invention. The first organic solvent is not particularly limited, but is preferably an organic solvent having a ClogP value of 3.00 or more, more preferably a hydrocarbon-based solvent (preferably a hydrocarbon-based solvent having 10 or more carbon atoms, such as undecane).

The rinsing liquid used in the rinsing step after the developing step using an alkali developer may be, for example, pure water. Note that an appropriate amount of surfactant may be added to the pure water.

An appropriate amount of surfactant may be added to the rinsing liquid.

Examples of the method of performing the rinsing step include, but are not particularly limited to, a method of continuously ejecting a rinsing liquid onto the substrate rotating at a constant rate (spin-coating method), a method of immersing the substrate for a predetermined time in a tank filled with a rinsing liquid (dipping method), and a method of spraying a rinsing liquid onto a surface of the substrate (spraying method).

The pattern forming method may include a heating step (Post Bake) after the rinsing step. In this step, the developer and the rinsing liquid remaining between patterns and inside patterns are removed by baking. In addition, this step also provides an effect of annealing the resist pattern to improving the surface roughness of the pattern. The heating step after the rinsing step is performed usually at 40°C to 250°C (preferably 90°C to 200°C) for usually 10 seconds to 3 minutes (preferably 30 seconds to 120 seconds).

The formed pattern may be used as a mask to perform etching treatment of the substrate.

Specifically, the pattern formed in the step 3 may be used as a mask to process the substrate (or the underlayer film and the substrate), thereby forming a pattern in the substrate.

The method of processing the substrate (or the underlayer film and the substrate) is not particularly limited, but is preferably a method of subjecting the substrate (or the underlayer film and the substrate) to dry etching using the pattern formed in the step 3 as a mask, thereby forming a pattern in the substrate. The dry etching is preferably oxygen plasma etching.

The resist composition and various materials used in the pattern forming method in the present specification (for example, the solvent, the developer, the rinsing liquid, the antireflection film-forming composition, and the topcoat-forming composition) preferably do not include impurities such as metal. The content of impurities included in such materials is preferably 1 mass ppm or less, more preferably 10 mass ppb or less, still more preferably 100 mass ppt or less, particularly preferably 10 mass ppt or less, most preferably 1 mass ppt or less. The lower limit is not particularly limited, and is preferably 0 mass ppt or more. Examples of metal impurities include Na, K, Ca, Fe, Cu, Mg, Al, Li, Cr, Ni, Sn, Ag, As, Au, Ba, Cd, Co, Pb, Ti, V, W, and Zn.

The method of removing impurities, such as metal, from the various materials may be, for example, filtration using a filter. Details of filtration using a filter are described in paragraph [0321] of WO2020/004306A.

Examples of the method of reducing the amount of impurities such as metal included in the various materials include a method of selecting, as raw materials constituting the various materials, raw materials having low metal contents, a method of subjecting raw materials constituting the various materials to filter filtration, and a method of performing distillation under conditions in which contamination is minimized by, for example, lining the interior of apparatuses with Teflon (registered trademark).

Instead of filter filtration, an adsorbing material may be used to remove impurities; alternatively, filter filtration and an adsorbing material may be used in combination. Publicly known adsorbing materials can be used as such adsorbing materials, and, for example, inorganic adsorbing material such as silica gel and zeolite, and organic adsorbing materials such as activated carbon can be used. In order to reduce the amount of impurities such as metal included in the various materials, mixing of metal impurities in the production process needs to be prevented. Whether or not metal impurities are sufficiently removed from the production apparatuses can be determined by measuring the content of metal components included in a washing liquid that has been used for washing the production apparatuses. The content of metal components included in the washing liquid that has been used is preferably 100 mass ppt (parts per trillion) or less, more preferably 10 mass ppt or less, still more preferably 1 mass ppt or less. The lower limit is not particularly limited, and is preferably 0 mass ppt or more.

A conductive compound may be added to an organic treatment liquid such as a rinsing liquid in order to prevent failure of a chemical liquid pipe and various parts (such as a filter, an O-ring, and a tube) caused by electrostatic charging and subsequent electrostatic discharging. The conductive compound is not particularly limited, but may be, for example, methanol. The amount of addition is not particularly limited, but is preferably 10 mass% or less, more preferably 5 mass% or less from the viewpoint of maintaining preferred developing performance or rinsing performance. The lower limit is not

particularly limited, and is preferably 0.01 mass% or more.

Examples of the chemical liquid pipe that can be used include various pipes made of SUS (stainless steel) or a material coated with polyethylene, polypropylene, or a fluororesin (such as polytetrafluoroethylene or a perfluoroalkoxy resin) subjected to antistatic treatment. Similarly, for the filter and the O-ring, polyethylene, polypropylene, or a fluororesin (such as polytetrafluoroethylene or a perfluoroalkoxy resin) subjected to antistatic treatment can be used.

[Method for producing electronic device]

The present specification also relates to a method for producing an electronic device, the method including the above-described pattern forming method, and an electronic device produced by the production method.

In a preferred embodiment of the electronic device in the present specification, the electronic device is mounted on electric or electronic devices (such as household appliances, OA (Office Automation), media-related devices, optical devices, and communication devices).

EXAMPLES

Hereinafter, the present invention will be described in more detail with reference to Examples. Materials, amounts used, ratios, details of processes, procedures of processes, and the like described in the following Examples may be appropriately changed without departing from the spirit and scope of the present invention. Accordingly, the scope of the present invention should not be construed as being limited to the following Examples.

[Various components of actinic ray-sensitive or radiation-sensitive resin composition]

[Resin (A)]

Resins (A) (resins A-1 to A-12) shown in Table 2 below will be described below.

The resins A used were synthesized in accordance with a method for synthesizing a resin A-1 (Synthesis Example 1) described later or a publicly known method.

Table 1 shows the contents of repeating units shown below (mol%, in order from the left), the weight-average molecular weight (Mw), and the polydispersity (Mw/Mn).

The weight-average molecular weight (Mw), the number-average molecular weight (Mn), and the polydispersity (Mw/Mn) of each of the resins A-1 to A-12 were measured as polystyrene equivalent values determined, using a GPC (Gel Permeation Chromatography) apparatus (HLC-8120GPC, manufactured by Tosoh Corporation), by GPC measurement (solvent: tetrahydrofuran, amount of flow (amount of sample injected): 10 μL, column: TSK gel Multipore HXL-M, manufactured by Tosoh Corporation, column temperature: 40°C, flow rate: 1.0 mL/min, detector: differential refractive index detector.

Table 1

| Resin (A) | Content of repeating unit (mol%) | | | | | Mw | Mw/Mn |
|---|---|---|---|---|---|---|---|
| | Repeating unit 1 | Repeating unit 2 | Repeating unit 3 | Repeating unit 4 | Repeating unit 5 | | |
| A-1 | 24 | 8 | 68 | | | 12,000 | 1.50 |
| A-2 | 24 | 4 | 3 | 69 | | 9,000 | 1.50 |
| A-3 | 20 | 10 | 15 | 55 | | 12,000 | 1.50 |
| A-4 | 20 | 5 | 5 | 15 | 55 | 12,000 | 1.50 |
| A-5 | 20 | 80 | | | | 8,600 | - |
| A-6 | 30 | 70 | | | | 8,200 | - |
| A-7 | 20 | 65 | 15 | | | 9,000 | - |
| A-8 | 24 | 8 | 68 | | | 12,000 | 1.10 |
| A-9 | 24 | 4 | 3 | 69 | | 9,000 | 1.10 |
| A-10 | 24 | 8 | 68 | | | 12,000 | 1.10 |
| A-11 | 24 | 8 | 68 | | | 12,000 | 1.10 |

(continued)

| Resin (A) | Content of repeating unit (mol%) | | | | | Mw | Mw/Mn |
|---|---|---|---|---|---|---|---|
| | Repeating unit 1 | Repeating unit 2 | Repeating unit 3 | Repeating unit 4 | Repeating unit 5 | | |
| A-12 | 24 | 8 | 68 | | | 12,000 | 1.10 |

**A-1**

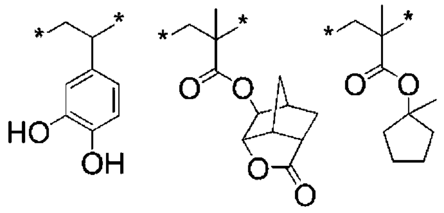

**A-2**

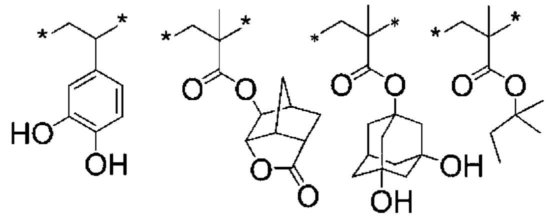

**A-3**

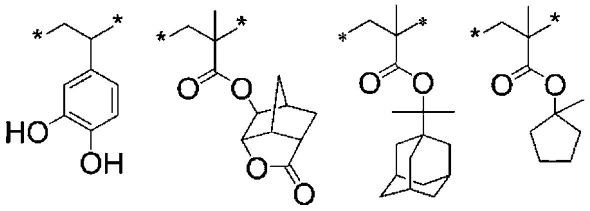

**A-4**

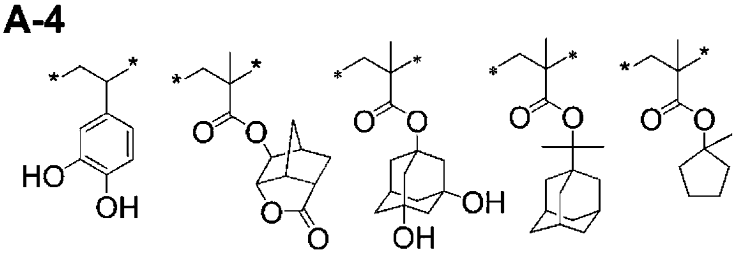

**A-5, 6**

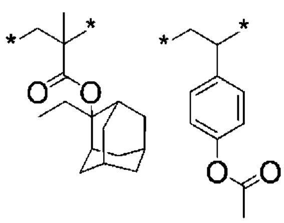

**A-7**

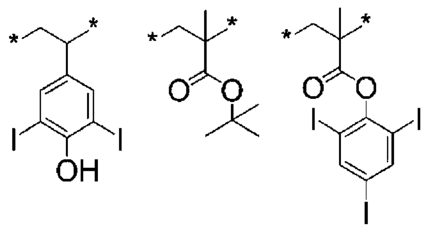

**A-8**

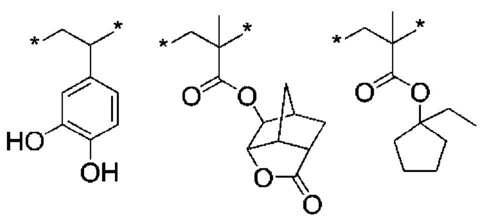

**A-9**

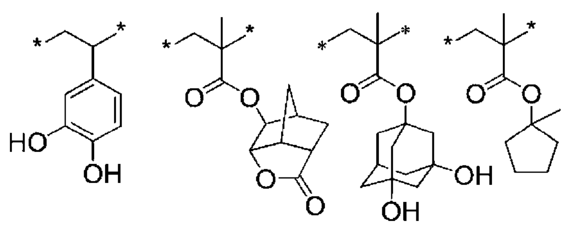

**A-10**

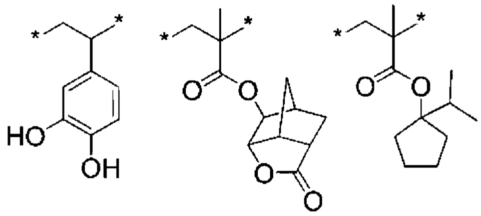

**A-11**

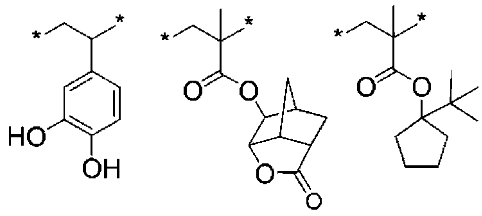

**A-12**

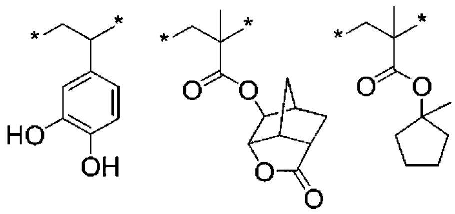

<Synthesis Example 1: Synthesis of resin A-1>

In a nitrogen stream, cyclohexanone (194.3 g) was placed in a three-necked flask and heated to 80°C. In order that the resin A-1 had the repeating units shown in Table 1, monomers from which the repeating units were derived were added thereto, and a solution prepared by dissolving a polymerization initiator V-601 (manufactured by FUJIFILM Wako Pure Chemical Corporation, 2.17 g) in cyclohexanone (105 g) was further added dropwise over six hours. After completion of the dropwise addition, the reaction was further performed at 80°C for two hours. The resulting reaction solution was left to cool and then added dropwise to a liquid mixture of methanol and water over 20 minutes. Subsequently, a precipitate precipitated by the dropwise addition was collected by filtration and dried to obtain the resin A-1 (31.6 g).

[Boron-containing compound]

<Cationic moiety>

The structures of cationic moieties (cations B-1 to B-17) of the boron-containing compounds shown in Table 2 below are shown below.

**B-1**

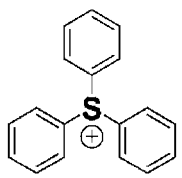

**B-2**

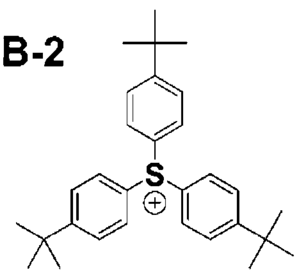

**B-3**

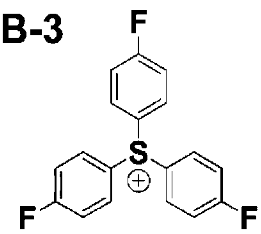

**B-4**

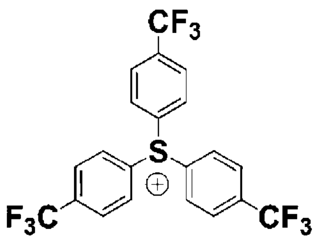

**B-5**

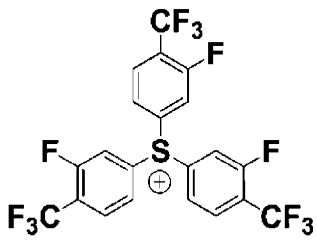

**B-6**

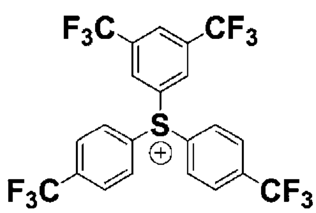

B-7

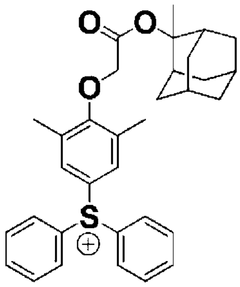

B-8

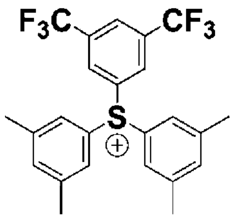

B-9

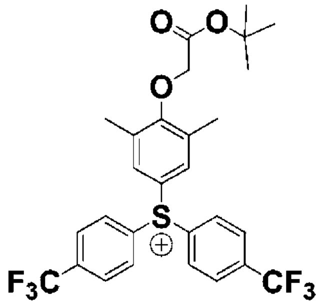

B-10

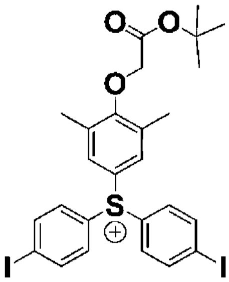

B-11

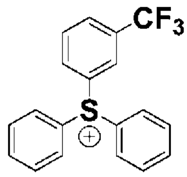

B-12

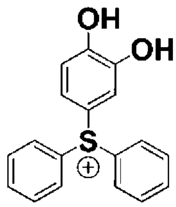

B-13

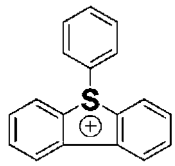

B-14

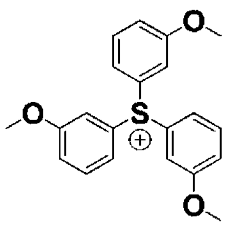

B-15

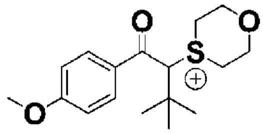

B-16

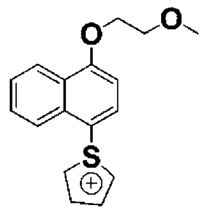

B-17

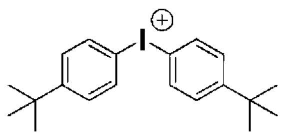

<Anionic moiety>

The structures of anionic moieties (anions C-1 to C-24) of the boron-containing compounds shown in Table 2 below are shown below.

C-1

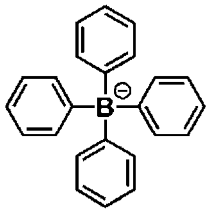

C-2

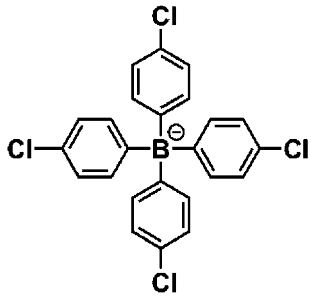

C-3

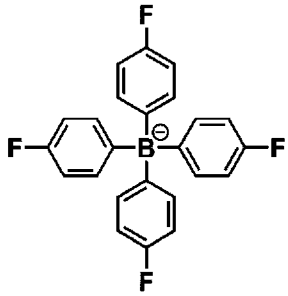

C-4

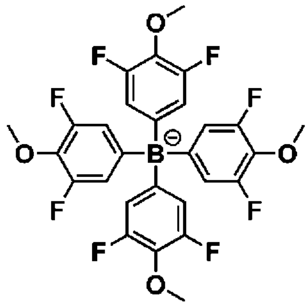

C-5

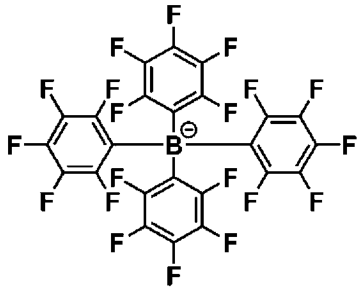

C-6

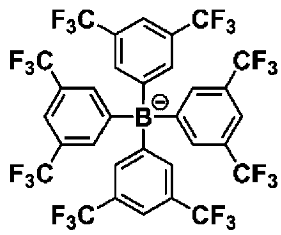

C-7

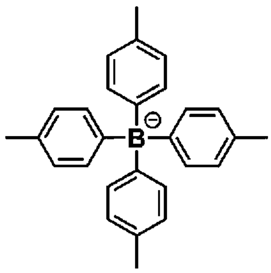

C-8

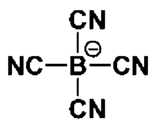

C-9

$BF_4^{\ominus}$

C-10

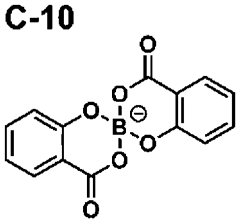

C-11

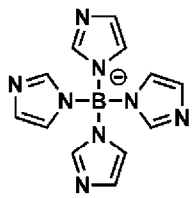

C-12

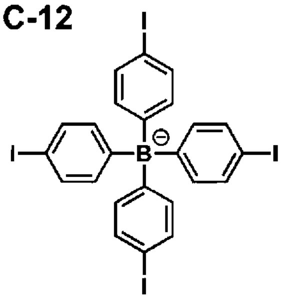

C-13

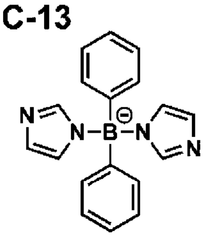

C-14

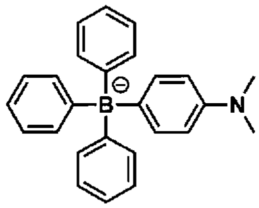

C-15

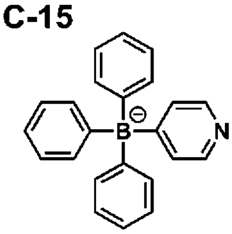

C-16

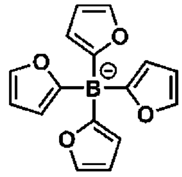

C-17

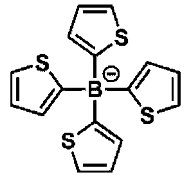

C-18

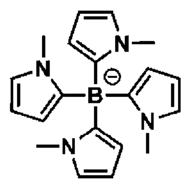

C-19

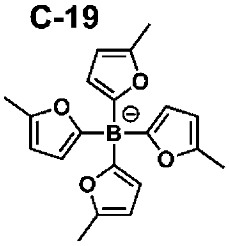

C-20

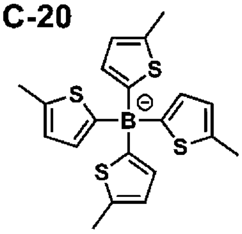

C-21

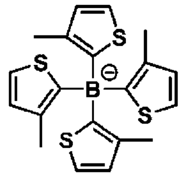

C-22

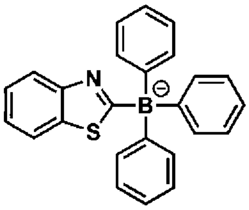

C-23

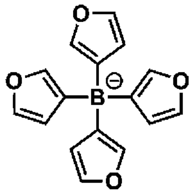

C-24

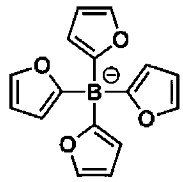

[Photoacid generator]

<Cationic moiety>

The structures of cationic moieties of the photoacid generators shown in Table 2 below are the same as those of the cationic moieties (cations B-1 to B-17) of the boron-containing compounds shown in the upper part.

<Anionic moiety>

The structures of anionic moieties (anions D-1 to D-18) of the photoacid generators shown in Table 2 below are shown below.

D-1

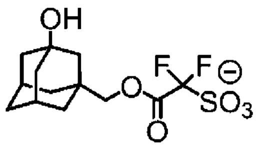

D-2

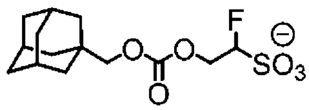

D-3

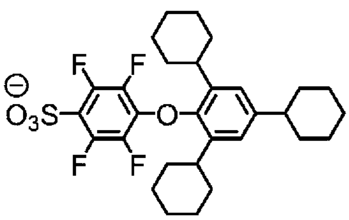

D-4

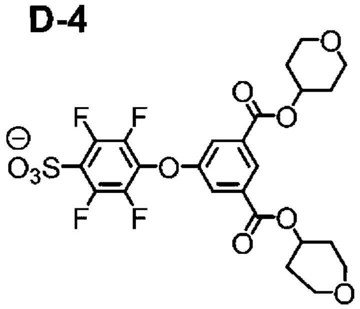

D-5

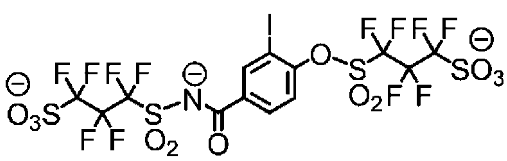

D-6

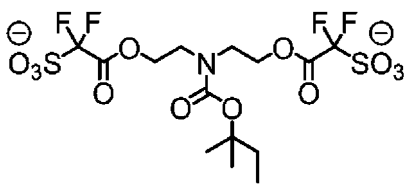

D-7

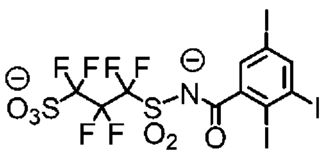

D-8

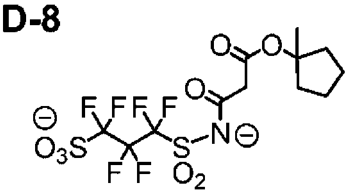

D-9

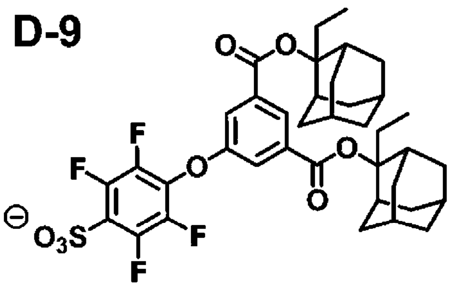

D-10

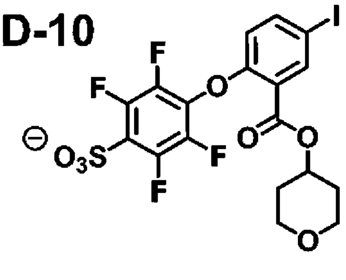

D-11

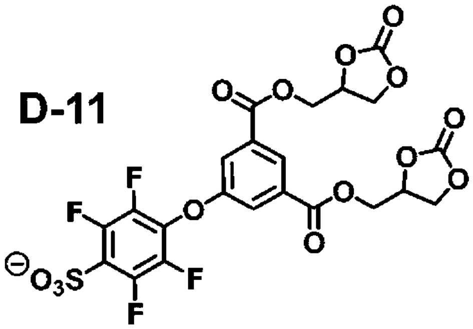

D-12

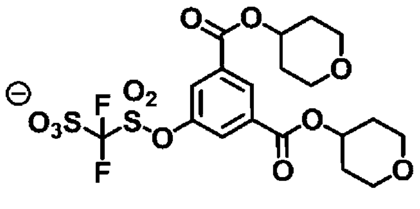

D-13

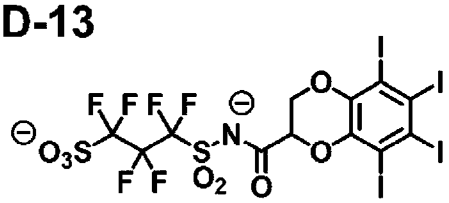

D-14

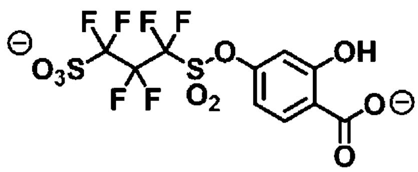

D-15

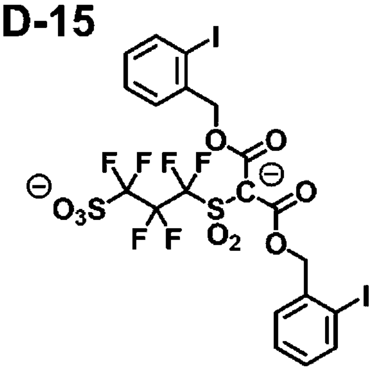

D-16

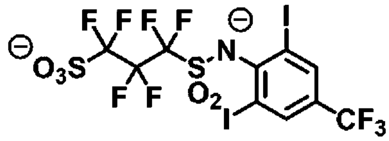

D-17

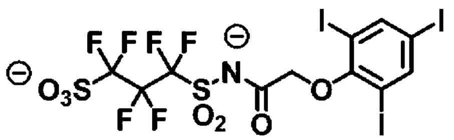

D-18

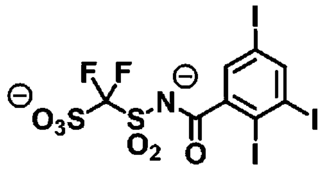

[Photodegradable base]

<Cationic moiety>

The structures of cationic moieties of the photodegradable bases shown in Table 2 below are the same as those of the cationic moieties (cations B-1 to B-17) of the boron-containing compounds shown in the upper part.

<Anionic moiety>

The structures of anionic moieties (anions E-1 to E-4) of the photodegradable bases shown in Table 2 below are shown below.

E-1

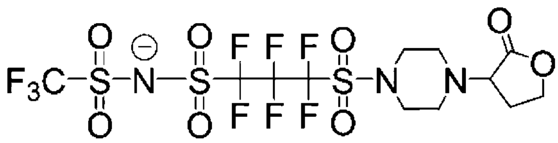

E-2

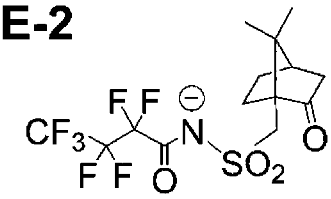

E-3

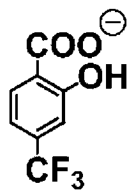

E-4

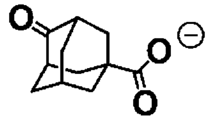

[Basic compound]

The structures of the basic compounds (basic compounds F-1 to F-4) shown in Table 2 below are shown below.

F-1

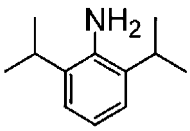

F-2

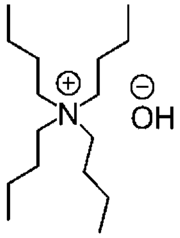

F-3

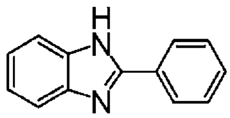

F-4

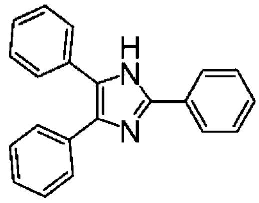

[Solvent]

The solvents (solvents G-1 to G-6) shown in Table 2 below are described below.

G-1: Propylene glycol monomethyl ether acetate (PGMEA)
G-2: Propylene glycol monomethyl ether (PGME)
G-3: Cyclohexanone
G-4: Ethyl lactate
G-5: γ-Butyrolactone
G-6: Diacetone alcohol

[Developer and rinsing liquid]

The developers and rinsing liquids shown in Tables 3 and 4 below are described below.

H-1: 2.38 wt% TMAH aqueous solution
H-2: Butyl acetate
H-3: Isopropyl acetate
H-4: Butyl acetate:n-Undecane = 90:10 (mass ratio)
H-5: Butyl acetate:n-Undecane = 80:20 (mass ratio)
H-6: Butyl acetate:n-Undecane = 70:30 (mass ratio)

[Preparation of resist composition]

Various components shown in Table 2 were mixed to prepare a liquid mixture, and the liquid mixture was filtered through a polyethylene filter having a pore size of 0.03 μm to prepare a resist composition. The concentration of solid contents in the resist composition was appropriately adjusted such that the resist composition could be applied in a film thickness shown in Tables 3 and 4. Solid contents means all components other than solvents.

Table 2 is shown below.

In the "Optical absorption properties" column in Table 2, the "Value A" is a value calculated based on the equation (1) described above. In the "Value B1 or Value B2" column, the "Value B1" is a value calculated based on the equation (2) described above, and the "Value B2" is a value calculated based on the equation (3) described above. Note that, in the "Value B1 or Value B2" column, specifically, the values B2 are shown for each of the compositions Re-7, Re-22, Re-23, Re-28, and Re-49, which do not include an acid diffusion control agent which is an onium salt compound. The values B1 are shown for compositions other than the above compositions and including an acid diffusion control agent which is an onium salt compound. In the "Value C1 or Value C2" column, the "Value C1" is a value calculated based on the equation (4) described above, and the "Value C2" is a value calculated based on the equation (5) described above. Note that, in the "Value C1 or Value C2" column, specifically, the values C2 are shown for each of the compositions Re-7, Re-22, Re-23, Re-28, and Re-49, which do not include an acid diffusion control agent which is a salt compound. The values C1 are shown for compositions other than the above compositions and including an acid diffusion control agent which is a salt compound.

| Table 2 (Part 1) | Composition of resist composition (mass%) | | | | | | | | | | | | | | | | | |
|---|---|---|---|---|---|---|---|---|---|---|---|---|---|---|---|---|---|---|
| | Resin (A) | | Boron-containing compound | | | Photoacid generator | | | Acid diffusion control agent | | | | | Solvent | | Optical absorption properties | | |
| | | | | | | | | | Photodegradable base | | | Basic compound | | | | | | |
| | Type | Content | Type of cationic moiety (B) | Type of anionic moiety (C) | Content | Type of cationic moiety (B) | Type of anionic moiety (D) | Content | Type of cationic moiety (B) | Type of anionic moiety (E) | Content | Type | Content | Type | Mass ratio | Value A | Value B1 or Value B2 | Value C1 or Value C2 |
| Re-1 | A-1 | 55 | B-6 | C-1 | 5 | B-6 | D-5 | 35 | B-6 | E-2 | 5 | - | - | G-1/-G-2/G-5 | 79/20/1 | 0.139 | 0.170 | 0.170 |
| Re-2 | A-7 | 65 | B-1 | C-1 | 5 | B-1 | D-6 | 25 | B-1 | E-2 | 5 | - | - | G-1/-G-2/G-5 | 79/20/1 | 0.168 | 0.076 | 0.076 |
| Re-3 | A-7 | 65 | B-6 | C-1 | 5 | B-6 | D-5 | 25 | B-6 | E-2 | 5 | - | - | G-1/-G-2/G-5 | 79/20/1 | 0.189 | 0.170 | 0.170 |
| Re-7 | A-1 | 57 | B-4 | C-1 | 13 | B-4 | D-15 | 20 | - | - | - | F-1 | 10 | G-1/G-2 | 80/20 | 0.123 | 0.157 | 0.157 |
| Re-8 | A-2 | 59 | B-4 | C-1 | 10 | B-4 | D-11<br>D-13 | 8<br>18 | B-4 | E-2 | 5 | - | - | G-1/G-2 | 70/30 | 0.135 | 0.157 | 0.157 |
| Re-9 | A-3 | 57 | B-4 | C-1 | 12 | B-4 | D-9 | 28 | B-4 | E-2 | 3 | - | - | G-1/-G-2/G-5 | 70/25/5 | 0.120 | 0.157 | 0.157 |
| Re-10 | A-4 | 53 | B-4 | C-1 | 7 | B-4 | D-14 | 32 | B-4 | E-2 | 8 | - | - | G-1/G-2 | 80/20 | 0.131 | 0.157 | 0.157 |
| Re-11 | A-7 | 56 | B-2 | C-7 | 5 | B-2 | D-5 | 29 | B-2 | E-2 | 10 | - | - | G-1/G-2 | 40/60 | 0.163 | 0.077 | 0.077 |
| Re-12 | A-8 | 60 | B-4 | C-1 | 11 | B-4 | D-10 | 25 | B-4 | E-2 | 4 | - | - | G-1/-G-2/G-5 | 80/10/10 | 0.128 | 0.157 | 0.157 |
| Re-13 | A-9 | 58 | B-4 | C-1 | 9 | B-4 | D-16 | 27 | B-4 | E-2 | 6 | - | - | G-1/G-2 | 70/30 | 0.135 | 0.157 | 0.157 |
| Re-14 | A-10 | 62 | B-4 | C-1 | 3 | B-4 | D-2<br>D-7 | 10<br>20 | - | - | - | F-2 | 5 | G-1/G-3 | 90/10 | 0.127 | 0.158 | 0.158 |
| Re-15 | A-11 | 62 | B-4 | C-1 | 8 | B-4 | D-13 | 23 | B-4 | E-2 | 7 | - | - | G-1/G-6 | 50/50 | 0.133 | 0.157 | 0.157 |
| Re-16 | A-7 | 61 | B-3 | C-8 | 6 | B-3 | D-9 | 24 | B-3 | E-2<br>E-3 | 6<br>3 | - | - | G-1/G-2 | 70/30 | 0.170 | 0.116 | 0.116 |

| Table 2 (Part 2) | Composition of resist composition (mass%) | | | | | | | | | | | | | | | | | |
|---|---|---|---|---|---|---|---|---|---|---|---|---|---|---|---|---|---|---|
| | Resin (A) | | Boron-containing compound | | | Photoacid generator | | | Acid diffusion control agent | | | | | Solvent | | Optical absorption properties | | |
| | | | | | | | | | Photodegradable base | | | Basic compound | | | | | | |
| | Type | Content | Type of cationic moiety (B) | Type of anionic moiety (C) | Content | Type of cationic moiety (B) | Type of anionic moiety (D) | Content | Type of cationic moiety (B) | Type of anionic moiety (E) | Content | Type | Content | Type | Mass ratio | Value A | Value B1 or Value B2 | Value C1 or Value C2 |
| Re-17 | A-12 | 64 | B-5 | C-1 | 4 | B-5 | D-12 | 21 | B-5 | E-2 | 11 | - | - | G-1/G-2 | 80/20 | 0.127 | 0.173 | 0.173 |
| Re-18 | A-1 | 67 | B-5 | C-1 | 3 | B-5 | D-17 | 20 | B-5 | E-2 | 10 | - | - | G-1/G-2 | 70/30 | 0.135 | 0.173 | 0.173 |
| Re-19 | A-2 | 61 | B-5 | C-1 | 8 | B-5 | D-6 | 26 | B-5 | E-2 | 5 | - | - | G-1/G-2 | 80/20 | 0.131 | 0.173 | 0.173 |
| Re-20 | A-3 | 58 | B-5 | C-1 | 6 | B-5 | D-11 | 28 | B-5 | E-2 | 8 | - | - | G-1/G-4 | 80/20 | 0.130 | 0.173 | 0.173 |
| Re-21 | A-7 | 59 | B-7 | C-9 | 4 | B-7 | D-12<br>D-14 | 10<br>22 | B-7 | E-2 | 5 | - | - | G-1/-G-2/G-5 | 80/10/10 | 0.165 | 0.091 | 0.091 |
| Re-22 | A-4 | 54 | B-4 | C-1 | 10 | B-4 | D-4 | 31 | - | - | - | F-3 | 5 | G-1/G-2 | 80/20 | 0.122 | 0.157 | 0.157 |
| Re-23 | A-8 | 52 | B-5 | C-1 | 13 | B-5 | D-7<br>D-12 | 7<br>18 | - | - | - | F-4 | 10 | G-2/G-4 | 50/50 | 0.125 | 0.173 | 0.173 |
| Re-24 | A-9 | 59 | B-8 | C-1 | 10 | B-8 | D-10 | 27 | B-8 | E-2 | 4 | - | - | G-2/G-3 | 30/70 | 0.125 | 0.132 | 0.132 |
| Re-25 | A-10 | 53 | B-9 | C-1 | 12 | B-9 | D-8 | 30 | B-9 | E-2 | 5 | - | - | G-3/G-4 | 50/50 | 0.121 | 0.135 | 0.135 |
| Re-26 | A-7 | 61 | B-11 | C-14 | 7 | B-11 | D-15 | 23 | B-11 | E-2 | 9 | - | - | G-4/G-5 | 40/60 | 0.178 | 0.114 | 0.114 |
| Re-27 | A-11 | 58 | B-10 | C-1 | 5 | B-10 | D-8 | 24 | B-10 | E-2<br>E-4 | 7<br>6 | - | - | G-1/G-6 | 20/80 | 0.131 | 0.176 | 0.176 |
| Re-28 | A-12 | 55 | B-4 | C-1 | 15 | B-4 | D-9 | 30 | - | - | - | - | - | G-2/G-5 | 30/70 | 0.120 | 0.157 | 0.157 |
| Re-29 | A-11 | 53 | B-4 | C-1 | 10 | B-4 | D-14 | 32 | B-4 | E-2 | 5 | - | - | G-1/-G-2/G-5 | 85/10/5 | 0.129 | 0.157 | 0.157 |
| Re-30 | A-2 | 51 | B-4 | C-1 | 12 | B-4 | D-5 | 29 | B-4 | E-2 | 8 | - | - | G-1/G-3 | 90/10 | 0.135 | 0.157 | 0.157 |
| Re-31 | A-7 | 59 | B-12 | C-15 | 7 | B-12 | D-13 | 25 | B-12 | E-2 | 9 | - | - | G-1/G-2 | 80/20 | 0.181 | 0.093 | 0.093 |
| Re-32 | A-3 | 62 | B-4 | C-1 | 5 | B-4 | D-1 | 27 | B-4 | E-2 | 6 | - | - | G-2/G-4 | 50/50 | 0.123 | 0.157 | 0.157 |

| Table 2 (Part 3) | Composition of resist composition (mass%) | | | | | | | | | | | | | | | | |
|---|---|---|---|---|---|---|---|---|---|---|---|---|---|---|---|---|---|
| | Resin (A) | | Boron-containing compound | | | Photoacid generator | | | Acid diffusion control agent | | | | | Solvent | | Optical absorption properties | | |
| | | | | | | | | | Photodegradable base | | | Basic compound | | | | | | |
| | Type | Content | Type of cationic moiety (B) | Type of anionic moiety (C) | Content | Type of cationic moiety (B) | Type of anionic moiety (D) | Content | Type of cationic moiety (B) | Type of anionic moiety (E) | Content | Type | Content | Type | Mass ratio | Value A | Value B1 or Value B2 | Value C1 or Value C2 |
| Re-33 | A-4 | 55 | B-4 | C-1 | 11 | B-4 | D-11 | 30 | B-4 | E-2 | 4 | - | - | G-1/G-2 | 40/60 | 0.127 | 0.157 | 0.157 |
| Re-34 | A-8 | 60 | B-4 | C-1 | 9 | B-4 | D-16 | 23 | B-4 | E-2 | 8 | - | - | G-1/-G-2/G-5 | 80/10/10 | 0.132 | 0.157 | 0.157 |
| Re-35 | A-9 | 68 | B-4 | C-1 | 3 | B-4 | D-7 | 24 | B-4 | E-2 | 5 | - | - | G-1/G-2 | 80/20 | 0.133 | 0.157 | 0.157 |
| Re-36 | A-7 | 68 | B-13 | C-16 | 8 | B-13 | D-4 | 21 | B-13 | E-3 | 3 | - | - | G-1/G-2 | 80/20 | 0.171 | 0.077 | 0.077 |
| Re-37 | A-10 | 62 | B-4 | C-1 | 6 | B-4 | D-10 | 20 | B-4 | E-3 | 12 | - | - | G-1/G-2 | 80/20 | 0.127 | 0.157 | 0.157 |
| Re-38 | A-11 | 61 | B-4 | C-2 | 4 | B-4 | D-11 | 26 | B-4 | E-3 | 9 | - | - | G-2/G-3 | 30/70 | 0.126 | 0.157 | 0.157 |
| Re-39 | A-12 | 62 | B-4 | C-3 | 3 | B-4 | D-14 | 28 | B-4 | E-3 | 7 | - | - | G-2/G-3 | 30/70 | 0.129 | 0.157 | 0.157 |
| Re-40 | A-1 | 54 | B-4 | C-1 | 20 | B-4 | D-10 | 21 | B-4 | E-3 | 5 | - | - | G-1/G-2 | 80/20 | 0.129 | 0.157 | 0.157 |
| Re-41 | A-7 | 64 | B-14 | C-17 | 10 | B-14 | D-4 | 23 | B-14 | E-3 | 3 | - | - | G-2/G-4 | 50/50 | 0.167 | 0.097 | 0.097 |
| Re-42 | A-2 | 54 | B-4 | C-4 | 12 | B-4 | D-9 | 24 | B-4 | E-3 | 10 | - | - | G-1/G-2 | 40/60 | 0.127 | 0.157 | 0.157 |
| Re-43 | A-3 | 55 | B-4 | C-5 | 7 | B-4 | D-12 | 26 | B-4 | E-3<br>E-4 | 6<br>6 | - | - | G-1/-G-2/G-5 | 80/10/10 | 0.129 | 0.157 | 0.157 |
| Re-44 | A-4 | 57 | B-4 | C-6 | 5 | B-4 | D-17 | 32 | B-4 | E-3 | 6 | - | - | G-2/G-3 | 30/70 | 0.142 | 0.157 | 0.157 |
| Re-45 | A-8 | 58 | B-4 | C-10 | 11 | B-4 | D-3 | 29 | B-4 | E-4 | 2 | - | - | G-3/G-4 | 50/50 | 0.122 | 0.157 | 0.157 |
| Re-46 | A-7 | 59 | B-15 | C-22 | 9 | B-15 | D-11 | 25 | B-15 | E-4 | 7 | - | - | G-1/G-2 | 80/20 | 0.166 | 0.098 | 0.098 |
| Re-47 | A-9 | 64 | B-4 | C-11 | 3 | B-4 | D-13 | 27 | B-4 | E-4 | 6 | - | - | G-1/G-2 | 70/30 | 0.137 | 0.157 | 0.157 |
| Re-48 | A-10 | 58 | B-4 | C-12 | 8 | B-4 | D-3 | 30 | B-4 | E-4 | 4 | - | - | G-1/-G-2/G-5 | 70/25/5 | 0.125 | 0.157 | 0.157 |

| Table 2 (Part 4) | Composition of resist composition (mass%) | | | | | | | | | | | | | | | | | |
|---|---|---|---|---|---|---|---|---|---|---|---|---|---|---|---|---|---|---|
| | Resin (A) | | Boron-containing compound | | | Photoacid generator | | | Acid diffusion control agent | | | | | Solvent | | Optical absorption properties | | |
| | | | | | | | | | Photodegradable base | | | Basic compound | | | | | | |
| | Type | Content | Type of cationic moiety (B) | Type of anionic moiety (C) | Content | Type of cationic moiety (B) | Type of anionic moiety (D) | Content | Type of cationic moiety (B) | Type of anionic moiety (E) | Content | Type | Content | Type | Mass ratio | Value A | Value B1 or Value B2 | Value C1 or Value C2 |
| Re-49 | A-11 | 59 | B-4 | C-16 | 25 | B-4 | D-11 | 16 | - | - | - | - | - | G-1/G-2 | 80/20 | 0.122 | 0.157 | 0.157 |
| Re-50 | A-12 | 62 | B-1 | C-13 | 6 | B-4 | D-6<br>D-10 | 6<br>17 | B-4 | E-4 | 9 | - | - | G-1/G-2 | 40/60 | 0.125 | 0.146 | 0.146 |
| Re-51 | A-7 | 61 | B-16 | C-23 | 4 | B-16 | D-10 | 24 | B-16 | E-4 | 11 | - | - | G-1/-G-2/G-5 | 80/10/10 | 0.173 | 0.092 | 0.092 |
| Re-52 | A-1 | 54 | B-1 | C-18 | 10 | B-4 | D-8 | 26 | B-4 | E-2<br>E-4 | 5<br>5 | - | - | G-1/G-2 | 70/30 | 0.125 | 0.144 | 0.144 |
| Re-53 | A-2 | 50 | B-1 | C-19 | 13 | B-4 | D-14 | 32 | B-4 | E-4 | 5 | - | - | G-1/G-3 | 90/10 | 0.127 | 0.142 | 0.142 |
| Re-54 | A-3 | 50 | B-1 | C-20 | 10 | B-4 | D-4 | 32 | B-1 | E-2 | 8 | - | - | G-1/G-6 | *50/50* | 0.120 | 0.131 | 0.131 |
| Re-55 | A-4 | 58 | B-1 | C-21 | 12 | B-4 | D-4<br>D-7 | 10<br>15 | B-1 | E-2 | 5 | - | - | G-1/G-2 | 70/30 | 0.122 | 0.129 | 0.129 |
| Re-56 | A-7 | 52 | B-17 | C-24 | 10 | B-17 | D-12 | 35 | B-17 | E-2 | 3 | - | - | G-1/G-2 | 80/20 | 0.173 | 0.154 | 0.154 |
| Re-57 | A-1 | 55 | B-4 | C-1 | 2 | B-4 | D-4<br>D-18 | 17<br>24 | B-4 | E-2 | 2 | - | - | G-1/-G-2/G-5 | 85/10/5 | 0.138 | 0.157 | 0.157 |
| Re-4 | A-5<br>A-6 | 42.5<br>42.5 | B-1 | C-4 | 1.7 | B-1 | D-1 | 12.78 | - | - | - | F-1<br>F-2 | 0.26<br>0.26 | G-1/-G-2/G-5 | 79/20/1 | 0.106 | 0.077 | 0.077 |
| Re-5 | A-5<br>A-6 | 30<br>30 | - | - | - | B-6 | D-5 | 35 | B-6 | E-1 | 5 | - | - | G-1/-G-2/G-5 | 79/20/1 | 0.135 | 0.170 | 0.170 |
| Re-6 | A-7 | 70 | - | - | - | B-1 | D-6 | 25 | B-1 | E-1 | 5 | - | - | G-1/-G-2/G-5 | 79/20/1 | 0.175 | 0.076 | 0.076 |

[Pattern formation and evaluation (1)]

[Pattern formation by EUV exposure]

An underlayer film-forming composition E2Stack (registered trademark) AL412 (manufactured by Brewer Science, Inc.) was applied to a silicon wafer and baked at 205°C for 60 seconds to form an underlayer film having a film thickness of 20 nm. The resist composition shown in Table 3 was applied thereto, and a resist film was formed under the conditions (film thickness and PreBake) shown in Table 3. Thus, a silicon wafer having a resist film was formed.

The silicon wafer having the resist film obtained by the above-described procedure was subjected to pattern irradiation using an EUV scanner NXE3400B (NA 0.33, dipole modified illumination (FlexRay)) manufactured by ASML. A mask having a line size of 25 nm and a line:space ratio of 1:1 was used as a reticle. Subsequently, only in the case where there is a description in Table 3, baking (Post Exposure Bake; PEB) was performed using a coater/developer CLEAN TRAC LITHIUS Pro Z manufactured by Tokyo Electron Ltd. under the conditions shown in Table 3; development was then performed with a developer shown in Table 3 by puddling for 30 seconds; only in the case where there is a description in Table 3, rinsing was performed by pouring a rinsing liquid shown in Table 3 for 10 seconds while the wafer was rotated at a rotational speed of 1,000 rpm; and the wafer was then rotated at a rotational speed of 4,000 rpm for 30 seconds to obtain a line-and-space pattern with a pitch of 50 nm.

[Evaluation]

<Optimum exposure dose (Sensitivity)>

In the above pattern formation by EUV exposure, a line width of the line-and-space pattern was measured using a critical dimension-scanning electron microscope (SEM: Scanning Electron Microscope (CG-6300, manufactured by Hitachi High-Tech Corporation)) while changing the exposure dose, an exposure dose at which the line width became 25 nm was determined, and this was defined as an optimum exposure dose ($mJ/cm^2$). The smaller this value, the better the performance.

<Space width at which bridge defects start to occur in formation of L/S pattern (Bridge margin)>

A line-and-space pattern (line:space = 1:1) having a line width of 25 nm was exposed while the irradiation dose was changed from the above sensitivity, and a space width at which a bridge starts to form between spaces was determined as an indicator of a "bridge margin". The smaller the above value, the better the performance.

<Line edge roughness (LER)>

With respect to randomly selected 30 points in 50 μm in the length direction of a line pattern (L/S = 1/1) having a line width of 25 nm and formed at the exposure dose providing the above sensitivity, the distance from a reference line on which the edge should be located was measured using a scanning electron microscope (CG-6300, manufactured by Hitachi High-Tech Corporation), the standard deviation was determined, and 3σ was calculated. The smaller the value, the better the performance.

<Resolution>

A limit resolving power (a minimum line width at which a line and a space (line:space = 1:1) were separately resolved) at the exposure dose providing the above sensitivity was defined as a resolution (unit: nm). The smaller the value, the better the performance.

Table 6

| Table 3 (Part 1) | Resist composition application conditions | | | PEB·Development conditions | | EUV evaluation results | | | |
|---|---|---|---|---|---|---|---|---|---|
| | Resist composition | Film thickness [nm] | PreBake | PEB | Developer | Bridge margin [nm] | Sensitivity [$mJ/cm^2$] | LER [nm] | Resolution [nm] |
| Example 1-1 | Re-1 | 30 | 90°C/60 sec | 110°C/60 sec | H-1 | 14 | 45 | 1.8 | 16.0 |

(continued)

| Table 3 (Part 1) | Resist composition application conditions | | | PEB·Development conditions | | EUV evaluation results | | | |
|---|---|---|---|---|---|---|---|---|---|
| | Resist composition | Film thickness [nm] | PreBake | PEB | Developer | Bridge margin [nm] | Sensitivity [mJ/cm$^2$] | LER [nm] | Resolution [nm] |
| Example 1-2 | Re-2 | 30 | 90°C/60 sec | 110°C/60 sec | H-1 | 21 | 50 | 2.0 | 21.0 |
| Example 1-3 | Re-3 | 30 | 90°C/60 sec | 110°C/60 sec | H-1 | 8 | 30 | 1.1 | 10.0 |
| Example 1-4 | Re-1 | 30 | 90°C/60 sec | 110°C/60 sec | H-5 | 11 | 38 | 1.7 | 13.0 |
| Example 1-5 | Re-7 | 30 | 90°C/60 sec | 110°C/60 sec | H-1 | 17 | 56 | 2.1 | 19.0 |
| Example 1-6 | Re-8 | 30 | 90°C/60 sec | 110°C/60 sec | H-1 | 10 | 42 | 1.6 | 15.0 |
| Example 1-7 | Re-9 | 30 | 90°C/60 sec | 110°C/60 sec | H-1 | 15 | 49 | 1.9 | 16.0 |
| Example 1-8 | Re-10 | 30 | 90°C/60 sec | 110°C/60 sec | H-1 | 10 | 41 | 1.7 | 15.0 |
| Example 1-9 | Re-11 | 30 | 90°C/60 sec | 110°C/60 sec | H-1 | 21 | 46 | 1.8 | 21.0 |
| Example 1-10 | Re-12 | 30 | 90°C/60 sec | 110°C/60 sec | H-1 | 12 | 45 | 1.8 | 15.0 |
| Example 1-11 | Re-13 | 30 | 90°C/60 sec | 110°C/60 sec | H-1 | 10 | 41 | 1.6 | 15.0 |
| Example 1-12 | Re-14 | 30 | 90°C/60 sec | 110°C/60 sec | H-1 | 16 | 50 | 2.0 | 17.0 |
| Example 1-13 | Re-15 | 30 | 90°C/60 sec | 110°C/60 sec | H-1 | 11 | 42 | 1.7 | 15.0 |
| Example 1-14 | Re-16 | 30 | 90°C/60 sec | 110°C/60 sec | H-1 | 8 | 33 | 1.2 | 11.0 |
| Example 1-15 | Re-17 | 30 | 90°C/60 sec | 110°C/60 sec | H-1 | 9 | 37 | 1.4 | 14.0 |
| Example 1-16 | Re-18 | 30 | 90°C/60 sec | 110°C/60 sec | H-1 | 9 | 35 | 1.4 | 13.0 |
| Example 1-17 | Re-19 | 30 | 90°C/60 sec | 110°C/60 sec | H-1 | 9 | 36 | 1.3 | 14.0 |
| Example 1-18 | Re-20 | 30 | 90°C/60 sec | 110°C/60 sec | H-1 | 9 | 38 | 1.4 | 14.0 |
| Example 1-19 | Re-21 | 30 | 90°C/60 sec | 110°C/60 sec | H-1 | 21 | 46 | 1.8 | 21.0 |
| Example 1-20 | Re-22 | 30 | 90°C/60 sec | 110°C/60 sec | H-1 | 18 | 52 | 2.3 | 18.0 |
| Example 1-21 | Re-23 | 30 | 90°C/60 sec | 110°C/60 sec | H-1 | 14 | 47 | 1.9 | 15.0 |
| Example 1-22 | Re-24 | 30 | 90°C/60 sec | 110°C/60 sec | H-1 | 14 | 47 | 1.8 | 15.0 |

(continued)

| Table 3 (Part 1) | Resist composition application conditions | | | PEB·Development conditions | | EUV evaluation results | | | |
|---|---|---|---|---|---|---|---|---|---|
| | Resist composition | Film thickness [nm] | PreBake | PEB | Developer | Bridge margin [nm] | Sensitivity [mJ/cm$^2$] | LER [nm] | Resolution [nm] |
| Example 1-23 | Re-25 | 30 | 90°C/60 sec | 110°C/60 sec | H-1 | 15 | 49 | 1.9 | 15.0 |
| Example 1-24 | Re-26 | 30 | 90°C/60 sec | 110°C/60 sec | H-1 | 21 | 39 | 1.5 | 21.0 |
| Example 1-25 | Re-27 | 30 | 90°C/60 sec | 110°C/60 sec | H-1 | 9 | 37 | 1.3 | 14.0 |
| Example 1-26 | Re-28 | 30 | 90°C/60 sec | 110°C/60 sec | H-1 | 20 | 60 | 1.8 | 20.0 |
| Example 1-27 | Re-29 | 30 | 90°C/60 sec | 110°C/60 sec | H-1 | 13 | 45 | 1.8 | 15.0 |
| Example 1-28 | Re-30 | 30 | 90°C/60 sec | 110°C/60 sec | H-1 | 10 | 42 | 1.6 | 15.0 |
| Example 1-29 | Re-31 | 30 | 90°C/60 sec | 110°C/60 sec | H-1 | 21 | 46 | 1.8 | 21.0 |
| Example 1-30 | Re-32 | 30 | 90°C/60 sec | 110°C/60 sec | H-1 | 14 | 47 | 1.9 | 15.0 |
| Example 1-31 | Re-33 | 30 | 90°C/60 sec | 110°C/60 sec | H-1 | 14 | 48 | 1.8 | 15.0 |
| Example 1-32 | Re-34 | 30 | 90°C/60 sec | 110°C/60 sec | H-1 | 11 | 42 | 1.7 | 15.0 |
| Example 1-33 | Re-35 | 30 | 90°C/60 sec | 110°C/60 sec | H-1 | 11 | 43 | 1.8 | 15.0 |
| Example 1-34 | Re-36 | 30 | 90°C/60 sec | 110°C/60 sec | H-1 | 21 | 46 | 1.8 | 21.0 |
| Example 1-35 | Re-37 | 30 | 90°C/60 sec | 110°C/60 sec | H-1 | 13 | 46 | 1.8 | 15.0 |
| Example 1-36 | Re-38 | 30 | 90°C/60 sec | 110°C/60 sec | H-1 | 13 | 45 | 1.7 | 15.0 |
| Example 1-37 | Re-39 | 30 | 90°C/60 sec | 110°C/60 sec | H-1 | 13 | 46 | 1.8 | 15.0 |

Table 7

| Table 3 (Part 2) | Resist composition application conditions | | | PEB·Development conditions | | EUV evaluation results | | | |
|---|---|---|---|---|---|---|---|---|---|
| | Resist composition | Film thickness [nm] | PreBake | PEB | Developer | Bridge margin [nm] | Sensitivity [mJ/cm$^2$] | LER [nm] | Resolution [nm] |
| Example 1-38 | Re-40 | 30 | 90°C/60 sec | 110°C/60 sec | H-1 | 13 | 46 | 1.8 | 15.0 |
| Example 1-39 | Re-41 | 30 | 90°C/60 sec | 110°C/60 sec | H-1 | 21 | 46 | 1.8 | 21.0 |
| Example 1-40 | Re-42 | 30 | 90°C/60 sec | 110°C/60 sec | H-1 | 13 | 45 | 1.8 | 15.0 |
| Example 1-41 | Re-43 | 30 | 90°C/60 sec | 110°C/60 sec | H-1 | 13 | 46 | 1.8 | 15.0 |
| Example 1-42 | Re-44 | 30 | 90°C/60 sec | 110°C/60 sec | H-1 | 10 | 41 | 1.7 | 15.0 |
| Example 1-43 | Re-45 | 30 | 90°C/60 sec | 110°C/60 sec | H-1 | 15 | 48 | 1.9 | 15.0 |
| Example 1-44 | Re-46 | 30 | 90°C/60 sec | 110°C/60 sec | H-1 | 21 | 46 | 1.8 | 21.0 |
| Example 1-45 | Re-47 | 30 | 90°C/60 sec | 110°C/60 sec | H-1 | 10 | 41 | 1.6 | 15.0 |
| Example 1-46 | Re-48 | 30 | 90°C/60 sec | 110°C/60 sec | H-1 | 13 | 46 | 1.8 | 15.0 |
| Example 1-47 | Re-49 | 30 | 90°C/60 sec | 110°C/60 sec | H-1 | 20 | 60 | 1.7 | 20.0 |
| Example 1-48 | Re-50 | 30 | 90°C/60 sec | 110°C/60 sec | H-1 | 13 | 45 | 1.8 | 15.0 |
| Example 1-49 | Re-51 | 30 | 90°C/60 sec | 110°C/60 sec | H-1 | 21 | 46 | 1.8 | 21.0 |
| Example 1-50 | Re-52 | 30 | 90°C/60 sec | 110°C/60 sec | H-1 | 13 | 45 | 1.8 | 15.0 |
| Example 1-51 | Re-53 | 30 | 90°C/60 sec | 110°C/60 sec | H-1 | 12 | 44 | 1.8 | 15.0 |
| Example 1-52 | Re-54 | 30 | 90°C/60 sec | 110°C/60 sec | H-1 | 15 | 48 | 1.9 | 15.0 |
| Example 1-53 | Re-55 | 30 | 90°C/60 sec | 110°C/60 sec | H-1 | 15 | 49 | 1.9 | 15.0 |
| Example 1-54 | Re-56 | 30 | 90°C/60 sec | 110°C/60 sec | H-1 | 8 | 31 | 1.2 | 10.0 |
| Example 1-55 | Re-57 | 30 | 90°C/60 sec | 110°C/60 sec | H-2 | 9 | 47 | 1.6 | 13.0 |
| Example 1-56 | Re-1 | 30 | 90°C/60 sec | 110°C/60 sec | H-2 | 13 | 41 | 1.7 | 15.0 |
| Example 1-57 | Re-1 | 30 | 90°C/60 sec | 110°C/60 sec | H-3 | 13 | 43 | 1.9 | 16.0 |
| Example 1-58 | Re-1 | 30 | 90°C/60 sec | 110°C/60 sec | H-4 | 12 | 40 | 1.8 | 14.0 |
| Example 1-59 | Re-1 | 30 | 90°C/60 sec | 110°C/60 sec | H-6 | 10 | 36 | 1.7 | 12.0 |
| Example 1-60 | Re-28 | 30 | 90°C/60 sec | 110°C/60 sec | H-2 | 20 | 56 | 2.5 | 20.0 |
| Example 1-61 | Re-28 | 30 | 90°C/60 sec | 110°C/60 sec | H-3 | 20 | 58 | 2.7 | 20.0 |

(continued)

| Table 3 (Part 2) | Resist composition application conditions | | | PEB·Development conditions | | EUV evaluation results | | | |
|---|---|---|---|---|---|---|---|---|---|
| | Resist composition | Film thickness [nm] | PreBake | PEB | Developer | Bridge margin [nm] | Sensitivity [$mJ/cm^2$] | LER [nm] | Resolution [nm] |
| Example 1-62 | Re-28 | 30 | 90°C/60 sec | 110°C/60 sec | H-4 | 19 | 55 | 2.6 | 19.0 |
| Example 1-63 | Re-28 | 30 | 90°C/60 sec | 110°C/60 sec | H-5 | 18 | 53 | 2.5 | 18.0 |
| Example 1-64 | Re-28 | 30 | 90°C/60 sec | 110°C/60 sec | H-6 | 17 | 51 | 2.5 | 17.0 |
| Example 1-65 | Re-40 | 30 | 90°C/60 sec | 110°C/60 sec | H-2 | 17 | 42 | 2.2 | 19.0 |
| Example 1-66 | Re-40 | 30 | 90°C/60 sec | 110°C/60 sec | H-3 | 17 | 44 | 2.4 | 19.0 |
| Example 1-67 | Re-40 | 30 | 90°C/60 sec | 110°C/60 sec | H-4 | 16 | 41 | 2.3 | 18.0 |
| Example 1-68 | Re-40 | 30 | 90°C/60 sec | 110°C/60 sec | H-5 | 15 | 39 | 2.2 | 17.0 |
| Example 1-69 | Re-40 | 30 | 90°C/60 sec | 110°C/60 sec | H-6 | 14 | 37 | 2.2 | 16.0 |
| Comparative Example 1-1 | Re-4 | 30 | 90°C/60 sec | 110°C/60 sec | H-1 | 25 | 68 | 3.0 | 25.0 |
| Comparative Example 1-2 | Re-5 | 30 | 90°C/60 sec | 110°C/60 sec | H-1 | 24 | 63 | 2.9 | 24.0 |
| Comparative Example 1-3 | Re-6 | 30 | 90°C/60 sec | 110°C/60 sec | H-1 | 23 | 68 | 2.8 | 23.0 |

From the results shown in Table 3, it is clear that the resist compositions of Examples provide a narrow space width at which bridge defects start to occur in the formation of the L/S pattern. It is also clear that the resist compositions of Examples are excellent in sensitivity and can provide a pattern excellent in LER and resolution.

In addition, from the comparison of Examples 1-56 to 1-59, the comparison of Examples 1-60 to 1-64, and the comparison of Examples 1-65 to 1-69, it was confirmed that when the developer was an organic solvent-based developer and included a hydrocarbon-based solvent and an ester-based solvent having 6 or less carbon atoms (preferably, when the mass ratio of the content of the hydrocarbon-based solvent to the content of the ester-based solvent having 6 or less carbon atoms was 10/90 to 30/70, and more preferably, when the mass ratio was 20/80 to 30/70), the space width at which bridge defects started to occur in the formation of the L/S pattern was narrower, and a pattern with even better resolution could be formed.

On the other hand, it was confirmed that the resist compositions of Comparative Examples did not exhibit the desired effects.

[Pattern formation and evaluation (2)]

[Pattern formation by EUV exposure]

An underlayer film-forming composition SHB-A940 (manufactured by Shin-Etsu Chemical Co., Ltd.) was applied to a silicon wafer and baked at 205°C for 60 seconds to form an underlayer film having a film thickness of 20 nm. Each of the resist compositions (Re-1 to Re-6) shown in Table 2 above was applied thereto, and a resist film was formed under the conditions (film thickness and PreBake) shown in Table 4. Thus, a silicon wafer having a resist film was formed.

The silicon wafer having the resist film obtained by the above-described procedure was subjected to pattern irradiation using an EUV scanner NXE3400 (NA 0.33, $\sigma$ 0.9/0.7, dipole illumination) manufactured by ASML. A mask having a line size of 18 nm and a line:space ratio of 1:1 was used as a reticle. Subsequently, only in the case where there is a description, baking (Post Exposure Bake; PEB) was performed using a coater/developer CLEAN TRAC LITHIUS Pro Z manufactured by Tokyo Electron Ltd. under the conditions shown in Table 4; development was then performed with a developer shown in Table 4 by puddling for 30 seconds; only in the case where there is a description in Table 4, rinsing was performed by pouring a rinsing liquid shown in Table 4 for 10 seconds while the wafer was rotated at a rotational speed of 1,000 rpm; and the wafer was then rotated at a rotational speed of 4,000 rpm for 30 seconds to obtain a line-and-space pattern with a pitch of 36 nm.

[Evaluation]

<Optimum exposure dose (Sensitivity)>

In the above pattern formation by EUV exposure, a line width of the line-and-space pattern was measured using a critical dimension-scanning electron microscope (SEM: Scanning Electron Microscope (CG-6300, manufactured by Hitachi High-Tech Corporation)) while changing the exposure dose, an exposure dose at which the line width became 18 nm was determined, and this was defined as an optimum exposure dose ($mJ/cm^2$). The smaller this value, the better the performance.

<<Space width at which bridge defects start to occur in formation of L/S pattern (Bridge margin)>

A line-and-space pattern (line:space = 1:1) having a line width of 18 nm was exposed while the irradiation dose was changed from the above sensitivity, and a space width (nm) at which a bridge starts to form between spaces was determined as an indicator of a "bridge margin". The smaller the above value, the narrower the space distance required for preventing the occurrence of bridge defects, and the better the performance.

<Line edge roughness (LER)>

With respect to randomly selected 30 points in 50 $\mu$m in the length direction of a line pattern (L/S = 1/1) having a line width of 18 nm and formed at the exposure dose providing the above sensitivity, the distance from a reference line on which the edge should be located was measured using a scanning electron microscope (CG-6300, manufactured by Hitachi High-Tech Corporation), the standard deviation was determined, and $3\sigma$ was calculated. The smaller the value, the better the performance.

<Resolution>

A limit resolving power (a minimum line width at which a line and a space (line:space = 1:1) were separately resolved) at the exposure dose providing the above sensitivity was defined as a resolution (unit: nm). The smaller the value, the better the performance.

Table 8

| Table 4 | Resist composition application conditions | | | PEB·Development conditions | | EUV evaluation results | | | |
|---|---|---|---|---|---|---|---|---|---|
| | Resist composition | Film thickness [nm] | PreBake | PEB | Developer | Bridge margin [nm] | Sensitivity [mJ/cm$^2$] | LER [nm] | Resolution [nm] |
| Example 2-1 | Re-1 | 30 | 90°C/60 sec | 110°C/60 sec | H-1 | 9 | 45 | 1.3 | 11.0 |
| Example 2-2 | Re-2 | 30 | 90°C/60 sec | 110°C/60 sec | H-1 | 14 | 50 | 1.5 | 10.0 |
| Example 2-3 | Re-3 | 30 | 90°C/60 sec | 110°C/60 sec | H-1 | 10 | 48 | 1.4 | 12.0 |
| Example 2-4 | Re-1 | 30 | 90°C/60 sec | 110°C/60 sec | H-5 | 8 | 41 | 1.2 | 10.0 |
| Comparative Example 2-1 | Re-4 | 30 | 90°C/60 sec | 110°C/60 sec | H-1 | 18 | 65 | 2.4 | 17.0 |
| Comparative Example 2-2 | Re-5 | 30 | 90°C/60 sec | 110°C/60 sec | H-1 | 17 | 60 | 2.3 | 16.0 |
| Comparative Example 2-3 | Re-6 | 30 | 90°C/60 sec | 110°C/60 sec | H-1 | 16 | 60 | 2.2 | 15.0 |

From the results shown in Table 4, it is clear that the resist compositions of Examples provide a narrow space width at which bridge defects start to occur in the formation of the L/S pattern. It is also clear that the resist compositions of Examples are excellent in sensitivity and can provide a pattern excellent in LER and resolution.

In addition, from the comparison of Examples 2-1 to 2-3, it was confirmed that when the resist composition satisfied the requirement 2 or the requirement 3, the space width at which bridge defects started to occur in the formation of the L/S pattern was narrower, the sensitivity was even better, and a pattern with even better LER could be formed.

In addition, from the comparison between Example 2-1 and Example 2-4, it was confirmed that when the developer was an organic solvent-based developer and included an ester-based solvent having 6 or less carbon atoms and a hydrocarbon-based solvent, the space width at which bridge defects started to occur in the formation of the L/S pattern was narrower, and a pattern with even better LER and resolution could be formed.

On the other hand, it was confirmed that the resist compositions of Comparative Examples did not exhibit the desired effects.

## Claims

**1.** An actinic ray-sensitive or radiation-sensitive resin composition comprising:

a resin having a group that is decomposed by an action of an acid to generate a polar group;
a photoacid generator; and
a boron-containing compound,
wherein a requirement 1 is satisfied,
Requirement 1: a value A determined by an equation (1) is 0.120 or more,

$$A = ([H_A] \times 0.04 + [C_A] \times 1.0 + [N_A] \times 2.1 + [O_A] \times 3.6 + [F_A] \times 5.6 + [S_A] \times 1.5 + [B_A] \times 0.5 + [I_A] \times 39.5)/([H_A] \times 1 + [C_A] \times 12 + [N_A] \times 14 + [O_A] \times 16 + [F_A] \times 19 + [S_A] \times 32 + [B_A] \times 11 + [I_A] \times 127) \quad \text{Equation (1):}$$

in the equation (1), $[H_A]$ represents a molar ratio of hydrogen atoms derived from total solid contents in the composition to all atoms of the total solid contents, $[C_A]$ represents a molar ratio of carbon atoms derived from the total solid contents in the composition to all the atoms of the total solid contents, $[N_A]$ represents a molar ratio of nitrogen atoms derived from the total solid contents in the composition to all the atoms of the total solid contents, $[O_A]$ represents a molar ratio of oxygen atoms derived from the total solid contents in the composition to all the atoms of the total solid contents, $[F_A]$ represents a molar ratio of fluorine atoms derived from the total solid contents in the composition to all the atoms of the total solid contents, $[S_A]$ represents a molar ratio of sulfur atoms derived from the total solid contents in the composition to all the atoms of the total solid contents, $[B_A]$ represents a molar ratio of boron atoms derived from the total solid contents in the composition to all the atoms of the total solid contents, and $[I_A]$ represents a molar ratio of iodine atoms derived from the total solid contents in the composition to all the atoms of the total solid contents.

**2.** The actinic ray-sensitive or radiation-sensitive resin composition according to claim 1, further comprising an acid diffusion control agent.

**3.** The actinic ray-sensitive or radiation-sensitive resin composition according to claim 1 or 2,

wherein the photoacid generator is an onium salt compound,
the boron-containing compound is a salt compound,
when the composition includes an acid diffusion control agent which is a salt compound, a requirement 4 is satisfied, and
when the composition does not include an acid diffusion control agent which is a salt compound, a requirement 5 is satisfied,
Requirement 4: a value Cl determined by an equation (4) is 0.115 or more,

$$C1 = ([H_{C1}] \times 0.04 + [C_{C1}] \times 1.0 + [N_{C1}] \times 2.1 + [O_{C1}] \times 3.6 + [F_{C1}] \times 5.6 + [S_{C1}] \times 1.5 + [B_{C1}] \times 0.5 + [I_{C1}] \times 39.5)/([H_{C1}] \times 1 + [C_{C1}] \times 12 + [N_{C1}] \times 14 + [O_{C1}] \times 16 + [F_{C1}] \times 19 + [S_{C1}] \times 32 + [B_{C1}] \times 11 + [I_{C1}] \times 127) \quad \text{Equation (4):}$$

in the equation (4), $[H_{C1}]$ represents a molar ratio of a total of hydrogen atoms derived from a cation of the photoacid generator, hydrogen atoms derived from a cation of the boron-containing compound, and hydrogen atoms derived from a cation of the acid diffusion control agent to a total of all atoms derived from the cation of the photoacid generator, all atoms derived from the cation of the boron-containing compound, and all atoms derived from the cation of the acid diffusion control agent, $[C_{C1}]$ represents a molar ratio of a total of carbon atoms derived from the cation of the photoacid generator, carbon atoms derived from the cation of the boron-containing compound, and carbon atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[N_{C1}]$ represents a molar ratio of a total of nitrogen atoms derived from the cation of the photoacid generator, nitrogen atoms derived from the cation of the boron-containing compound, and nitrogen atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[O_{C1}]$ represents a molar ratio of a total of oxygen atoms derived from the cation of the photoacid generator, oxygen atoms derived from the cation of the boron-containing compound, and oxygen atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[F_{C1}]$ represents a molar ratio of a total of fluorine atoms derived from the cation of the photoacid generator, fluorine atoms derived from the cation of the boron-containing compound, and fluorine atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[S_{C1}]$ represents a molar ratio of a total of sulfur atoms derived from the cation of the photoacid generator, sulfur atoms derived from the cation of the boron-containing compound, and sulfur atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[B_{C1}]$ represents a molar ratio of a total of boron atoms derived from the cation of the photoacid generator, boron atoms derived from the cation of the boron-containing compound, and boron atoms derived from the cation of the acid diffusion control agent to the total of all the atoms derived from the cation of the photoacid generator, all the atoms derived from the cation of the boron-containing compound, and all the atoms derived from the cation of the acid diffusion control agent, and $[I_{C1}]$ represents a molar ratio of a total of iodine atoms derived from the cation of the photoacid generator, iodine atoms derived from the cation of the boron-containing compound, and iodine atoms derived from the cation of the acid diffusion control agent to the total of all the atoms derived from the cation of the photoacid generator, all the atoms derived from the cation of the boron-containing compound, and all the atoms derived from the cation of the acid diffusion control agent,

Requirement 5: a value C2 determined by an equation (5) is 0.115 or more,

$$C2 = ([H_{C2}] \times 0.04 + [C_{C2}] \times 1.0 + [N_{C2}] \times 2.1 + [O_{C2}] \times 3.6 + [F_{C2}] \times 5.6 + [S_{C2}] \times 1.5 + [B_{C2}] \times 0.5 + [I_{C2}] \times 39.5)/([H_{C2}] \times 1 + [C_{C2}] \times 12 + [N_{C2}] \times 14 + [O_{C2}] \times 16 + [F_{C2}] \times 19 + [S_{C2}] \times 32 + [B_{C2}] \times 11 + [I_{C2}] \times 127) \quad \text{Equation (5):}$$

in the equation (5), $[H_{C2}]$ represents a molar ratio of a total of hydrogen atoms derived from the cation of the photoacid generator and hydrogen atoms derived from the cation of the boron-containing compound to a total of all the atoms derived from the cation of the photoacid generator and all the atoms derived from the cation of the boron-containing compound, $[C_{C2}]$ represents a molar ratio of a total of carbon atoms derived from the cation of the photoacid generator and carbon atoms derived from the cation of the boron-containing compound to the total of all the atoms derived from the cation of the photoacid generator and all the atoms derived from the cation of the boron-containing compound, $[N_{C2}]$ represents a molar ratio of a total of nitrogen atoms derived from the cation of the photoacid generator and nitrogen atoms derived from the cation of the boron-containing compound to the total of all the atoms derived from the cation of the photoacid generator and all the atoms derived from the cation of the boron-containing compound, $[O_{C2}]$ represents a molar ratio of a total of oxygen atoms derived from the cation of the photoacid generator and oxygen atoms derived from the cation of the boron-containing compound to the total of all the atoms derived from the cation of the photoacid generator and all the atoms derived from the cation of the boron-containing compound, $[F_{C2}]$ represents a molar ratio of a total of fluorine atoms derived from the cation of the photoacid generator and fluorine atoms derived from the cation of the boron-containing compound to the total of all the atoms derived from the cation of the photoacid generator and all the atoms derived from the cation of the boron-containing compound, $[S_{C2}]$ represents a molar ratio of a total of sulfur atoms derived from the cation of the

photoacid generator and sulfur atoms derived from the cation of the boron-containing compound to the total of all the atoms derived from the cation of the photoacid generator and all the atoms derived from the cation of the boron-containing compound, $[B_{C2}]$ represents a molar ratio of a total of boron atoms derived from the cation of the photoacid generator and boron atoms derived from the cation of the boron-containing compound to the total of all the atoms derived from the cation of the photoacid generator and all the atoms derived from the cation of the boron-containing compound, and $[I_{C2}]$ represents a molar ratio of a total of iodine atoms derived from the cation of the photoacid generator and iodine atoms derived from the cation of the boron-containing compound to the total of all the atoms derived from the cation of the photoacid generator and all the atoms derived from the cation of the boron-containing compound.

**4.** The actinic ray-sensitive or radiation-sensitive resin composition according to claim 3,

wherein the composition includes an acid diffusion control agent, and
the acid diffusion control agent is not a salt compound.

**5.** The actinic ray-sensitive or radiation-sensitive resin composition according to claim 3,

wherein the composition includes an acid diffusion control agent, and
the acid diffusion control agent is a salt compound.

**6.** The actinic ray-sensitive or radiation-sensitive resin composition according to claim 5, wherein the acid diffusion control agent is an onium salt compound.

**7.** The actinic ray-sensitive or radiation-sensitive resin composition according to claim 3, wherein the photoacid generator includes a cation including a fluorine atom.

**8.** A resist film formed using the actinic ray-sensitive or radiation-sensitive resin composition according to claim 3.

**9.** A pattern forming method comprising:

a step of forming a resist film on a substrate using the actinic ray-sensitive or radiation-sensitive resin composition according to claim 3;
a step of exposing the resist film; and
a step of developing the exposed resist film with a developer to form a pattern.

**10.** The pattern forming method according to claim 9, wherein the developer is a developer including an organic solvent.

**11.** The pattern forming method according to claim 10, wherein the developer includes an ester-based solvent having 6 or less carbon atoms and a hydrocarbon-based solvent.

**12.** A method for producing an electronic device, the method comprising the pattern forming method according to claim 9.

**13.** The actinic ray-sensitive or radiation-sensitive resin composition according to claim 1,

wherein the photoacid generator is an onium salt compound,
the boron-containing compound is a salt compound,
when the composition includes an acid diffusion control agent which is an onium salt compound, a requirement 2 is satisfied, and
when the composition does not include an acid diffusion control agent which is an onium salt compound, a requirement 3 is satisfied,
Requirement 2: a value B1 determined by an equation (2) is 0.115 or more,

$$B1 = ([H_{B1}] \times 0.04 + [C_{B1}] \times 1.0 + [N_{B1}] \times 2.1 + [O_{B1}] \times 3.6 + [F_{B1}] \times 5.6 + [S_{B1}] \times 1.5 + [B_{B1}] \times 0.5 + [I_{B1}] \times 39.5)/([H_{B1}] \times 1 + [C_{B1}] \times 12 + [N_{B1}] \times 14 + [O_{B1}] \times 16 + [F_{B1}] \times 19 + [S_{B1}] \times 32 + [B_{B1}] \times 11 + [I_{B1}] \times 127) \quad \text{Equation (2):}$$

in the equation (2), $[H_{B1}]$ represents a molar ratio of a total of hydrogen atoms derived from a cation of the

photoacid generator, hydrogen atoms derived from a cation of the boron-containing compound, and hydrogen atoms derived from a cation of the acid diffusion control agent to a total of all atoms derived from the cation of the photoacid generator, all atoms derived from the cation of the boron-containing compound, and all atoms derived from the cation of the acid diffusion control agent, $[C_{B1}]$ represents a molar ratio of a total of carbon atoms derived from the cation of the photoacid generator, carbon atoms derived from the cation of the boron-containing compound, and carbon atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[N_{B1}]$ represents a molar ratio of a total of nitrogen atoms derived from the cation of the photoacid generator, nitrogen atoms derived from the cation of the boron-containing compound, and nitrogen atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[O_{B1}]$ represents a molar ratio of a total of oxygen atoms derived from the cation of the photoacid generator, oxygen atoms derived from the cation of the boron-containing compound, and oxygen atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[F_{B1}]$ represents a molar ratio of a total of fluorine atoms derived from the cation of the photoacid generator, fluorine atoms derived from the cation of the boron-containing compound, and fluorine atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[S_{B1}]$ represents a molar ratio of a total of sulfur atoms derived from the cation of the photoacid generator, sulfur atoms derived from the cation of the boron-containing compound, and sulfur atoms derived from the cation of the acid diffusion control agent to the total of all the atoms derived from the cation of the photoacid generator, all the atoms derived from the cation of the boron-containing compound, and all the atoms derived from the cation of the acid diffusion control agent, $[B_{B1}]$ represents a molar ratio of a total of boron atoms derived from the cation of the photoacid generator, boron atoms derived from the cation of the boron-containing compound, and boron atoms derived from the cation of the acid diffusion control agent to the total of all the atoms derived from the cation of the photoacid generator, all the atoms derived from the cation of the boron-containing compound, and all the atoms derived from the cation of the acid diffusion control agent, and $[I_{B1}]$ represents a molar ratio of a total of iodine atoms derived from the cation of the photoacid generator, iodine atoms derived from the cation of the boron-containing compound, and iodine atoms derived from the cation of the acid diffusion control agent to the total of all the atoms derived from the cation of the photoacid generator, all the atoms derived from the cation of the boron-containing compound, and all the atoms derived from the cation of the acid diffusion control agent,
Requirement 3: a value B2 determined by an equation (3) is 0.115 or more,

$$B2 = ([H_{B2}] \times 0.04 + [C_{B2}] \times 1.0 + [N_{B2}] \times 2.1 + [O_{B2}] \times 3.6 + [F_{B2}] \times 5.6 + [S_{B2}] \times 1.5 + [B_{B2}] \times 0.5 + [I_{B2}] \times 39.5)/([H_{B2}] \times 1 + [C_{B2}] \times 12 + [N_{B2}] \times 14 + [O_{B2}] \times 16 + [F_{B2}] \times 19 + [S_{B2}] \times 32 + [B_{B2}] \times 11 + [I_{B2}] \times 127) \quad \text{Equation (3):}$$

in the equation (3), $[H_{B2}]$ represents a molar ratio of a total of hydrogen atoms derived from the cation of the photoacid generator and hydrogen atoms derived from the cation of the boron-containing compound to a total of all the atoms derived from the cation of the photoacid generator and all the atoms derived from the cation of the boron-containing compound, $[C_{B2}]$ represents a molar ratio of a total of carbon atoms derived from the cation of the photoacid generator and carbon atoms derived from the cation of the boron-containing compound to the total of all the atoms derived from the cation of the photoacid generator and all the atoms derived from the cation of the boron-containing compound, $[N_{B2}]$ represents a molar ratio of a total of nitrogen atoms derived from the cation of the photoacid generator and nitrogen atoms derived from the cation of the boron-containing compound to the total of all the atoms derived from the cation of the photoacid generator and all the atoms derived from the cation of the boron-containing compound, $[O_{B2}]$ represents a molar ratio of a total of oxygen atoms derived from the cation of the photoacid generator and oxygen atoms derived from the cation of the boron-containing compound to the total of all the atoms derived from the cation of the photoacid generator and all the atoms derived from the cation of the boron-containing compound, $[F_{B2}]$ represents a molar ratio of a total of fluorine atoms derived from the cation of the photoacid generator and fluorine atoms derived from the cation of the boron-containing compound to the total of all the atoms derived from the cation of the photoacid generator and all the atoms derived from the cation of the boron-containing compound, $[S_{B2}]$ represents a molar ratio of a total of sulfur atoms derived from the cation of the photoacid generator and sulfur atoms derived from the cation of the boron-containing compound to the total of all

the atoms derived from the cation of the photoacid generator and all the atoms derived from the cation of the boron-containing compound, $[B_{B2}]$ represents a molar ratio of a total of boron atoms derived from the cation of the photoacid generator and boron atoms derived from the cation of the boron-containing compound to the total of all the atoms derived from the cation of the photoacid generator and all the atoms derived from the cation of the boron-containing compound, and $[I_{B2}]$ represents a molar ratio of a total of iodine atoms derived from the cation of the photoacid generator and iodine atoms derived from the cation of the boron-containing compound to the total of all the atoms derived from the cation of the photoacid generator and all the atoms derived from the cation of the boron-containing compound.

**14.** The actinic ray-sensitive or radiation-sensitive resin composition according to claim 1 or 13, wherein the photoacid generator includes a cation including a fluorine atom.

**15.** The actinic ray-sensitive or radiation-sensitive resin composition according to claim 1 or 13, further comprising an acid diffusion control agent.

**16.** The actinic ray-sensitive or radiation-sensitive resin composition according to claim 15, wherein the acid diffusion control agent is an onium salt compound.

**17.** A resist film formed using the actinic ray-sensitive or radiation-sensitive resin composition according to claim 1 or 13.

**18.** A pattern forming method comprising:

a step of forming a resist film on a substrate using the actinic ray-sensitive or radiation-sensitive resin composition according to claim 1 or 13;
a step of exposing the resist film; and
a step of developing the exposed resist film with a developer to form a pattern.

**19.** The pattern forming method according to claim 18, wherein the developer is a developer including an organic solvent.

**20.** The pattern forming method according to claim 19, wherein the developer includes an ester-based solvent having 6 or less carbon atoms and a hydrocarbon-based solvent.

**21.** A method for producing an electronic device, the method comprising the pattern forming method according to claim 18.

## INTERNATIONAL SEARCH REPORT

International application No.
**PCT/JP2024/008673**

### A. CLASSIFICATION OF SUBJECT MATTER

***G03F 7/004***(2006.01)i; ***C07D 233/56***(2006.01)i; ***C07D 235/18***(2006.01)i; ***C07D 307/33***(2006.01)i; ***C07D 309/12***(2006.01)i; ***C07D 317/38***(2006.01)i; ***C07D 319/20***(2006.01)i; ***C07D 327/06***(2006.01)i; ***C07D 333/46***(2006.01)i; ***C07D 333/76***(2006.01)i; ***C08F 212/14***(2006.01)i; ***C08L 101/02***(2006.01)i; ***G03F 7/20***(2006.01)i; ***G03F 7/32***(2006.01)i; ***G03F 7/038***(2006.01)i; ***G03F 7/039***(2006.01)i

FI: G03F7/004 501; G03F7/004 503A; G03F7/039 601; G03F7/32; G03F7/038 601; C08L101/02; G03F7/20 501; G03F7/20 521; C08F212/14; C07D333/76; C07D327/06; C07D333/46; C07D309/12; C07D317/38; C07D319/20; C07D307/33 320; C07D233/56; C07D235/18

According to International Patent Classification (IPC) or to both national classification and IPC

### B. FIELDS SEARCHED

Minimum documentation searched (classification system followed by classification symbols)

G03F7/004; C07D233/56; C07D235/18; C07D307/33; C07D309/12; C07D317/38; C07D319/20; C07D327/06; C07D333/46; C07D333/76; C08F212/14; C08L101/02; G03F7/20; G03F7/32; G03F7/038; G03F7/039

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2016-080940 A (SHIN-ETSU CHEMICAL CO., LTD.) 16 May 2016 (2016-05-16) examples | 1-21 |
| X | JP 2020-067599 A (JSR CORPORATION) 30 April 2020 (2020-04-30) examples 14-15 | 1-21 |
| X | JP 2016-141796 A (SHIN-ETSU CHEMICAL CO., LTD.) 08 August 2016 (2016-08-08) example 32 | 1-21 |
| X | JP 2016-040598 A (SHIN-ETSU CHEMICAL CO., LTD.) 24 March 2016 (2016-03-24) example 11 | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/008673**

**C. DOCUMENTS CONSIDERED TO BE RELEVANT**

| Category* | Citation of document, with indication, where appropriate, of the relevant passages | Relevant to claim No. |
|---|---|---|
| X | JP 2004-053980 A (TOYO INK MANUFACTURING CO., LTD.) 19 February 2004 (2004-02-19) claims, examples 1-3 | 1-21 |
| X | JP 2011-081358 A (SUMITOMO CHEMICAL COMPANY, LIMITED) 21 April 2011 (2011-04-21) claims, examples 1-6 | 1-21 |
| X | WO 2012/169620 A1 (TOKYO OHKA KOGYO CO., LTD.) 13 December 2012 (2012-12-13) examples 79-81 | 1-2, 15-21 |
| X | JP 2021-092759 A (TOKYO OHKA KOGYO CO., LTD.) 17 June 2021 (2021-06-17) example 6 | 1-21 |
| X | JP 2020-076871 A (TOKYO OHKA KOGYO CO., LTD.) 21 May 2020 (2020-05-21) example 4 | 1-2, 15-21 |
| A | WO 2023/017711 A1 (FUJIFILM CORPORATION) 16 February 2023 (2023-02-16) paragraph [0188] | 11, 20 |
| A | JP 2006-126694 A (TOYO INK MANUFACTURING CO., LTD.) 18 May 2006 (2006-05-18) paragraphs [0030], [0033], [0088] | 1-21 |

Form PCT/ISA/210 (second sheet) (July 2022)

**INTERNATIONAL SEARCH REPORT**
**Information on patent family members**

International application No.
**PCT/JP2024/008673**

| Patent document cited in search report | Publication date (day/month/year) | Patent family member(s) | Publication date (day/month/year) |
|---|---|---|---|
| JP 2016-080940 A | 16 May 2016 | US 2016/0109803 A1<br>examples | |
| JP 2020-067599 A | 30 April 2020 | (Family: none) | |
| JP 2016-141796 A | 08 August 2016 | US 2016/0229940 A1<br>example 32<br>KR 10-2016-0096549 A<br>TW 201634498 A | |
| JP 2016-040598 A | 24 March 2016 | US 2016/0048076 A1<br>example 11<br>KR 10-2016-0019860 A<br>TW 201610585 A | |
| JP 2004-053980 A | 19 February 2004 | (Family: none) | |
| JP 2011-081358 A | 21 April 2011 | (Family: none) | |
| WO 2012/169620 A1 | 13 December 2012 | US 9134617 B2<br>examples 79-81<br>KR 10-2014-0047045 A<br>TW 201314365 A | |
| JP 2021-092759 A | 17 June 2021 | KR 10-2021-0067912 A<br>CN 112882341 A | |
| JP 2020-076871 A | 21 May 2020 | US 2020/0142307 A1<br>example 4<br>KR 10-2020-0052839 A<br>TW 202026759 A | |
| WO 2023/017711 A1 | 16 February 2023 | TW 202314229 A | |
| JP 2006-126694 A | 18 May 2006 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016085382 A
- WO 2022024928 A
- JP 2014041327 A
- WO 2018193954 A
- JP 2014098921 A
- WO 2020066824 A
- WO 2017154345 A
- WO 2020158313 A
- WO 2020158337 A
- US 20160070167 A
- US 20150004544 A
- US 20160237190 A
- US 20160274458 A
- WO 2020004306 A
- JP 2014059543 A
- JP 2013061648 A


### Non-patent literature cited in the description


- *Materials Letters*, 2008, vol. 62, 3152
- Prediction of polymer properties. Marcel Dekker Inc., 1993